# EUROPÄISCHE PATENTSCHRIFT

(11) **EP 2 593 184 B1**
(45) Veröffentlichungstag und Bekanntmachung des Hinweises auf die Patenterteilung: **31.12.2014**
(21) Anmeldenummer: 11731259.5
(22) Anmeldetag: 28.06.2011
(51) Int. Cl.: A61N 5/00, C07F 15/00, H01L 51/00, H05B 33/00

(54) **METALLKOMPLEXE MIT ORGANISCHEN LIGANDEN UND DEREN VERWENDUNG IN OLEDS**
METAL COMPLEXES WITH ORGANIC LIGANDS AND THE USE THEREOF IN OLEDS
COMPLEXES METALLIQUES A LIGANDS ORGANIQUES ET LEUR UTILISATION DANS DES OLED

(30) Priorität: 15.07.2010 DE 102010027218
(43) Veröffentlichungstag der Anmeldung: 22.05.2013
(73) Patentinhaber: Merck Patent GmbH, 64293 Darmstadt (DE)
(72) Erfinder: ANÉMIAN, Rémi Manouk, Seoul 140-210 (KR); SCHROEDER, Bernd, 65606 Villmar-Weyer (DE); DE NONANCOURT, Claire, 65929 Frankfurt am Main (DE)
(86) Internationale Anmeldenummer: PCT/EP2011/003168
(87) Internationale Veröffentlichungsnummer: WO 2012/007103

(56) Entgegenhaltungen:
- EP-A1- 1 818 077
- EP-A1- 1 820 801
- WO-A2-2004/048881
- DE-T5- 10 392 453
- US-A1- 2005 168 687

## Beschreibung

Der Aufbau organischer Elektrolumineszenzvorrichtungen (OLEDs), in denen organische Halbleiter als funktionelle Materialien eingesetzt werden, ist beispielsweise in US 4539507, US 5151629, EP 0676461 und WO 98/27136 beschrieben. Dabei werden als emittierende Materialien zunehmend metallorganische Komplexe eingesetzt, die Phosphoreszenz statt Fluoreszenz zeigen (M. A. Baldo et al., Appl. Phys. Lett. 1999, 75, 4-6). Aus quantenmechanischen Gründen ist unter Verwendung metallorganischer Verbindungen als Phosphoreszenzemitter eine bis zu vierfache Energie- und Leistungseffizienz möglich. Generell gibt es bei OLEDs, die Triplettemission zeigen, jedoch immer noch Verbesserungsbedarf, insbesondere im Hinblick auf Effizienz, Betriebsspannung und Lebensdauer. Dies gilt insbesondere für OLEDs, welche im kürzerwelligen Bereich, also grün und blau, emittieren. Weiterhin weisen viele phosphoreszierende Emitter keine ausreichende Löslichkeit für eine Verarbeitung aus Lösung auf, so dass es auch hier weiteren Verbesserungsbedarf gibt.

Gemäß dem Stand der Technik werden in phosphoreszierenden OLEDs als Triplettemitter insbesondere Iridium- und Platinkomplexe eingesetzt, welche üblicherweise als cyclometallierte Komplexe eingesetzt werden. Dabei sind die Liganden häufig Derivate von Phenylpyridin. Jedoch ist die Löslichkeit derartiger Komplexe häufig gering, was eine Verarbeitung aus Lösung erschwert oder gänzlich verhindert.

Aus dem Stand der Technik sind Iridiumkomplexe bekannt, welche am Phenylring des Phenylpyridinliganden in para-Position zur Koordination an das Metall mit einer gegebenenfalls substituierten Aryl- oder Heteroarylgruppe substituiert sind (WO 2004/026886 A2). Dadurch wurde eine verbesserte Löslichkeit der Komplexe erzielt. Jedoch gibt es auch hier noch weiteren Verbesserungsbedarf in Bezug auf die Löslichkeit sowie die Effizienz und die Lebensdauer der Komplexe.

DE 103 92 453T5 offenbart homoleptische Iridium-Metallkomplexe enthaltend Phenylpyridin-Liganden, die mit Carbazolyl-Phenylen substituiert sind.

Überraschend wurde gefunden, dass bestimmte, unten näher beschriebene Metallchelatkomplexe eine verbesserte Löslichkeit aufweisen und weiterhin zu Verbesserungen der organischen Elektrolumineszenzvorrichtung führen, insbesondere hinsichtlich der Effizienz und der Lebensdauer. Diese Metallkomplexe und organische Elektrolumineszenzvorrichtungen, welche diese Komplexe enthalten, sind daher der Gegenstand der vorliegenden Erfindung.

Gegenstand der Erfindung ist eine Verbindung gemäß Anspruch 1.
dabei können auch mehrere Liganden L miteinander über eine beliebige Brücke Z verknüpft sein und so ein tridentates, tetradentates, pentadentates oder hexadentates Ligandensystem aufspannen.

In einer weiteren Ausführungsform der vorliegenden Erfindung ist der Rest W gleich der Formel (3d).

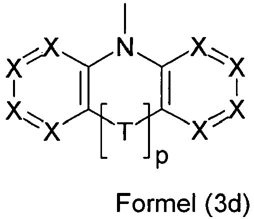

In einer bevorzugten Ausführungsform der vorliegenden Erfindung ist p = 0. In einer weiteren bevorzugten Ausführungsform der vorliegenden Erfindung ist p = 1 und T ist ausgewählt aus der Gruppe C(R¹)₂, O, S und NR¹, Si, Ge und C(R¹)₂-C(R¹)₂.

In einer weiteren bevorzugten Ausführungsform der vorliegenden Erfindung ist der Rest W ausgewählt aus der Gruppe von Resten der Formeln (47) bis (55)

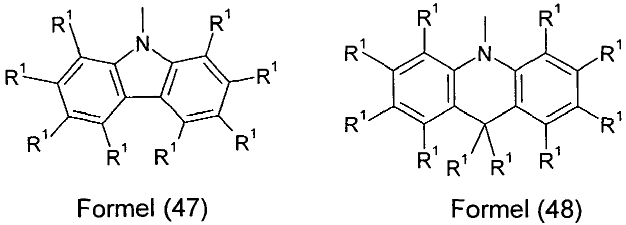

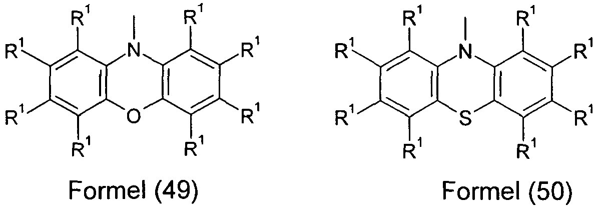

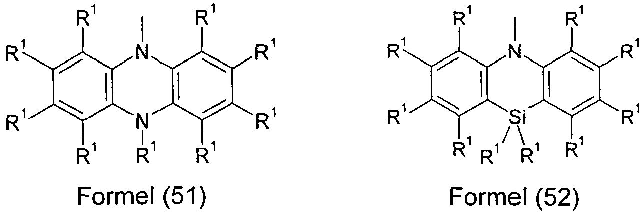

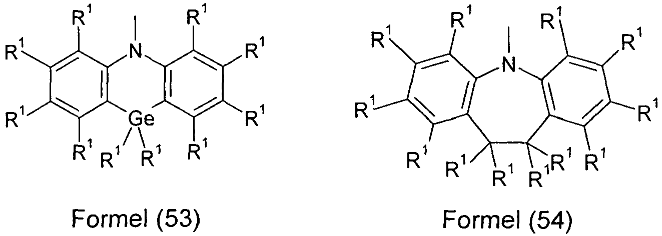

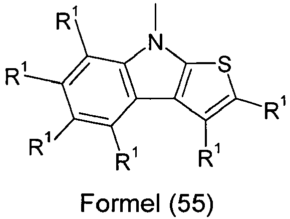

In einer weiteren ganz besonders bevorzugten Ausführungsform der vorliegenden Erfindung ist der Rest W ausgewählt aus den Resten der Formeln (47) bis (51).

In einer insbesondere bevorzugten Ausführungsform der vorliegenden Erfindung ist der Rest W gleich dem Rest der Formel (47).

Weiter ganz besonders bevorzugte Ausführungsformen der vorliegenden Erfindung sind homoleptische Verbindungen aus der Gruppe der Verbindungen mit den Formel (71) bis (85).

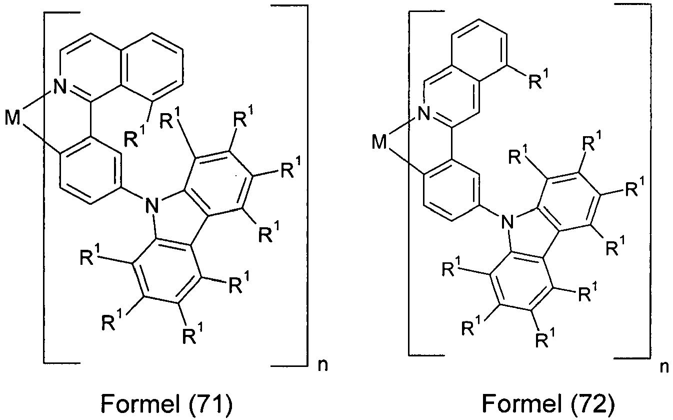

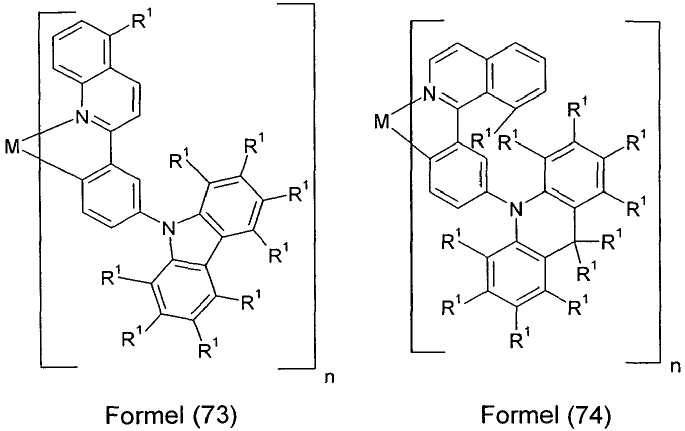

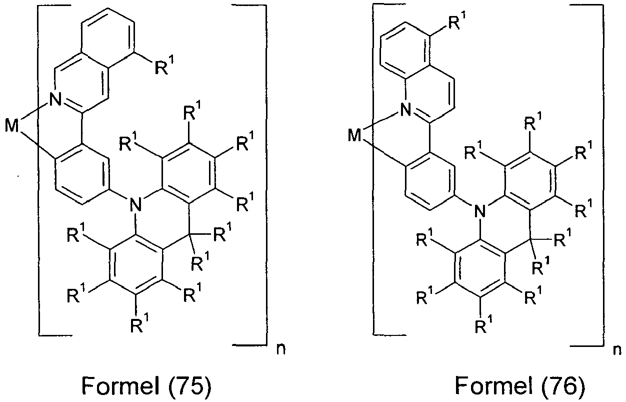

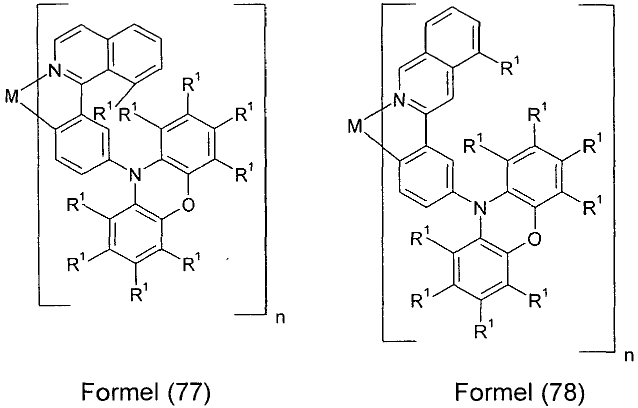

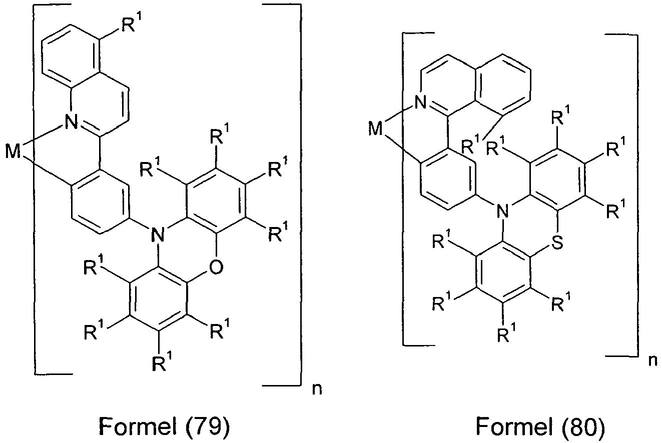

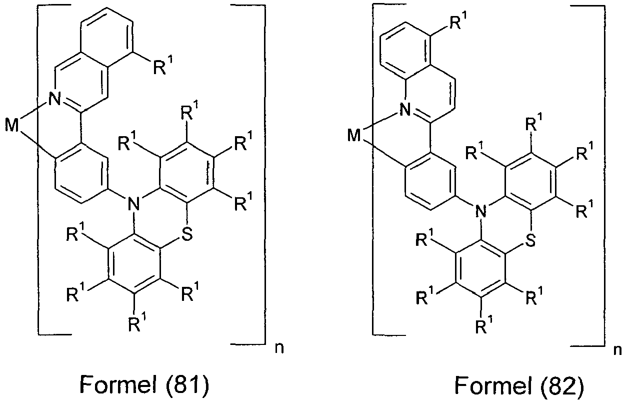

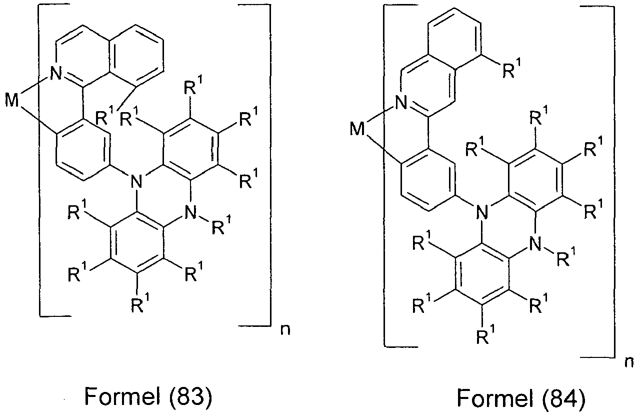

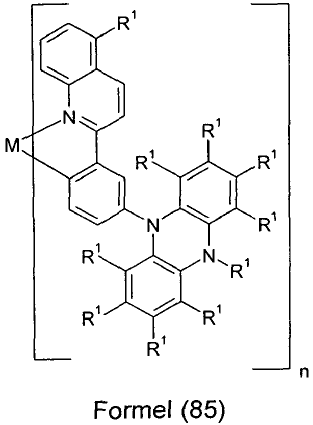

Die Verbindungen der Formel (71) bis (73) repräsentieren hierbei eine ganz besonders bevorzugte Ausführungsform der vorliegenden Erfindung.

In einer besonders bevorzugten Ausführungsform ist R¹ gleich oder verschieden bei jedem Auftreten H, D, F, Cl, Br, I, N(R²)₂, CN, NO₂, eine geradkettige Alkyl- oder Alkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl- oder Alkoxygruppe mit 3 bis 40 C-Atomen, die jeweils mit einem oder mehreren Resten R² substituiert sein kann oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R² substituiert sein kann, oder eine Kombination aus zwei oder mehr dieser Gruppen; dabei können zwei oder mehr Reste R¹ auch miteinander ein mono- oder polycyclisches, aliphatisches, aromatisches und/oder benzoannelliertes Ringsystem bilden.

In einer weiteren ganz besonders bevorzugten Ausführungsform der vorliegenden Erfindung ist der Rest R¹ gleich oder verscheiden bei jedem Auftreten H, geradkettige Alkyl- oder Alkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl- oder Alkoxygruppe mit 3 bis 40 C-Atomen, -NH₂, -SR³, -OR³ oder ein Rest der Formeln (86) bis (224) wobei die angegebenen Formeln ihrerseits mit einem oder mehreren Resten R³ substituiert sein können, die bei jedem Auftreten gleich oder verschieden sein können. 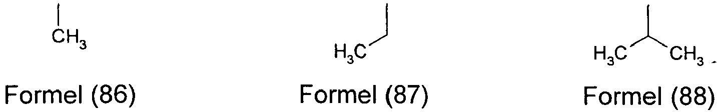 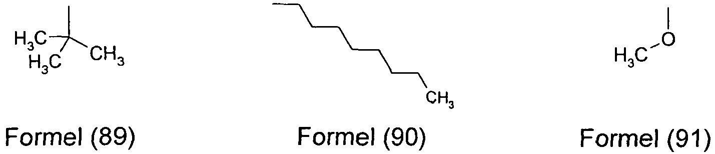 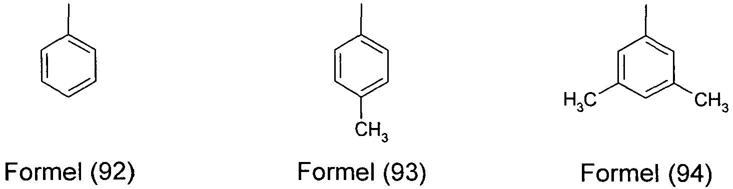 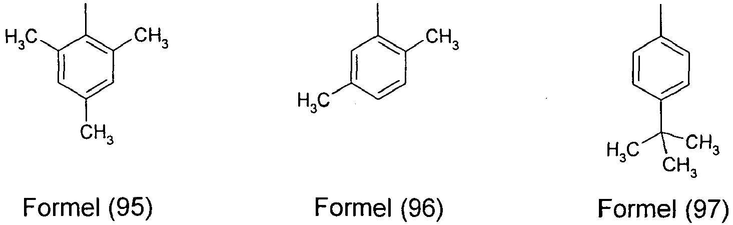 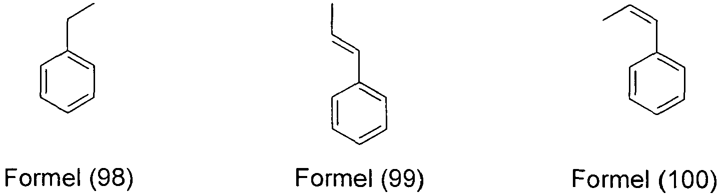 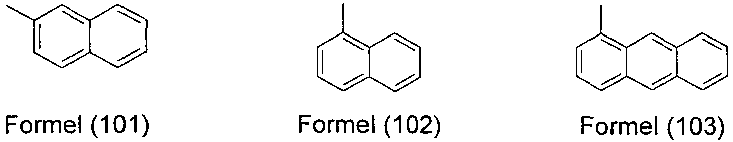 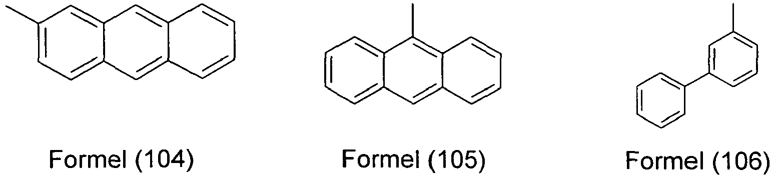 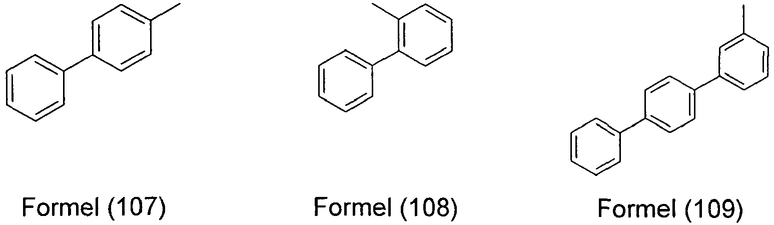 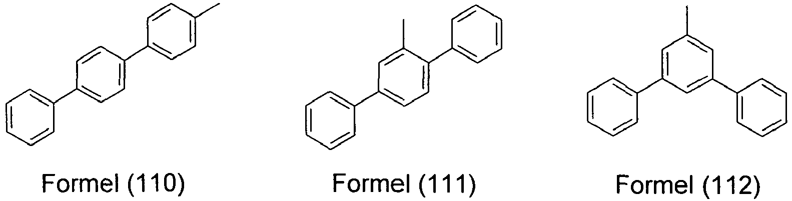 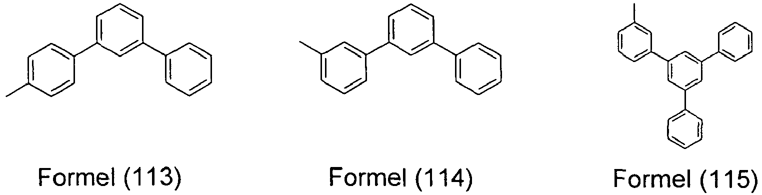 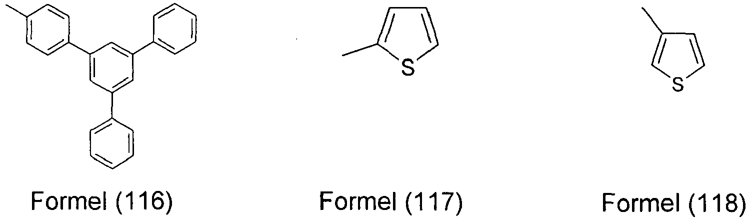 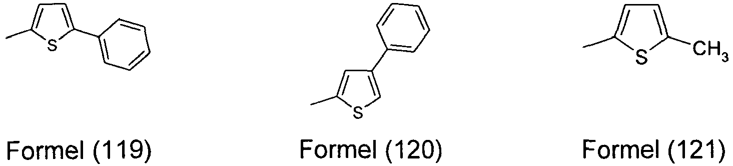 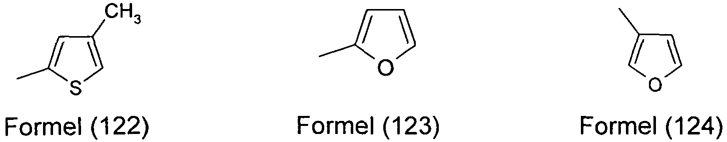 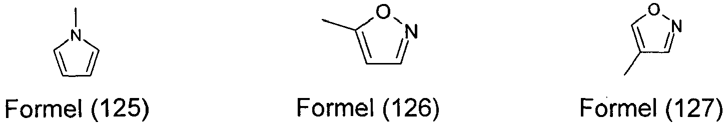 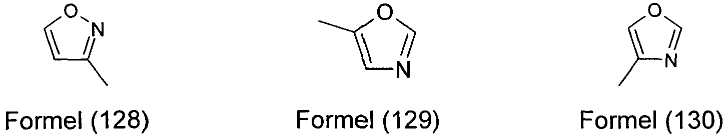 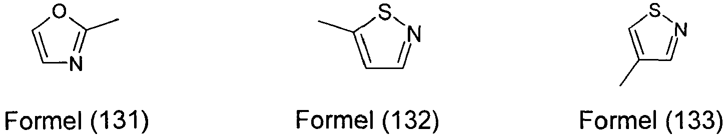 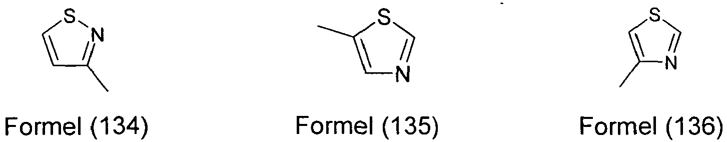 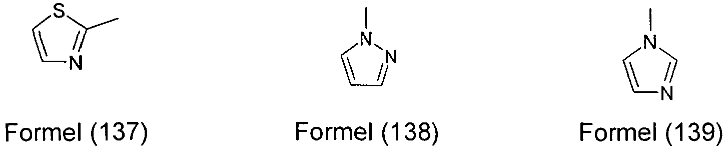 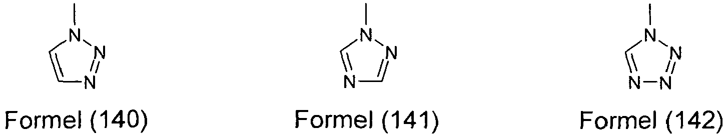 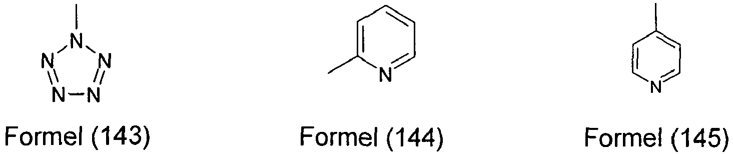 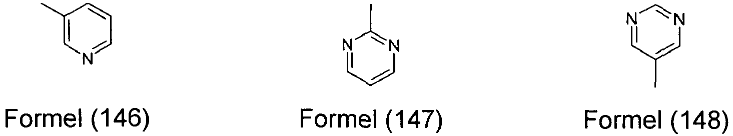 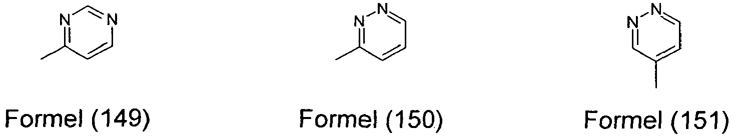 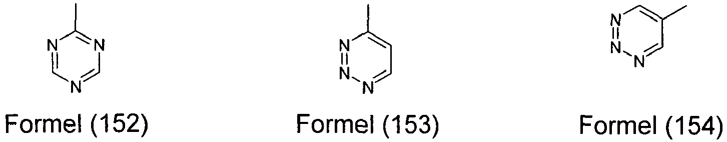 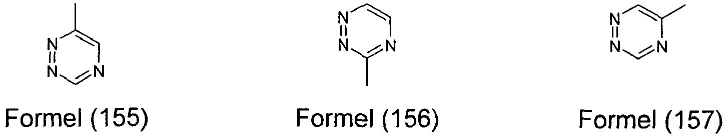 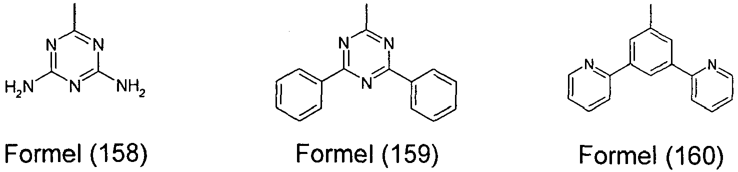 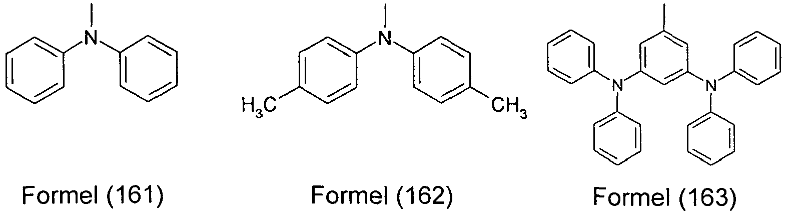 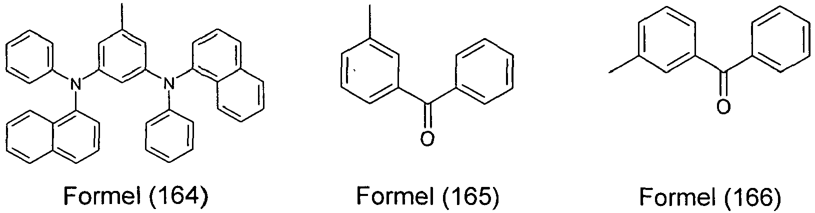 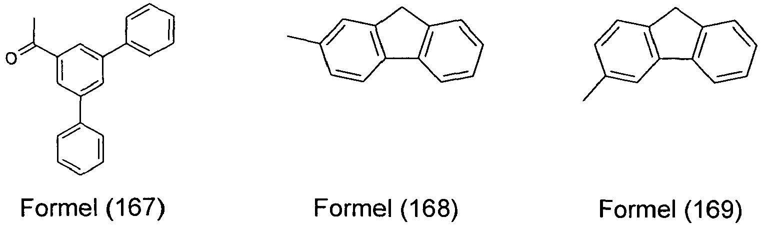 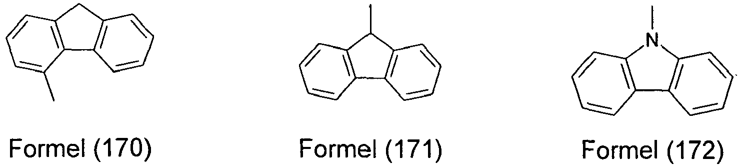 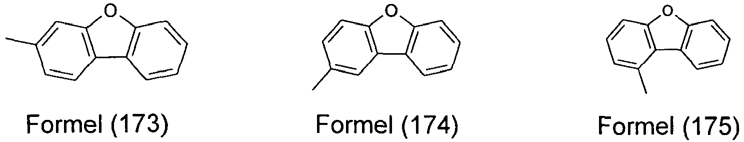 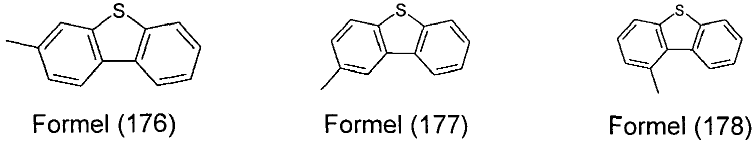 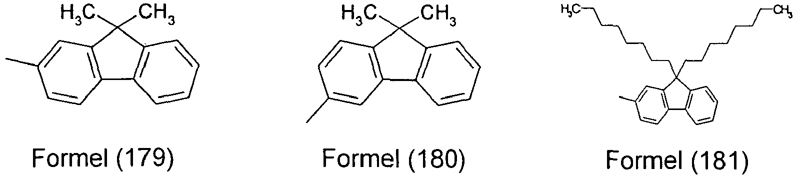 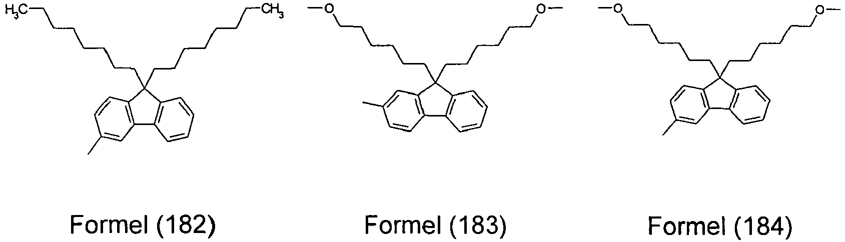 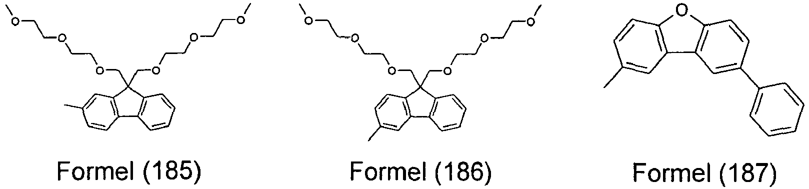 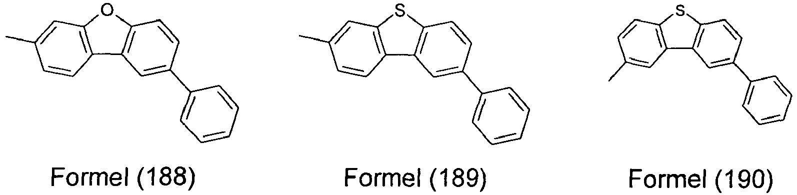 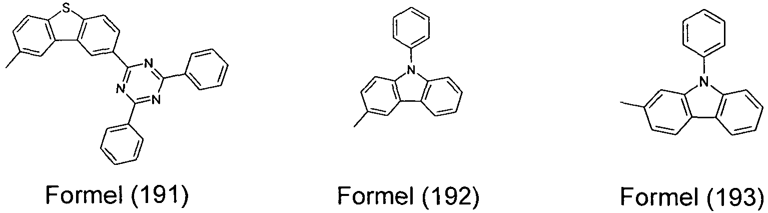 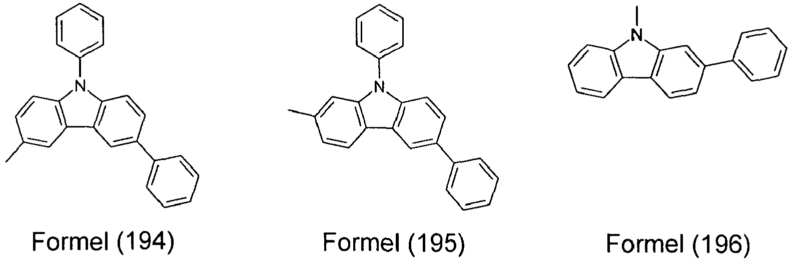 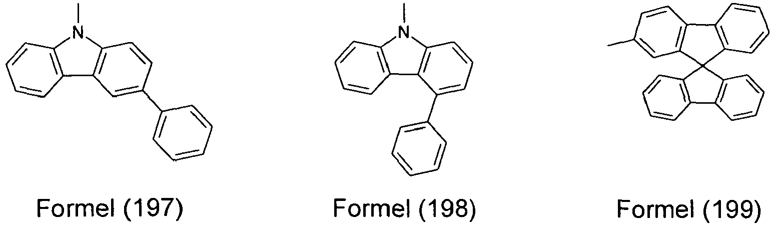 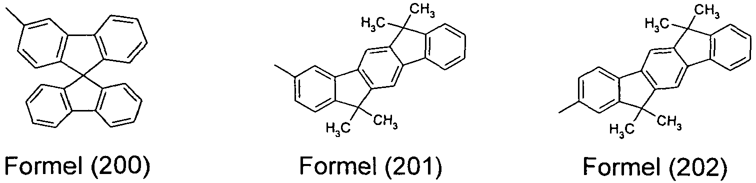 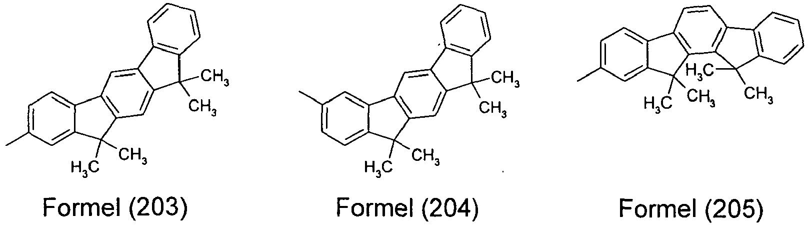 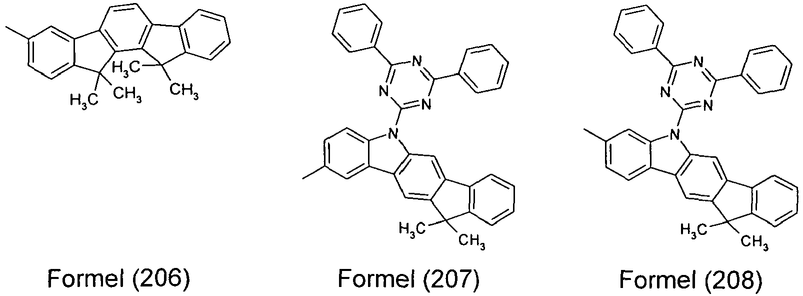 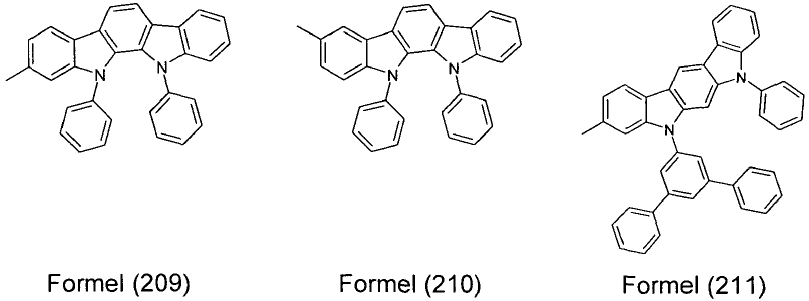 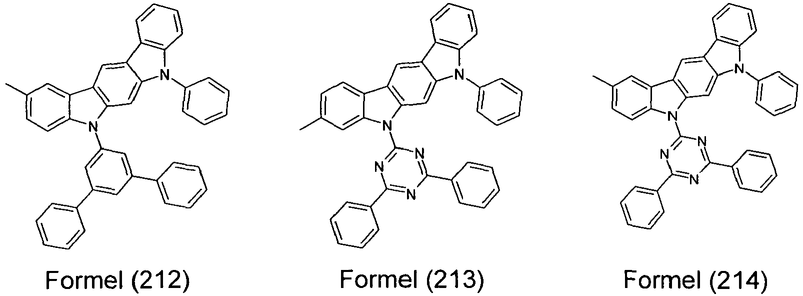 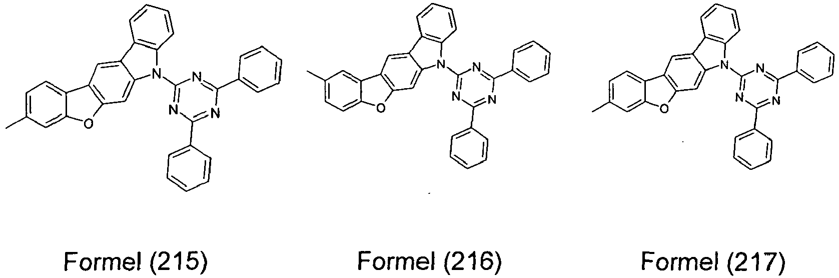 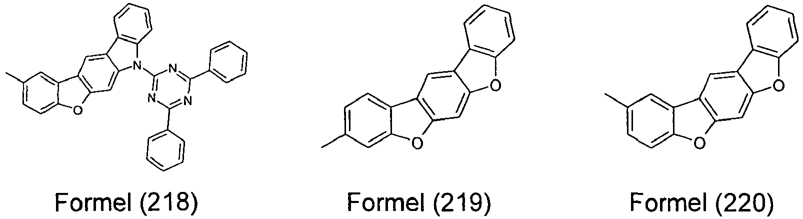 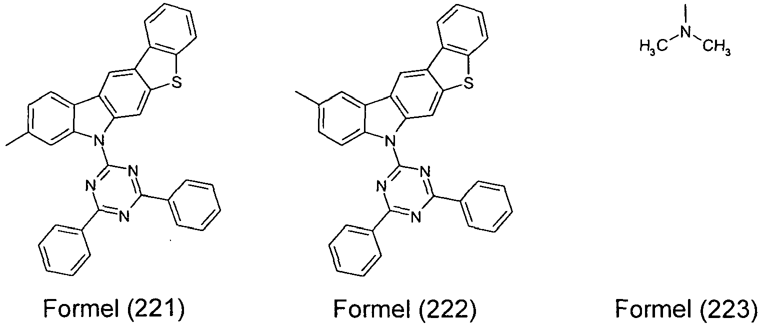 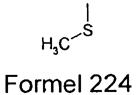 wobei die Linie die Verknüpfungsstelle des Restes R¹ zu dem Liganden angibt.

In einer insbesondere bevorzugten Ausführungsform der vorliegenden Erfindung ist der Rest R¹ gleich oder verscheiden bei jedem Auftreten H, NH₂ oder ein Rest der Formeln (86), (90), (91), (92), (93), (94), (97), (102), (106), (112), (117), (119), (144), (145), (147), (158), (159), (160), (161), (162), (163), (164), (165), (167), (172), (176), (179), (181), (188), (191), (192), (194), (196), (199), (202), (207), (223) und (224), wobei die angegebenen Formeln ihrerseits mit einem oder mehreren Resten R³ substituiert sein können, die bei jedem Auftreten gleich oder verschieden sein können.

In einer weiteren, insbesondere bevorzugten Ausführungsform der vorliegenden Erfindung ist der Rest R¹ gleich oder verschieden bei jedem Auftreten H, -CH₃ oder ein Rest der Formel (92), wobei die angegebenen Formel (92) ihrerseits mit einem oder mehreren Resten R³ substituiert sein können, die bei jedem Auftreten gleich oder verschieden sein können.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 60 C-Atome im Ringsystem. Ein heteroaromatisches Ringsystem im Sinne dieser Erfindung enthält 2 bis 60 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein sp³-hybridisiertes C-, N-oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer C₁- bis C₄₀-Alkylgruppe, in der auch einzelne H-Atome oder CH₂-Gruppen durch die oben genannten Gruppen substituiert sein können, beispielsweise die Reste Methyl, Ethyl, n-Propyl, i-Propyl, n-Butyl, i-Butyl, s-Butyl, t-Butyl, 2-Methylbutyl, n-Pentyl, s-Pentyl, tert-Pentyl, 2-Pentyl, Cyclopentyl, n-Hexyl, s-Hexyl, tert-Hexyl, 2-Hexyl, 3-Hexyl, Cyclohexyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Trifluormethyl, Pentafluorethyl oder 2,2,2-Trifluorethyl verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer C₁- bis C₄₀-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden. Unter einem aromatischen oder heteroaromatischen Ringsystem mit 5 - 60 aromatischen Ringatomen, welches noch jeweils mit den oben genannten Resten R substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Die erfindungsgemäßen Verbindungen können elektrisch geladen oder ungeladen sein. In einer bevorzugten Ausführungsform sind die erfindungsgemäßen Verbindungen elektrisch neutral. Dies wird auf einfache Weise dadurch erreicht, dass die Ladung der Liganden L so gewählt werden, dass sie die Ladung des komplexierten Metallatoms M kompensieren.

Bevorzugt sind weiterhin erfindungsgemäße Verbindungen, dadurch gekennzeichnet, dass die Summe der Valenzelektronen um das Metallatom für Platin und Palladium 16 und für Iridium oder Rhodium 18 beträgt.

Diese Bevorzugung ist durch die besondere Stabilität dieser Metallkomplexe begründet.

In einer bevorzugten Ausführungsform der Erfindung steht M für Iridium oder Platin. Besonders bevorzugt steht M für Iridium.

In einer bevorzugten Ausführungsform der Erfindung steht das Symbol X gleich oder verschieden bei jedem Auftreten für CR¹.

In einer weiteren bevorzugten Ausführungsform der Erfindung stehen entweder alle Symbole X gleich oder verschieden bei jedem Auftreten für CR¹ oder alle Symbole X stehen für N.

In einer weiteren bevorzugten Ausführungsform der Erfindung steht das Symbol Q gleich oder verschieden bei jedem Auftreten für R¹C=CR¹, R¹C=N, O oder NR¹, besonders bevorzugt für R¹C=CR¹ und R¹C=N und insbesondere bevorzugt für R¹C=CR¹.

Es ist besonders bevorzugt, wenn die oben genannten Bevorzugungen gleichzeitig gelten.

In einer besonders bevorzugten Ausführungsform der Erfindung gilt für die verwendeten Symbole:
- M: ist Iridium oder Platin, besonders bevorzugt Iridium;
- X: ist bei jedem Auftreten für alle Positionen, die nicht direkt an M gebunden sind, CR¹;
- Q: ist gleich oder verschieden bei jedem Auftreten R¹C=CR¹ oder R¹C=N, besonders bevorzugt R¹C=CR¹;
wobei die verwendeten Symbole und Indizes die oben genannten Bedeutungen aufweisen, insbesondere die oben genannten bevorzugten Bedeutungen.

An einem der Reste R¹ kann auch eine verbrückende Einheit Z vorhanden sein, die den Liganden L mit einem oder mehreren weiteren Liganden L verknüpft. In einer bevorzugten Ausführungsform der Erfindung ist statt einem der Reste R¹ eine verbrückende Einheit Z vorhanden, so dass die Liganden dreizähnigen oder mehrzähnigen oder polypodalen Charakter aufweisen. Es können auch zwei solcher verbrückenden Einheiten Z vorhanden sein. Dies führt zur Bildung makrocyclischer Liganden bzw. zur Bildung von Kryptaten.

Z stellt vorzugsweise eine verbrückende Einheit dar, enthaltend 1 bis 80 Atome aus der dritten, vierten, fünften und/oder sechsten Hauptgruppe (Gruppe 13, 14, 15 oder 16 gemäß IUPAC) oder einen 3- bis 6-gliedrigen Homo- oder Heterocyclus, die die Teilliganden L miteinander kovalent verbindet. Dabei kann die verbrückende Einheit Z auch unsymmetrisch aufgebaut sein, d. h. die Verknüpfung von Z zu L muss nicht identisch sein.

Die verbrückende Einheit Z kann neutral, einfach, zweifach oder dreifach negativ oder einfach, zweifach oder dreifach positiv geladen sein. Bevorzugt ist Z neutral oder einfach negativ oder einfach positiv geladen. Dabei wird die Ladung von Z bevorzugt so gewählt, dass insgesamt ein neutraler Komplex entsteht.

Wenn Z eine trivalente Gruppe ist, also drei Liganden L miteinander verbrückt, ist Z bevorzugt gleich oder verschieden bei jedem Auftreten gewählt aus der Gruppe bestehend aus B, B(R²)-, B(C(R²)₂)₃, (R²)B(C(R²)₂)₃⁻, B(O)₃, (R²)B(O)³-, B(C(R²)₂C(R²)₂)₃, (R²)B(C(R²)₂C(R²)₂)₃⁻, B(C(R²)₂O)₃, (R²)B(C(R²)₂O)₃⁻, B(OC(R²)₂)₃, (R²)B(OC(R²)₂)₃⁻, C(R²), CO⁻, CN(R²)₂, (R²)C(C(R²)₂)₃, (R²)C(O)₃, (R²)C(C(R²)₂C(R²)₂)₃, (R²)C(C(R²)₂O)₃, (R²)C(OC(R²)₂)₃, (R²)C(Si(R²)₂)₃, (R²)C(Si(R²)₂C(R²)₂)₃, (R²)C(C(R²)₂Si(R²)₂)₃, (R²)C(Si(R²)₂Si(R²)₂)₃, Si(R²), (R²)Si(C(R²)₂)₃, (R²)Si(O)₃, (R²)Si(C(R²)₂C(R²)₂)₃, (R²)Si(OC(R²)₂)₃, (R²)Si(C(R²)₂O)₃, (R²)Si(Si(R²)₂)₃, (R²)Si(Si(R²)₂C(R²)₂)₃, (R²)Si(C(R²)₂Si(R²)₂)₃, (R²)Si(Si(R²)₂Si(R²)₂)₃, N, NO, N(R²)⁺, N(C(R²)₂)₃, (R²)N(C(R²)₂)₃⁺, N(C=O)₃, N(C(R²)₂C(R²)₂)₃, (R²)N(C(R²)₂C(R²)₂)⁺, P, P(R²)⁺, PO, PS, PSe, PTe, P(O)₃, PO(O)₃, P(OC(R²)₂)₃, PO(OC(R²)₂)₃, P(C(R²)₂)₃, P(R²)(C(R²)₂)₃⁺, PO(C(R²)₂)₃, P(C(R²)₂C(R²)₂)₃, P(R²) (C(R²)₂C(R²)₂)₃⁺, PO(C(R²)₂C(R²)₂)₃, S⁺, S(C(R²)₂)₃⁺, S(C(R²)₂C(R²)₂)₃⁺, oder eine Einheit gemäß Formel (237), (238), (239) oder (240), 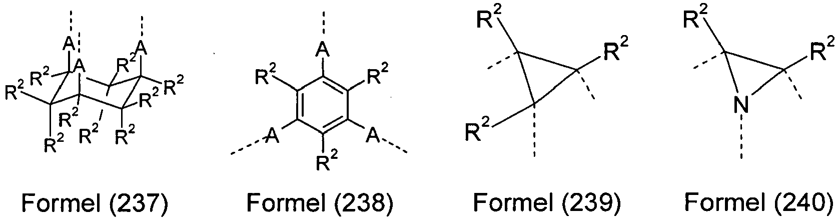 wobei die gestrichelten Bindungen jeweils die Bindung zu den Teilliganden L andeuten und A gleich oder verschieden bei jedem Auftreten ausgewählt ist aus der Gruppe bestehend aus einer Einfachbindung, O, S, S(=O), S(=O)₂, NR², PR², P(=O)R², P(=NR²), C(R²)₂, C(=O), C(=NR²), C(=C(R²)₂), Si(R²)₂ oder BR². Die weiteren verwendeten Symbole haben die oben genannten Bedeutungen.

Wenn Z eine bivalente Gruppe ist, also zwei Liganden L miteinander verbrückt, ist Z bevorzugt gleich oder verschieden bei jedem Auftreten gewählt aus der Gruppe bestehend aus BR², B(R²)₂⁻, C(R²)₂, C(=O), Si(R²)₂, NR², PR², P(R²)₂⁺, P(=O)(R²), P(=S)(R²), AsR², As(=O)(R²), As(=S)(R²), O, S, Se, oder eine Einheit gemäß Formel (241) bis (249), 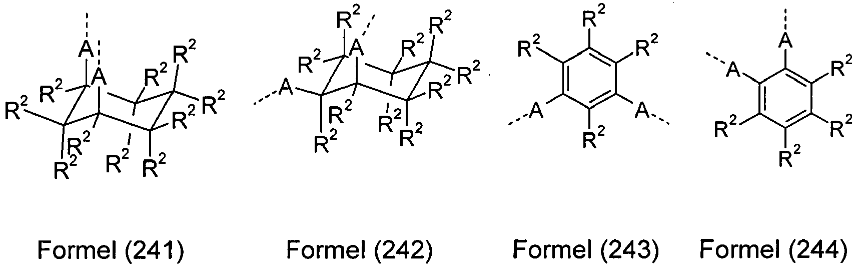 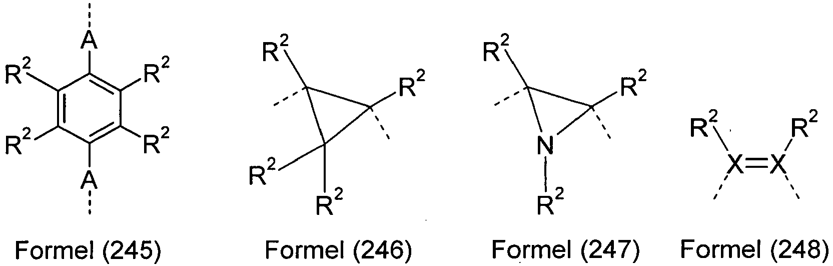 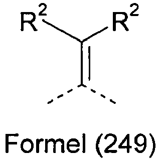 wobei die gestrichelten Bindungen jeweils die Bindung zu den Teilliganden L andeuten und die weiteren verwendeten Symbole jeweils die oben aufgeführten Bedeutungen haben.

Bevorzugte Reste R¹ in den oben aufgeführten Strukturen sind bei jedem Auftreten gleich oder verschieden ausgewählt aus der Gruppe bestehend aus H, D, F, Br, N(R²)₂, CN, B(OR²)₂, C(=O)R², P(=O)(R²)₂, einer geradkettige Alkylgruppe mit 1 bis 10 C-Atomen oder einer geradkettige Alkenyl- oder Alkinylgruppe mit 2 bis 10 C-Atomen oder einer verzweigten oder cyclischen Alkyl-, Alkenyl- oder Alkinylgruppe mit 3 bis 10 C-Atomen, die jeweils mit einem oder mehreren Resten R² substituiert sein kann, wobei ein oder mehrere H-Atome durch F ersetzt sein können, oder ein aromatisches oder heteroaromatisches Ringsystem mit 5 bis 14 aromatischen Ringatomen, das jeweils durch einen oder mehrere Reste R² substituiert sein kann; dabei können mehrere Reste R¹ auch miteinander ein mono- oder polycyclisches, aliphatisches, aromatisches und/oder benzoannelliertes Ringsystem bilden. Besonders bevorzugte Reste R¹ sind bei jedem Auftreten gleich oder verschieden ausgewählt aus der Gruppe bestehend aus H, F, Br, CN, B(OR²)₂, einer geradkettigen Alkylgruppe mit 1 bis 6 C-Atomen, insbesondere Methyl, oder einer verzweigten oder cyclischen Alkylgruppe mit 3 bis 10 C-Atomen, insbesondere iso-Propyl oder tert-Butyl, wobei ein oder mehrere H-Atome durch F ersetzt sein können, oder ein aromatisches oder heteroaromatisches Ringsystem mit 5 bis 12 aromatischen Ringatomen, das jeweils durch einen oder mehrere Reste R² substituiert sein kann; dabei können mehrere Reste R¹ auch miteinander ein mono- oder polycyclisches, aliphatisches, aromatisches und/oder benzoannelliertes Ringsystem bilden.

Die erfindungsgemäßen Metallkomplexe sind prinzipiell durch verschiedene Verfahren darstellbar. Es haben sich jedoch die im Folgenden beschriebenen Verfahren als besonders geeignet herausgestellt.

Daher ist ein weiterer Gegenstand der vorliegenden Erfindung ein Verfahren zur Herstellung der erfindungsgemäßen Metallkomplex-Verbindungen durch Umsetzung der entsprechenden freien Liganden mit Metallalkoholaten der Formel (282), mit Metallketoketonaten der Formel (283) oder mit Metallhalogeniden der Formel (284), 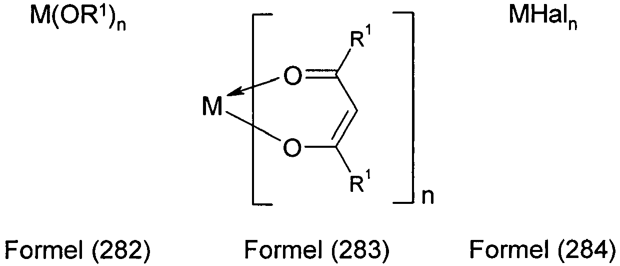 wobei die Symbole M, n und R¹ die oben angegebenen Bedeutungen haben und Hal = F, Cl, Br oder I ist.

Es können ebenfalls Metallverbindungen, insbesondere Iridiumverbindungen, die sowohl Alkoholat- und/oder Halogenid- und/oder Hydroxy- wie auch Ketoketonatreste tragen, verwendet werden. Diese Verbindungen können auch geladen sein. Entsprechende Iridiumverbindungen, die als Edukte besonders geeignet sind, sind in WO 2004/085449 offenbart. Besonders geeignet ist [IrCl₂(acac)₂]⁻, beispielsweise Na[IrCl₂(acac)₂].

Die Synthese der Komplexe wird bevorzugt durchgeführt wie in WO 2002/060910 und in WO 2004/085449 beschrieben. Dabei kann die Synthese beispielsweise auch thermisch, photochemisch und/oder durch Mikrowellenstrahlung aktiviert werden.

Durch diese Verfahren lassen sich die erfindungsgemäßen Verbindungen in hoher Reinheit, bevorzugt mehr als 99% (bestimmt mittels ¹H-NMR und/oder HPLC) erhalten.

Mit den hier erläuterten Synthesemethoden lassen sich unter anderem die im Folgenden dargestellten erfindungsgemäßen Verbindungen der Formeln (285) bis (383) herstellen.

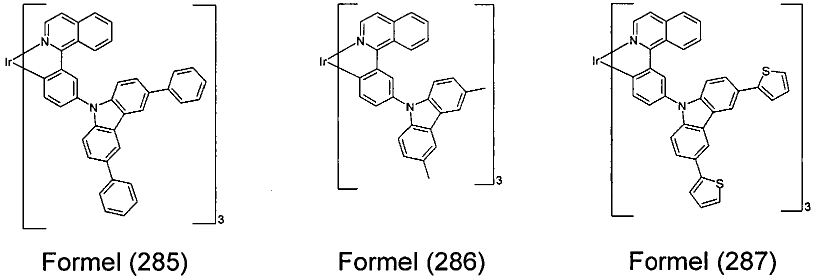

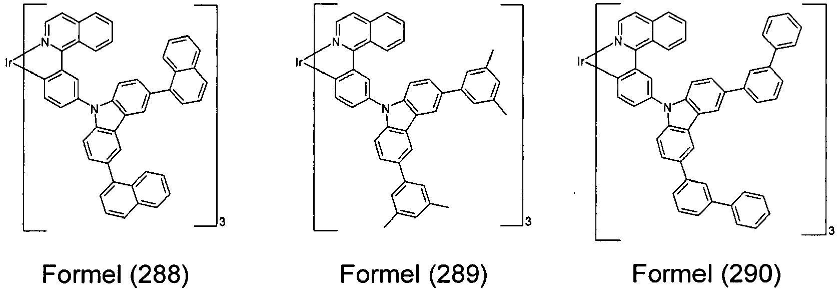

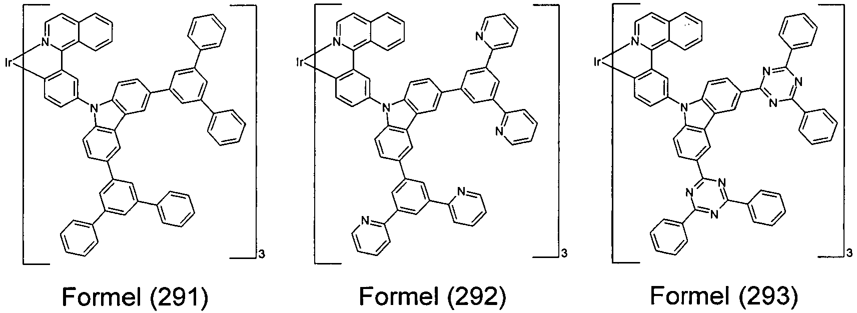

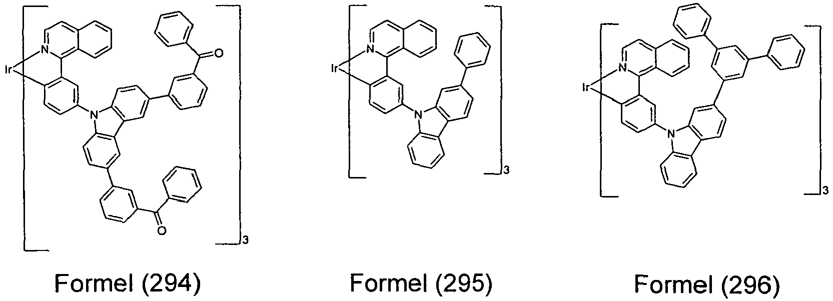

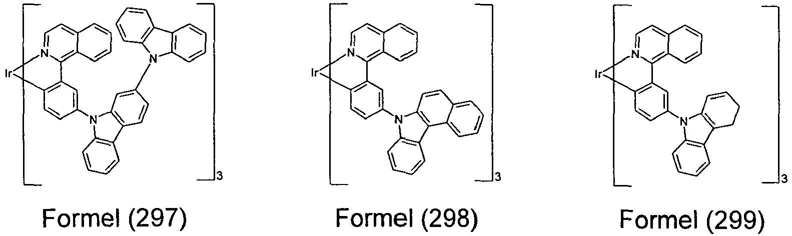

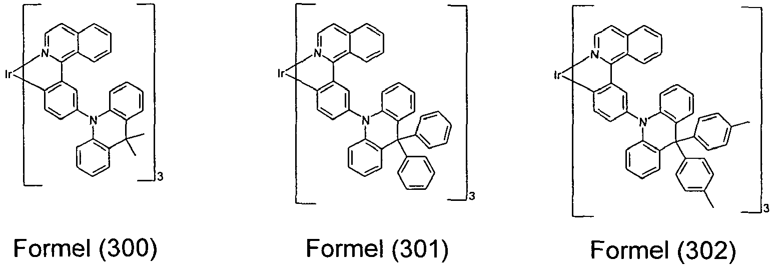

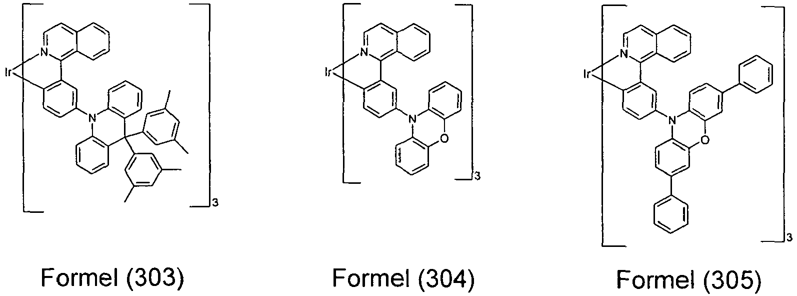

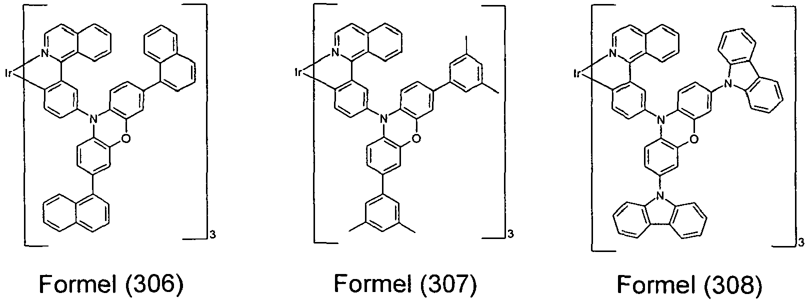

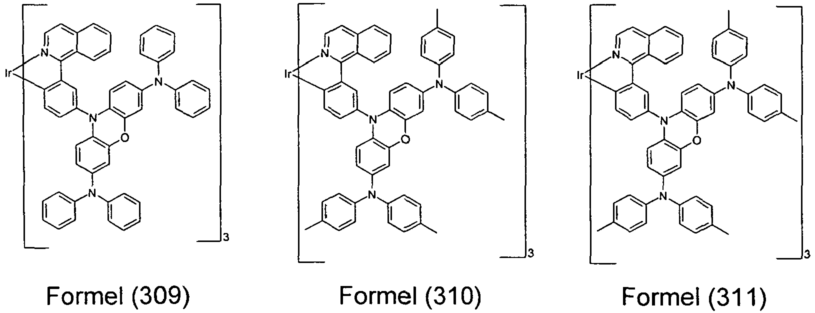

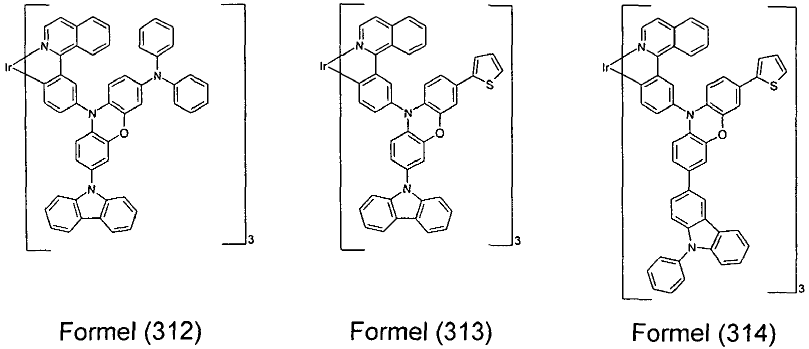

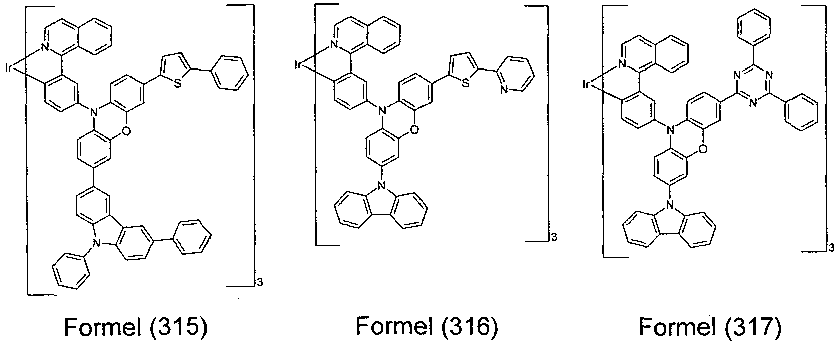

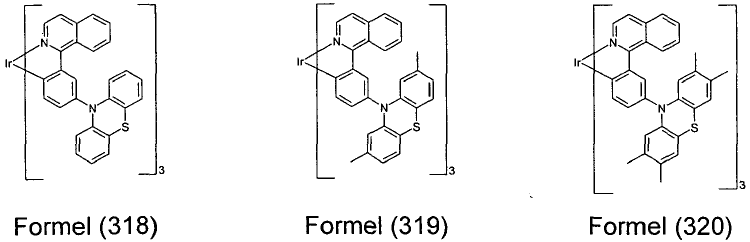

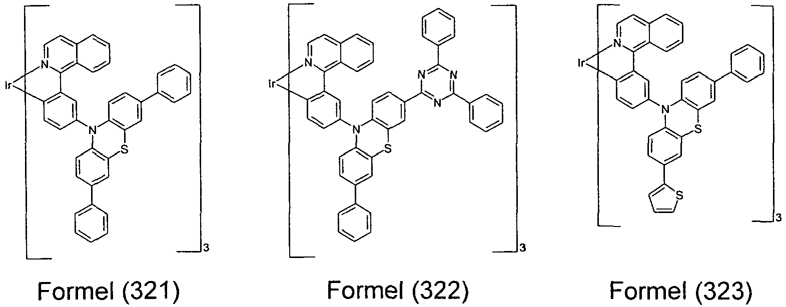

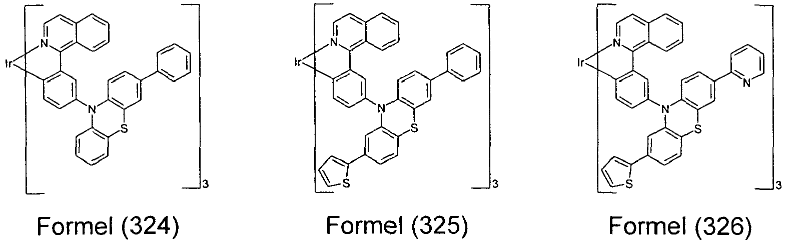

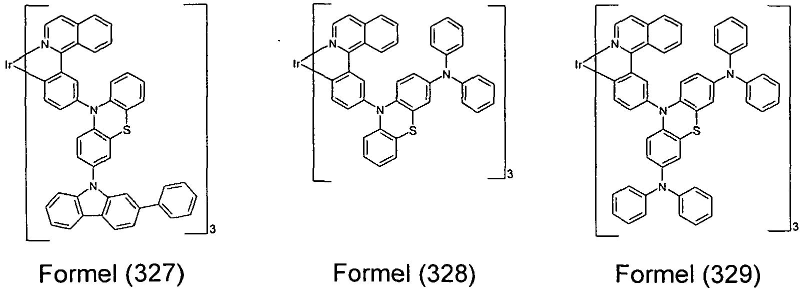

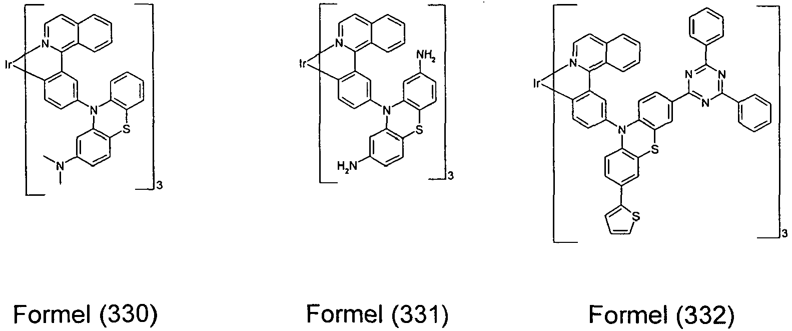

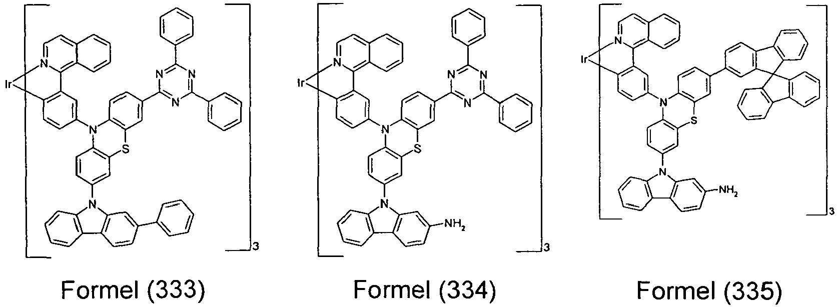

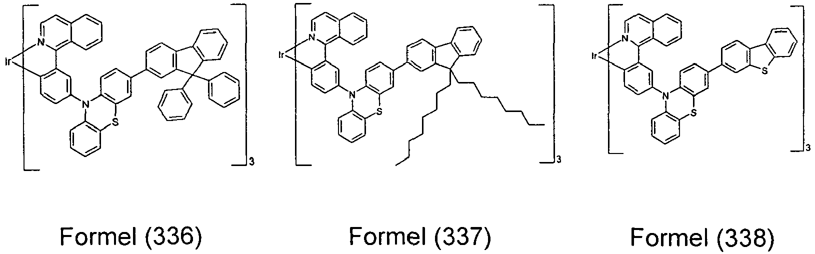

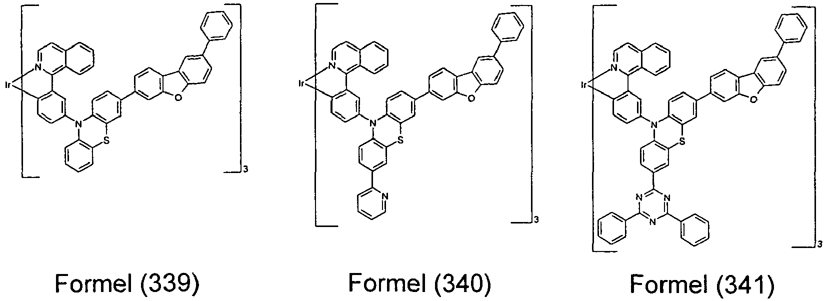

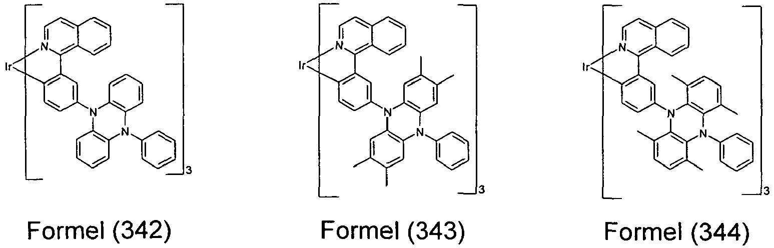

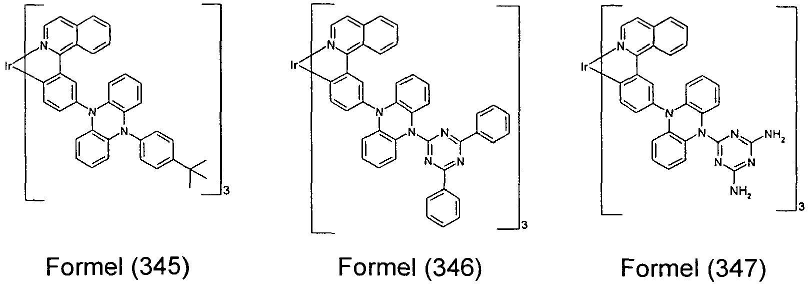

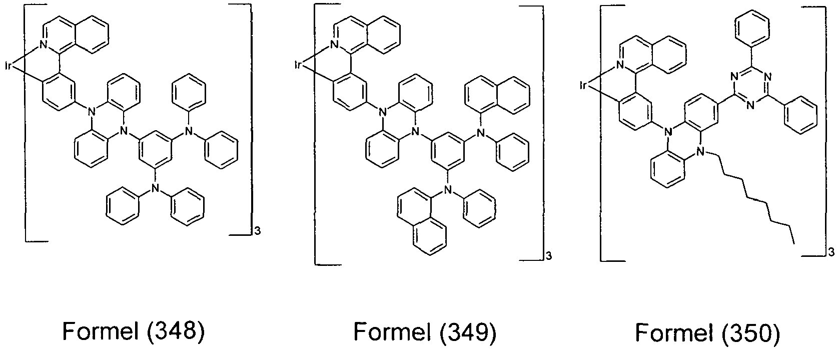

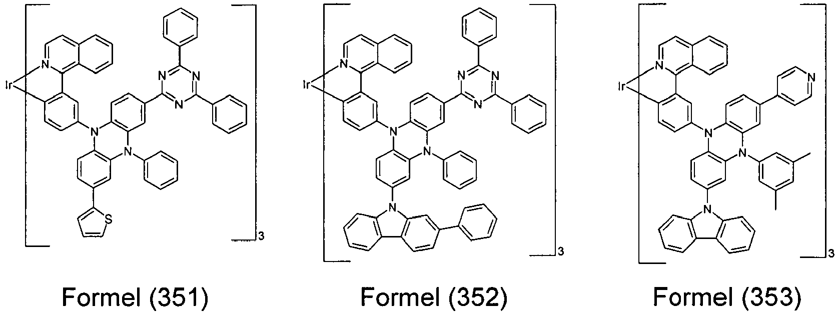

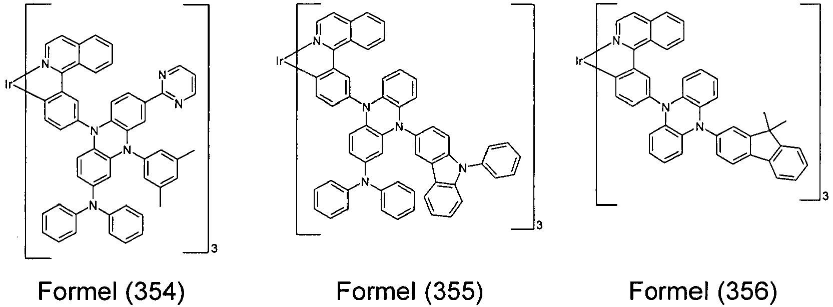

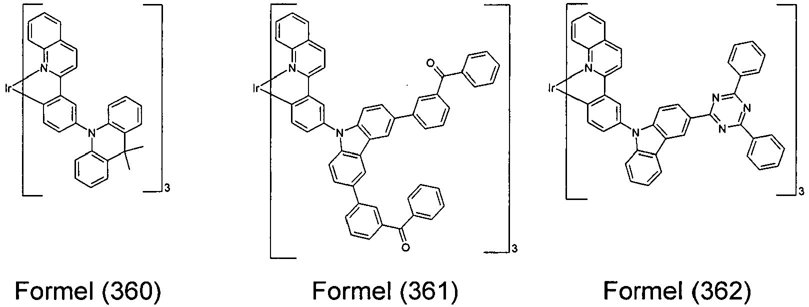

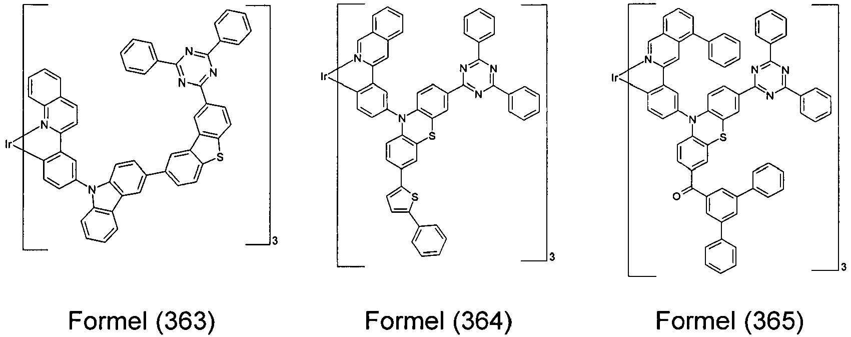

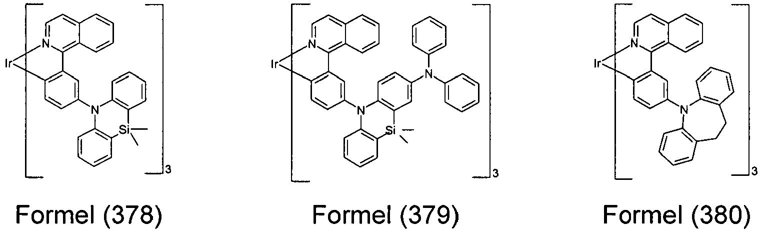

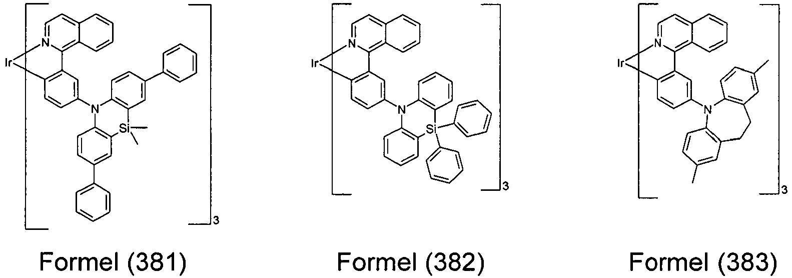

Die oben beschriebenen erfindungsgemäßen Komplexe bzw. die oben aufgeführten bevorzugten Ausführungsformen können in der elektronischen Vorrichtung als aktive Komponente verwendet werden. Unter einer elektronischen Vorrichtung wird eine Vorrichtung verstanden, welche Anode, Kathode und mindestens eine Schicht enthält, wobei diese Schicht mindestens eine organische bzw. metallorganische Verbindung enthält. Die erfindungsgemäße elektronische Vorrichtung enthält also Anode, Kathode und mindestens eine Schicht, welche mindestens eine erfindungsgemäße Verbindung der oben aufgeführten Formeln enthält. Dabei sind bevorzugte elektronische Vorrichtungen ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs) oder organischen Laserdioden (O-Laser), enthaltend in mindestens einer Schicht mindestens eine erfindungsgemäße Verbindung. Besonders bevorzugt sind organische Elektrolumineszenzvorrichtungen. Aktive Komponenten sind generell die organischen oder anorganischen Materialien, welche zwischen Anode und Kathode eingebracht sind, beispielsweise Ladungsinjektions-, Ladungstransport- oder Ladungsblockiermaterialien, insbesondere aber Emissionsmaterialien und Matrixmaterialien. Die erfindungsgemäßen Verbindungen zeigen besonders gute Eigenschaften als Emissionsmaterial in organischen Elektrolumineszenzvorrichtungen. Eine bevorzugte Ausführungsform der Erfindung sind daher organische Elektrolumineszenzvorrichtungen.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Excitonenblockierschichten, Ladungserzeugungsschichten und/oder organische oder anorganische p/n-Übergänge. Ebenso können zwischen zwei emittierende Schichten Interlayers eingebracht sein, welche beispielsweise eine Exzitonen-blockierende Funktion aufweisen und/oder die Ladungsbalance in der Elektrolumineszenzvorrichtung steuern. Es sei aber darauf hingewiesen, dass nicht notwendigerweise jede dieser Schichten vorhanden sein muss. Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 2005/011013) bzw. Systeme, welche mehr als drei emittierende Schichten aufweisen.

In einer bevorzugten Ausführungsform der Erfindung enthält die organische Elektrolumineszenzvorrichtung die erfindungsgemäße Verbindung bzw. die oben aufgeführten bevorzugten Ausführungsformen als emittierende Verbindung in einer oder mehreren emittierenden Schichten.

Wenn die erfindungsgemäße Verbindung als emittierende Verbindung in einer emittierenden Schicht eingesetzt wird, wird sie bevorzugt in Kombination mit einem oder mehreren Matrixmaterialien eingesetzt. Die Mischung aus der erfindungsgemäßen Verbindung und dem Matrixmaterial enthält zwischen 0.1 und 99 Gew.-%, vorzugsweise zwischen 0.5 und 40 Gew.-%, besonders bevorzugt zwischen 1 und 30 Gew.-%, insbesondere zwischen 2 und 25 Gew.-% der erfindungsgemäßen Verbindung bezogen auf die Gesamtmischung aus Emitter und Matrixmaterial. Entsprechend enthält die Mischung zwischen 99 und 1 Gew.-%, vorzugsweise zwischen 98 und 60 Gew.-%, besonders bevorzugt zwischen 97 und 70 Gew.-%, insbesondere zwischen 95 und 75 Gew.-% des Matrixmaterials bezogen auf die Gesamtmischung aus Emitter und Matrixmaterial.

Geeignete Matrixmaterialien für die erfindungsgemäßen Verbindungen sind Ketone, Phosphinoxide, Sulfoxide und Sulfone, z. B. gemäß WO 2004/013080, WO 2004/093207, WO 2006/005627 oder der Anmeldung DE 102008033943, Triarylamine, Carbazolderivate, z. B. CBP (N,N-Biscarbazolylbiphenyl) oder die in WO 2005/039246, US 2005/0069729, JP 2004/288381, EP 1205527 oder WO 2008/086851 offenbarten Carbazolderivate, Indolocarbazolderivate, z. B. gemäß WO 2007/063754 oder WO 2008/056746, Indenocarbazolderivate, z. B. gemäß den Anmeldungen DE 102009023155.2 und DE 102009031021.5, Azacarbazole, z. B. gemäß EP 1617710, EP 1617711, EP 1731584,JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Triazinderivate, z. B. gemäß der DE 102008036982.9, WO 2007/063754 oder WO 2008/056746, Zinkkomplexe, z. B. gemäß EP 652273 oder gemäß WO 09/062578, Diaza- oder Tetraazasilolderivate, z. B. gemäß der Anmeldung DE 102008056688.8, oder Diazaphospholderivate, z. B. gemäß der Anmeldung DE 102009022858.6.

Es kann auch bevorzugt sein, mehrere verschiedene Matrixmaterialien als Mischung einzusetzen, insbesondere mindestens ein elektronenleitendes Matrixmaterial und mindestens ein lochleitendes Matrixmaterial. Eine bevorzugte Kombination ist beispielsweise die Verwendung eines aromatischen Ketons oder eines Triazins mit einem Triarylamin-Derivat oder einem Carbazol-Derivat als gemischte Matrix für den erfindungsgemäßen Metallkomplex. Ebenfalls bevorzugt sind auch Mischungen aus einen loch- oder elektronentransportierenden Material mit einem Material, welches weder am Loch- noch am Elektronentransport beteiligt ist, wie beispielsweise in der Anmeldung DE 102009014513.3 offenbart.

In einer weiteren bevorzugten Ausführungsform der vorliegenden Erfindung können die erfindungsgemäßen Verbindungen in Mischungen mit einem oder mehreren weiteren Emittern eingesetzt werden. Ganz besonders bevorzugt ist dabei eine Mischung der erfindungsgemäßen Verbindungen mit einem oder mehreren fluoreszierenden Emittern. Weiterhin bevorzugt ist eine Mischung mit einem oder mehreren phosphoreszierenden Emittern. Dabei emittieren fluoreszierende Emitter hauptsächlich aus angeregten Singulett-Zuständen, wohingegen phosphoreszierende Emitter hauptsächlich aus höheren Spinzuständen (z.B. Triplett und Quintett) Licht emittieren. Die Komplexe organischer Übergangsmetalle werden im Sinne dieser Erfindung als phosphoreszierende Emitter verstanden. Vorzugsweise sind die weiteren Emitter organische Verbindungen.

In einer besonders bevorzugten Ausführungsform der vorliegenden Erfindung werden die erfindungsgemäßen Verbindungen mit 3 weiteren, in einer insbesondere bevorzugten Ausführungsform mit 2 weiteren und in einer ganz besonders bevorzugten Ausführungsform mit einem weiteren Emitter gemischt.

In einer weiteren bevorzugten Ausführungsform der vorliegenden Erfindung enthalten die Emitter-Mischungen 3, insbesondere bevorzugt 2 und ganz besonders bevorzugt eine erfindungsgemäße Verbindung.

In einer besonders bevorzugten Ausführungsform der vorliegenden Erfindung enthalten die Emitter-Mischungen genau eine der erfindungsgemäßen Verbindung und genau einen weiteren Emitter.

Es ist weiterhin bevorzugt im Sinne der vorliegenden Erfindung, dass die Absorptionsspektren wenigstens eines Emitters und das Emissionsspektrum wenigstens eines anderen Emitters der Mischung überlappen, so dass ein Energietransfer (Double Doping) zwischen den Emittern erleichtert wird. Der Energietransfer kann dabei nach unterschiedlichen Mechanismen erfolgen. Nicht abschließende Beispiele hierfür sind der Energietransfer nach Förster oder Dexter.

Die beschriebenen Emitter-Mischungen enthalten bevorzugt wenigstens zwei Emitter, die beide rotes Licht emittieren. Weiterhin bevorzugt sind Emitter-Mischungen enthaltend wenigstens zwei Emitter, die beide grünes Licht emittieren. Weiterhin bevorzugt sind Emitter-Mischungen enthaltend wenigstens einen Emitter, der rotes Licht emittiert und wenigstens einen Emitter, der grünes Licht emittiert.

Als Kathode sind Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lathanoide (z. B. Ca, Ba, Mg, Al, In, Mg, Yb, Sm, etc.). Weiterhin eignen sich Legierungen aus einem Alkali- oder Erdalkalimetall und Silber, beispielsweise eine Legierung aus Magnesium und Silber. Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide oder Carbonate in Frage (z. B. LiF, Li₂O, BaF₂, MgO, NaF, CsF, Cs₂CO₃, etc.). Die Schichtdicke dieser Schicht beträgt bevorzugt zwischen 0.5 und 5 nm.

Als Anode sind Materialien mit hoher Austrittsarbeit bevorzugt. Bevorzugt weist die Anode eine Austrittsarbeit größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektronen (z. B. Al/Ni/NiOₓ, Al/PtOₓ) bevorzugt sein. Für einige Anwendungen muss mindestens eine der Elektroden transparent sein, um entweder die Bestrahlung des organischen Materials (O-SC) oder die Auskopplung von Licht (OLED/PLED, O-LASER) zu ermöglichen. Ein bevorzugter Aufbau verwendet eine transparente Anode. Bevorzugte Anodenmaterialien sind hier leitfähige gemischte Metalloxide. Besonders bevorzugt sind Indium-Zinn-Oxid (ITO) oder Indium-Zink Oxid (IZO). Bevorzugt sind weiterhin leitfähige, dotierte organische Materialien, insbesondere leitfähige dotierte Polymere.

In den weiteren Schichten können generell alle Materialien verwendet werden, wie sie gemäß dem Stand der Technik für die Schichten verwendet werden und der Fachmann kann ohne erfinderisches Zutun jedes dieser Materialien in einer elektronischen Vorrichtung mit den erfindungsgemäßen Materialien kombinieren.

Die Vorrichtung wird entsprechend (je nach Anwendung) strukturiert, kontaktiert und schließlich hermetisch versiegelt, da sich die Lebensdauer derartiger Vorrichtungen bei Anwesenheit von Wasser und/oder Luft drastisch verkürzt.

Weiterhin bevorzugt ist eine organische Elektrolumineszenzvorrichtung, dadurch gekennzeichnet, dass eine oder mehrere Schichten mit einem Sublimationsverfahren beschichtet werden. Dabei werden die Materialien in Vakuum-Sublimationsanlagen bei einem Anfangsdruck von üblicherweise kleiner 10⁻⁵ mbar, bevorzugt kleiner 10⁻⁶ mbar aufgedampft. Es ist auch möglich, dass der Anfangsdruck noch geringer ist, beispielsweise kleiner 10⁻⁷ mbar.

Bevorzugt ist ebenfalls eine organische Elektrolumineszenzvorrichtung, dadurch gekennzeichnet, dass eine oder mehrere Schichten mit dem OVPD (Organic Vapour Phase Deposition) Verfahren oder mit Hilfe einer Trägergassublimation beschichtet werden. Dabei werden die Materialien bei einem Druck zwischen 10⁻⁵ mbar und 1 bar aufgebracht. Ein Spezialfall dieses Verfahrens ist das OVJP (Organic Vapour Jet Printing) Verfahren, bei dem die Materialien direkt durch eine Düse aufgebracht und so strukturiert werden (z. B. M. S. Arnold et al., Appl. Phys. Lett. 2008, 92, 053301).

Weiterhin bevorzugt ist eine organische Elektrolumineszenzvorrichtung, dadurch gekennzeichnet, dass eine oder mehrere Schichten aus Lösung, wie z. B. durch Spincoating, oder mit einem beliebigen Druckverfahren, wie z. B. Siebdruck, Flexodruck oder Offsetdruck, besonders bevorzugt aber LITI (Light Induced Thermal Imaging, Thermotransferdruck) oder Ink-Jet Druck (Tintenstrahldruck), hergestellt werden. Da die erfindungsgemäßen Verbindungen eine sehr gute Löslichkeit in organischen Lösemitteln aufweisen, eignen sie sich besonders gut für die Verarbeitung aus Lösung.

Die organische Elektrolumineszenzvorrichtung kann auch als Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend eine erfindungsgemäße Verbindung und ein Matrixmaterial aus Lösung aufzubringen und darauf eine Lochblockierschicht und/oder eine Elektronentransportschicht im Vakuum aufzudampfen.

Diese Verfahren sind dem Fachmann generell bekannt und können von ihm ohne Probleme auf organische Elektrolumineszenzvorrichtungen enthaltend die erfindungsgemäßen Verbindungen bzw. die oben aufgeführten bevorzugten Ausführungsformen angewandt werden.

Für die Verarbeitung aus Lösung sind Lösungen bzw. Formulierungen der erfindungsgemäßen Verbindungen erforderlich. Es kann auch bevorzugt sein, Mischungen aus zwei oder mehr Lösemitteln zu verwenden. Geeignete und bevorzugte Lösemittel sind beispielsweise Toluol, Anisol, o-, m- oder p-Xylol, Methylbenzoat, Dimethylanisol, Mesitylen, Tetralin, Veratrol, THF, Methyl-THF, THP, Chlorbenzol, Dioxan oder Mischungen dieser Lösemittel.

Ein weiterer Gegenstand der vorliegenden Erfindung ist daher eine Lösung bzw. eine Formulierung enthaltend mindestens eine erfindungsgemäße Verbindung und ein oder mehrere Lösemittel, insbesondere organische Lösemittel. Wie solche Lösungen hergestellt werden können, ist dem Fachmann bekannt und beispielsweise in der WO 2002/072714, der WO 03/019694 und der darin zitierten Literatur beschrieben.

Die erfindungsgemäßen elektronischen Vorrichtungen, insbesondere organische Elektrolumineszenzvorrichtungen, zeichnen sich durch folgende überraschende Vorteile gegenüber dem Stand der Technik aus:
1. Die erfindungsgemäßen Verbindungen weisen eine sehr gute Löslichkeit in einer Vielzahl gängiger organischer Lösemittel auf und sind daher sehr gut für die Verarbeitung aus Lösung geeignet. Insbesondere weisen die erfindungsgemäßen Verbindungen eine höhere Löslichkeit auf als die im Stand der Technik beschriebenen ähnlichen Verbindungen.
2. Organische Elektrolumineszenzvorrichtungen enthaltend die erfindungsgemäßen Verbindungen als emittierende Materialien weisen eine exzellente Lebensdauer auf. Insbesondere ist die Lebensdauer besser als bei ähnlichen Verbindungen gemäß dem Stand der Technik.
3. Organische Elektrolumineszenzvorrichtungen enthaltend die erfindungsgemäßen Verbindungen als emittierende Materialien weisen eine hervorragende Effizienz auf. Insbesondere ist die Effizienz besser als bei ähnlichen Verbindungen gemäß dem Stand der Technik.

Diese oben genannten Vorteile gehen nicht mit einer Verschlechterung der weiteren elektronischen Eigenschaften einher.

Die erfindungsgemäßen Verbindungen sind in der Lage unter bestimmten Voraussetzungen Licht zu emittieren. Somit sind diese Verbindungen sehr vielseitig einsetzbar. Einige der Hauptanwendungsgebiete sind dabei Display- oder Beleuchtungs-Technologien. Weiterhin ist es besonders vorteilhaft, die Verbindungen sowie Vorrichtungen enthaltend diese Verbindungen im Bereich der Phototherapie einzusetzen.

Ein weiterer Gegenstand der vorliegenden Erfindung bezieht sich daher auf die Verwendung der erfindungsgemäßen Verbindungen und Vorrichtungen enthaltend die Verbindungen zur Behandlung, Prophylaxe und Diagnose von Erkrankungen. Noch ein weiterer Gegenstand der vorliegenden Erfindung bezieht sich auf die Verwendung, der erfindungsgemäßen Verbindungen und Vorrichtungen enthaltend die Verbindungen zur Behandlung und Prophylaxe kosmetischen Umstände.

Ein weiterer Gegenstand der vorliegenden Erfindung bezieht sich auf die erfindungsgemäßen Verbindungen zu Herstellung von Vorrichtungen zur Therapie, Prophylaxe und/oder Diagnose therapeutischer Erkrankungen.

Dabei sind viele Erkrankungen sind mit kosmetischen Aspekten assoziiert. So leidet ein Patient mit schwerer Akne in der Gesichtspartie nicht nur an den medizinischen Ursachen und Folgen der Erkrankung, sondern auch an den kosmetischen Begleitumständen.

Phototherapie oder Lichttherapie findet in vielen medizinischen und/oder kosmetischen Bereichen Anwendung. Die erfindungsgemäßen Verbindungen sowie die Vorrichtungen enthaltend diese Verbindungen können daher zur Therapie und/oder Prophylaxe und/oder Diagnose von allen Erkrankungen und/oder in kosmetischen Anwendungen eingesetzt werden, für die der Fachmann die Anwendung von Phototherapie in Betracht zieht. Der Begriff Phototherapie beinhaltet dabei neben der Bestrahlung auch die photodynamischen Therapie (PDT) sowie das Desinfizieren und Sterilisieren im Allgemeinen. Behandelt werden können mittels Phototherapie oder Lichttherapie nicht nur Menschen oder Tiere, sondern auch jegliche andere Art lebender oder unbelebter Materie. Hierzu gehören, bspw., Pilze, Bakterien, Mikroben, Viren, Eukaryonten, Prokaryonten, Nahrungsmittel, Getränke, Wasser und Trinkwasser.

Der Begriff Phototherapie beinhaltet auch jede Art der Kombination von Lichttherapie und anderen Therapiearten, wie bspw. die Behandlung mit Wirkstoffen. Viele Lichttherapien habe zum Ziel äußere Partien eines Objektes zu bestrahlen oder zu behandeln, so wie die Haut von Menschen und Tieren, Wunden, Schleimhäute, Auge, Haare, Nägel, das Nagelbett, Zahnfleisch und die Zunge. Die erfindungsgemäße Behandlung oder Bestrahlung kann daneben auch innerhalb eines Objektes durchgeführt werden, um bspw. innere Organe (Herz, Lunge etc.) oder Blutgefäße oder die Brust zu behandeln.

Die erfindungsgemäßen therapeutischen und/oder kosmetischen Anwendungsgebiete sind bevorzugt ausgewählt aus der Gruppe der Hauterkrankungen und Haut-assoziierten Erkrankungen oder Veränderungen bzw. Umstände wie bspw. Psoriasis, Hautalterung, Hautfaltenbildung, Hautverjüngung, vergrößerte Hautporen, Cellulite, ölige/fettige Haut, Follikulitis, aktinische Keratose, precancerose aktinische Keratose, Haut Läsionen, sonnengeschädigte und sonnengestresste Haut, Krähenfüße, Haut Ulkus, Akne, Akne rosacea, Narben durch Akne, Akne Bakterien, Photomodulierung fettiger/öliger Talgdrüsen sowie deren umgebende Gewebe, Ikterus, Neugeborenenikterus, Vitiligo, Hautkrebs, Hauttumore, Crigler Naijar, Dermatitis, atopische Dermatitis, diabetische Hautgeschwüre sowie Desensibilisierung der Haut.

Besonders bevorzugt im Sinne der Erfindung sind die Behandlung und/oder Prophylaxe von Psoriasis, Akne, Cellulite, Hautfaltenbildung, Hautalterung, Ikterus und Vitiligo.
Weitere erfindungsgemäße Anwendungsgebiete für die Zusammensetzungen und/oder Vorrichtungen enthaltend die erfindungsgemäßen Zusammensetzungen sind ausgewählt aus der Gruppe der Entzündungserkrankungen, rheumatoide Arthritis, Schmerztherapie, Behandlung von Wunden, neurologische Erkrankungen und Umstände, Ödeme, Paget's Erkrankung, primäre und metastasierende Tumoren, Bindegewebserkrankungen bzw. -Veränderungen, Veränderungen des Kollagens, Fibroblasten und von Fibroblasten stammende Zellspiegel in Geweben von Säugetieren, Bestrahlung der Retina, neovasculare und hypertrophe Erkrankungen, allergische Reaktionen, Bestrahlung der Atemwege, Schwitzen, okulare neovaskulare Erkrankungen, virale Infektionen besonders Infektionen durch Herpes Simplex oder HPV (Humane Papillomviren) zur Behandlung von Warzen und Genitalwarzen.

Besonders bevorzugt im Sinne der Erfindung sind die Behandlung und/oder Prophylaxe von rheumatoider Arthritis, viraler Infektionen, und Schmerzen.

Weitere erfindungsgemäße Anwendungsgebiete für die Verbindungen und/oder Vorrichtungen enthaltend die erfindungsgemäßen Verbindungen sind ausgewählt aus der Winterdepression, Schlafkrankheit, Bestrahlung zur Verbesserung der Stimmung, Linderung von Schmerzen besonders Muskelschmerzen durch bspw. Verspannungen oder Gelenkschmerzen, Beseitigung der Steifheit von Gelenken und das Aufhellen der Zähne (Bleaching).

Weitere erfindungsgemäße Anwendungsgebiete für die Verbindungen und/oder Vorrichtungen enthaltend die erfindungsgemäßen Verbindungen sind ausgewählt aus der Gruppe der Desinfektionen. Mit den erfindungsgemäßen Verbindungen und/oder mit den erfindungsgemäßen Vorrichtungen können jegliche Art von Objekten (unbelebte Materie) oder Subjekten (lebende Materie wie bspw. Mensch und Tier) zum Zweck der Desinfektion behandelt werden. Hierzu zählt, zum Beispiel, die Desinfektion von Wunden, die Reduktion von Bakterien, das Desinfizieren chirurgischer Instrumente oder anderer Gegenstände, das Desinfizieren von Nahrungs- und Lebensmitteln, von Flüssigkeiten, insbesondere Wasser, Trinkwasser und andere Getränke, das Desinfizieren von Schleimhäuten und Zahnfleisch und Zähnen. Unter Desinfektion wird hierbei die Reduktion lebender mikrobiologischer Verursacher unerwünschter Effekte, wie Bakterien und Keime, verstanden.

Zu dem Zweck der oben genannten Phototherapie emittieren Vorrichtungen enthaltend die erfindungsgemäßen Verbindungen bevorzugt Licht der Wellenlänge zwischen 250 and 1250 nm, besonders bevorzugt zwischen 300 and 1000 nm und insbesondere bevorzugt zwischen 400 and 850 nm.

In einer besonders bevorzugten Ausführungsform der vorliegenden Erfindung werden die erfindungsgemäßen Verbindungen in einer organischen lichtemittierenden Diode (OLED) oder einer organischen lichtemittierenden elektrochemischen Zelle (OLEC) zum Zwecke der Phototherpie eingesetzt. Sowohl die OLED als auch die OLEC können dabei einen planaren oder Fiber- bzw. Faser-artigen Aufbau mit beliebigem Querschnitt (z.B. rund, oval, polygonal, quadratisch) mit einem ein- oder mehrschichtigen Aufbau aufweisen. Diese OLECs und/oder OLEDs können in andere Vorrichtungen eingebaut werden, die weitere mechanische, adhäsive und/oder elektronische Bausteine (z.B. Batterie und/oder Steuerungseinheit zur Einstellung der Bestrahlungszeiten, -intensitäten und -wellenlängen) enthalten. Diese Vorrichtungen enthaltend die erfindungsgemäßen OLECs und/order OLEDs sind vorzugsweise ausgewählt aus der Gruppe enthaltend Pflaster, Pads, Tapes, Bandagen, Manschetten, Decken, Hauben, Schlafsäcken,Textilien und Stents.

Die Verwendung von den genannten Vorrichtungen zu dem genannten therapeutischen und/oder kosmetischen Zweck ist besonders vorteilhaft gegenüber dem Stand der Technik, da mit Hilfe der erfindungsgemäßen Vorrichtungen unter Verwendung der OLEDs und/oder OLECs homogene Bestrahlungen geringerer Bestrahlungsintensitäten an nahezu jedem Ort und zu jeder Tageszeit möglich sind. Die Bestrahlungen können stationär, ambulant und/oder selbst, d.h., ohne Einleitung durch medizinisches oder kosmetisches Fachpersonal durchgeführt werden. So können, bspw., Pflaster unter der Kleidung getragen werden, so dass eine Bestrahlung auch während der Arbeitszeit, in der Freizeit oder während des Schlafes möglich ist. Auf aufwendige stationäre/ambulante Behandlungen mit kann in vielen Fällen verzichtet bzw. deren Häufigkeit reduziert werden. Die erfindungsgemäßen Vorrichtungen können zum Widergebrauch gedacht sein oder Wegwerfartikel darstellen, die nach ein-, zwei oder dreimaligem Gebrauch entsorgt werden können.

Weitere Vorteile gegenüber dem Stand der Technik sind bspw. eine geringere Wärmeentwicklung und emotionale Aspekte. So werden Neugeborene, die aufgrund einer Gelbsucht (Ikterus) therapiert werden müssen typischerweise mit verbundenen Augen in einem Brutkasten, ohne körperlichen Kontakt zur den Eltern bestrahlt, was eine emotionale Stresssituation für Eltern und Neugeborene darstellt. Mit Hilfe einer erfindungsgemäßen Decke enthaltend die erfindungsgemäßen OLEDs und/oder OLECs kann der emotionale Stress signifikant vermindert werden. Zudem ist eine bessere Temperierung des Kindes durch eine verringerte Wärmeproduktion der erfindungsgemäßen Vorrichtungen gegenüber herkömmlicher Bestrahlungsgeräte möglich.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen.

### Beispiele

Die nachfolgenden Synthesen werden, sofern nicht anders angegeben, unter einer Schutzgasatmosphäre in getrockneten Lösungsmitteln durchgeführt. Die Edukte IV und XI sowie die verwendeten Lösungsmittel können kommerziell bezogen werden, beispielsweise von ALDRICH. Verbindungen I und X können analog zu WO 02/068435 dargestellt werden. Verbindung II kann nach Synthesis, 2005, 1619-1624 dargestellt werden. Verbindung VI kann nach Chem. Ber., 1984, 117, 2703-2713 dargestellt werden. Verbindung VIII kann nach Bioorg. Med. Chem. Lett. 2007, 17, 3014-3017 dargestellt werden.

### Beispiel 1:

### Herstellung der Verbindung III

### Syntheseprozedur für die Darstellung von Verbindung III:

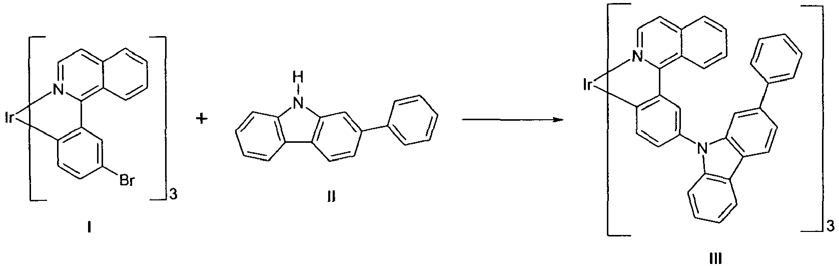

7.0 g (6.7 mmol) Verbindung I, 13.1 g (53.8 mmol) Verbindung II und 57 g (269 mmol) K₃PO₄ werden in 850 mL p-Xylol, suspendiert. Zu dieser Suspension werden 45 mg (0.20 mmol) Pd(OAc)₂ und 2.7 ml eine 1 M Tritert-butylphosphin Lösung gegeben. Die Reaktionsmischung wird 42 h unter Rückfluss erhitzt. Nach Erkalten wird die organische Phase abgetrennt, dreimal mit 125 mL Wasser gewaschen und anschließend zur Trockene eingeengt. Der Rückstand wird mit Ethanol gewaschen und aus Toluol umkristallisiert und abschließend bei vermindertem Druck getrocknet. Die Ausbeute beträgt 6.3 g (4.1 mmol), entsprechend 61.3% der Theorie.

### Beispiel 2:

### Herstellung der Verbindung V

Syntheseprozedur für die Darstellung von Verbindung **V:**

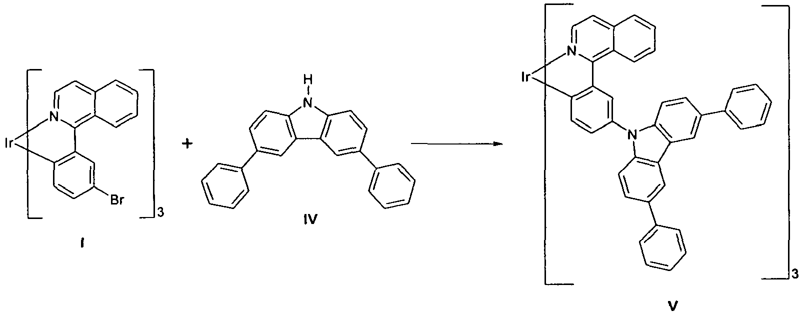

Die Synthese wird analog zu der von Verbindung **III** mit 6.2 g (6.0 mmol) Verbindung I durchgeführt, wobei die Verbindung **II** aus Beispiel 1 durch 15.21 g (47.6 mmol) 3,6-Diphenyl-9H-carbazol (Verbindung **IV**) ersetzt wird. Die Ausbeute beträgt 7.2 g (4,1 mmol), entsprechend 68.8% der Theorie.

### Beispiel 3:

### Herstellung der Verbindung VII

Syntheseprozedur für die Darstellung von Verbindung **VII:**

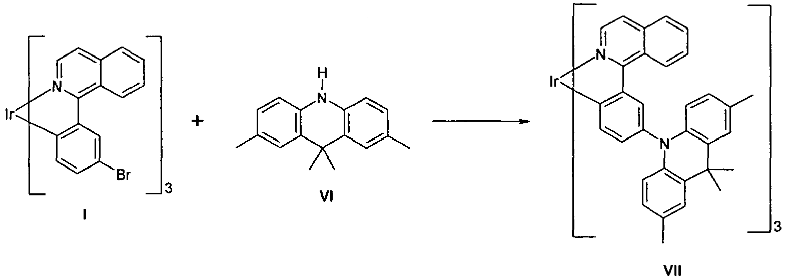

Die Synthese wird analog zu der von Verbindung **III** mit 5.8 g (5.6 mmol) Verbindung **I** durchgeführt, wobei die Verbindung **II** aus Beispiel 1 durch 10.6 g (44.5 mmol) 2,7,9,9-Tetramethyl-9,10-dihydro-acridin (Verbindung **VI**) ersetzt wird. Die Ausbeute beträgt 4.5 g (3.0 mmol), entsprechend 53.5% der Theorie.

### Beispiel 4:

### Herstellung der Verbindung IX

Syntheseprozedur für die Darstellung von Verbindung **IX:**

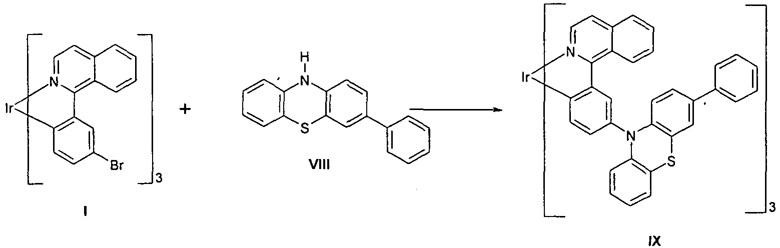

Die Synthese wird analog zu der von Verbindung **III** mit 6.2 g (6.0 mmol) Verbindung **I** durchgeführt, wobei die Verbindung **II** aus Beispiel 1 durch 13.1 g (47.6 mmol) 3-Phenyl-10H-phenothiazin ersetzt wird. Die Ausbeute beträgt 5.7 g (3.5 mmol), entsprechend 58.9% der Theorie

### Beispiel 5:

### Herstellung der Verbindung XII

Syntheseprozedur für die Darstellung von Verbindung **XII:**

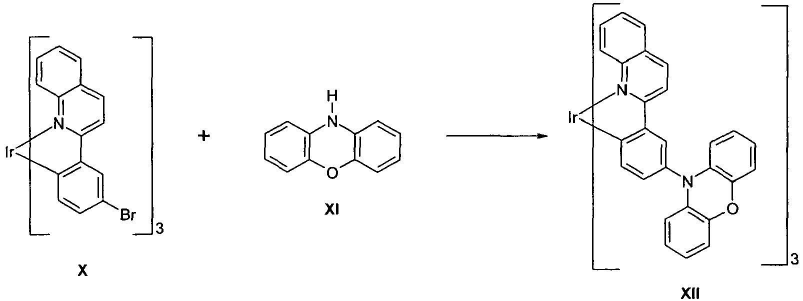

Die Synthese wird analog zu der von Verbindung **III** durchgeführt, wobei die Verbindung I durch 8.5 g (8.2 mmol) Verbindung **X** und die Verbindung **II** durch 12.0 g (65.3 mmol) 10H-Phenoxazin (Verbindung **XI**) ersetzt werden. Die Ausbeute beträgt 6.9 g (5.1 mmol), entsprechend 62.7% der Theorie.

### Beispiele 6 bis 10:

### Herstellung und Charakterisierung von organischen Elektrolumineszenzvorrichtungen enthaltend die erfindungsgemäßen Verbindungen

Die Strukturen von den erfindungsgemäßen Verbindungen T-1 bis T-5, TMM 1 (synthetisiert nach DE 102008036982.9) und TMM-2 (synthetisiert nach DE 102008017591.9) sind der Über¬sichtlichkeit halber im Folgenden abgebildet.

### Strukturen der Emitter

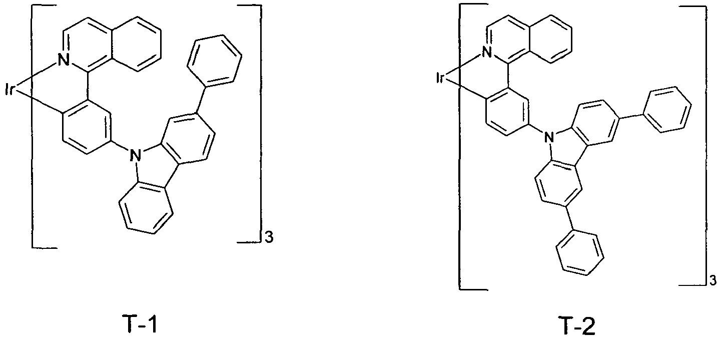

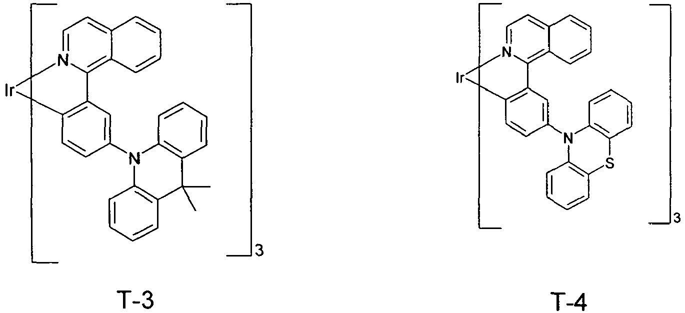

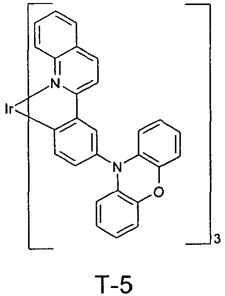

### Strukturen der Matrices

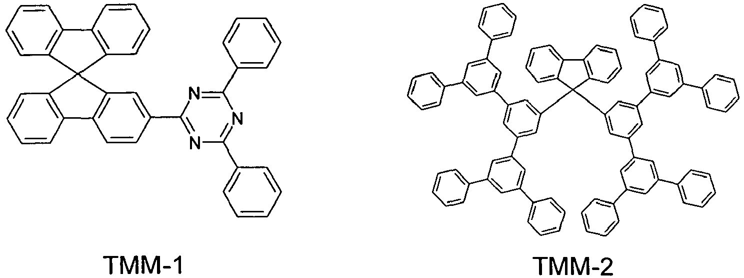

Erfindungsgemäße Materialien können aus Lösung verwendet werden und führen dort zu einfachen Vorrichtungen mit guten Eigenschaften. Die Herstellung solcher Bauteile lehnt sich an die Herstellung polymerer Leuchtdioden (PLEDs) an, die in der Literatur bereits vielfach beschrieben ist (z. B. in der WO 2004/037887). Im vorliegenden Fall werden die erfindungsgemäßen Verbindungen T-1 bis T-5 in Toluol gelöst. Der typische Feststoffgehalt solcher Lösungen liegt zwischen 16 und 25 g/L, wenn, wie hier, die für eine Device typische Schichtdicke von 80 nm mittels Spincoating erzielt werden soll. Die verwendete PLED Vorrichtung zeigt den folgenden Aufbau: ITO/PEDOT:PSS/Interlayer/EML/Kathode, wobei EML die Emissionsschicht dargstellt. Die Emissionsschicht (EML) enthält neben den erfindungsgemäßen Verbindungen T-1 bis T-5, die in einer Konzentration von jeweils 4 Gew.-% vorliegen, die Matrixmaterialien TMM-1 und TMM-2 in einer Konzentration von jeweils 48 Gew.-%, d.h. TMM-1:TMM2 = 1:1. Strukturierte ITO-Substrate und das Material für die sogenannte Pufferschicht (PEDOT, eigentlich PEDOT:PSS) sind käuflich erhältlich (ITO von Technoprint und anderen, PEDOT:PSS als wässrige Dispersion Clevios Baytron P von H.C. Starck). Die verwendete Interlayer dient der Lochinjektion; in diesem Fall wurde HIL-012 von Merck verwendet. Die Emissionsschicht wird in einer Inertgasatmosphäre, im vorliegenden Fall Argon, aufgeschleudert und 10 min bei 120°C ausgeheizt. Zuletzt wird eine Kathode aus Barium und Aluminium im Vakuum aufgedampft. Zwischen der emittierenden Schicht und der Kathode können auch eine Lochblockierschicht und/oder eine Elektronentransportschicht per Bedampfung aufgebracht werden, auch kann die Interlayer durch eine oder mehrere Schichten ersetzt werden, die lediglich die Bedingung erfüllen müssen, durch den nachgelagerten Prozessierungsschritt der Abscheidung der emittierenden Schicht aus Lösung nicht wieder abgelöst zu werden.

Die Devices werden standardmäßig charakterisiert, die genannten OLED-Beispiele sind noch nicht optimiert. Tabelle 1 fasst die erhaltenen Daten zusammen. Bei den prozessierten Devices zeigt sich hier, dass die erfindungsgemäßen Materialien den zuvor zur Verfügung stehenden in Effizienz und/oder Lebensdauer überlegen sind.

**Tabelle 1: Ergebnisse mit aus Lösung prozessierten Materialien in der angegebenen Devicekonfiguration**

| Bsp. | EML 80 nm | Max. Eff. [cd/A] | Spannung [V] bei 100 cd/m² | CIE (x, y) | Lebensdauer [h], Anfangshelligkeit 1000 cd/m² |
|---|---|---|---|---|---|
| 6 | TMM-1 :TMM-2 : T-1 | 8 | 5.7 | 0.65/0.35 | 25000 |
| 7 | TMM-1 :TMM-2 : T-2 | 9 | 5.6 | 0.65/0.35 | 22000 |
| 8 | TMM-1 :TMM-2 : T-3 | 10 | 5.7 | 0.66/0.34 | 15000 |
| 9 | TMM-1 :TMM-2 : T-4 | 8 | 5.4 | 0.64/0.35 | 14000 |
| 10 | TMM-1 :TMM-2 : T-5 | 9 | 5.5 | 0.66/0.36 | 18000 |

## Patentansprüche

1. Verbindung gemäß Formel M(L)ₙ ausgewählt aus den folgenden Formeln 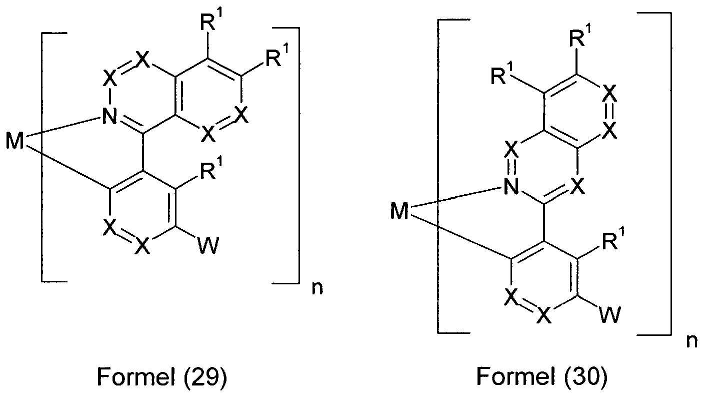 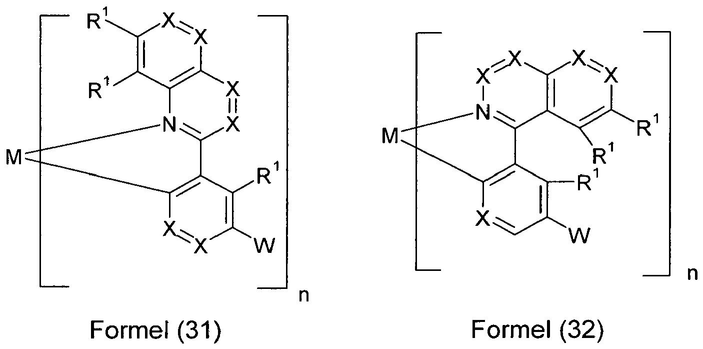 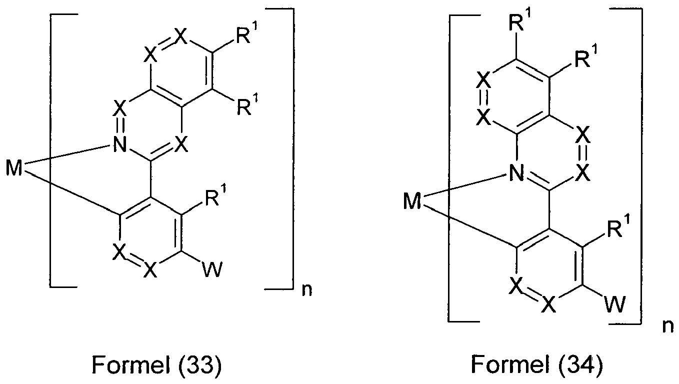 wobei für die verwendeten Symbole und Indices gilt:
M ist ein Metall ausgewählt aus der Gruppe bestehend aus Iridium, Rhodium, Platin und Palladium;
W ist gleich der Formel (3) 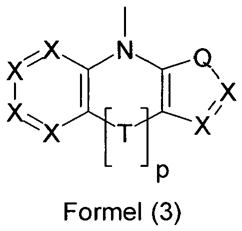 wobei T gleich oder verschieden bei jedem Auftreten ausgewählt aus der Gruppe bestehend aus -C(R¹)₂, -Si(R¹)₂, N, -NR¹, -O,- S, -C(=O), -S(=O), -SO₂, -CF₂, -SF₄, -P,- P(=O)R¹, -PF₂, -P(=S)R¹,- As, -As(=O), -As(=S), -Sb, -Ge, -Si, -Sb(=O), -C(R¹)₂-C(R¹)₂-, und -Sb(=S) ist;
Q ist gleich oder verschieden bei jedem Auftreten R¹C=CR¹, R¹C=N O, S, Se oder NR¹.
X ist gleich oder verschieden bei jedem Auftreten CR¹ oder N;
p ist 0 oder 1, wobei für p = 0 der mittlere Ring in Formel (3) einen fünfgliedrigen Ring ausbildet;
n ist 3 für M gleich Iridium oder Rhodium und ist 2 für M gleich Platin oder Palladium;
R¹ ist gleich oder verschieden bei jedem Auftreten H, D, F, Cl, Br, I, N(R²)₂, CN, NO₂, Si(R²)₃, B(OR²)₂, C(=O)R², P(=O)(R²)₂, S(=O)R², S(=O)₂R², OSO₂R², eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R² substituiert sein kann, wobei eine oder mehrere nicht benachbarte CH₂-Gruppen durch R²C=CR², C=C, Si(R²)₂, Ge(R²)₂, Sn(R²)₂, C=O, C=S, C=Se, C=NR², P(=O)(R²), SO, SO₂, NR², O, S oder CONR² ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R² substituiert sein kann, oder eine Aryloxy- oder Heteroaryloxygruppe mit 5 bis 60 aromatischen Ringatomen, die durch einen oder mehrere Reste R² substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R² substituiert sein kann, oder eine Kombination aus zwei oder mehr dieser Gruppen; dabei können zwei oder mehr Reste R¹ auch miteinander ein mono- oder polycyclisches, aliphatisches, aromatisches und/oder benzoannelliertes Ringsystem bilden;
R² ist gleich oder verschieden bei jedem Auftreten H, D, F, Cl, Br, I, N(R³)₂, CN, NO₂; Si(R³)₃, B(OR³)₂, C(=O)R³, P(=O)(R³)₂, S(=O)R³, S(=O)₂R³, OSO₂R³, eine geradkettige Alkyl-, Alkoxy- oder Thioalkoxygruppe mit 1 bis 40 C-Atomen oder eine geradkettige Alkenyl- oder Alkinylgruppe mit 2 bis 40 C-Atomen oder eine verzweigte oder cyclische Alkyl-, Alkenyl-, Alkinyl-, Alkoxy- oder Thioalkoxygruppe mit 3 bis 40 C-Atomen, die jeweils mit einem oder mehreren Resten R³ substituiert sein kann, wobei eine oder mehrere nicht benachbarte CH₂-Gruppen durch R³C=CR³, C≡C, Si(R³)₂, Ge(R³)₂, Sn(R³)₂, C=O, C=S, C=Se, C=NR³, P(=O)(R³), SO, SO₂, NR³, O, S oder CONR³ ersetzt sein können und wobei ein oder mehrere H-Atome durch D, F, Cl, Br, I, CN oder NO₂ ersetzt sein können, oder ein aromatisches oder heteroaromatisches Ringsystem mit 5 bis 60 aromatischen Ringatomen, das jeweils durch einen oder mehrere Reste R³ substituiert sein kann, oder eine Aryloxy- oder Heteroaryloxygruppe mit 5 bis 60 aromatischen Ringatomen, die durch einen oder mehrere Reste R³ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R³ substituiert sein kann, oder eine Kombination aus zwei oder mehr dieser Gruppen; dabei können zwei oder mehrere benachbarte Reste R² miteinander ein mono- oder polycyclisches, aliphatisches oder aromatisches Ringsystem bilden;
R³ ist gleich oder verschieden bei jedem Auftreten H, D, F oder ein aliphatischer, aromatischer und/oder heteroaromatischer Kohlenwasserstoffrest mit 1 bis 20 C-Atomen, in dem auch ein oder mehrere H-Atome durch F ersetzt sein können; dabei können zwei oder mehrere Substituenten R³ auch miteinander ein mono- oder polycyclisches, aliphatisches oder aromatisches Ringsystem bilden;
dabei werden die Indizes n und m so gewählt, dass die Koordinationszahl am Metall für M gleich Iridium oder Rhodium 6 entspricht und für M gleich Platin oder Palladium 4 entspricht;
dabei können auch mehrere Liganden L miteinander über eine beliebige Brücke Z verknüpft sein und so ein tridentates, tetradentates, pentadentates oder hexadentates Ligandensystem aufspannen.

2. Verbindung nach Anspruch 1, **dadurch gekennzeichnet, dass** der Rest W gegeben ist durch die Formeln (47) bis (55) 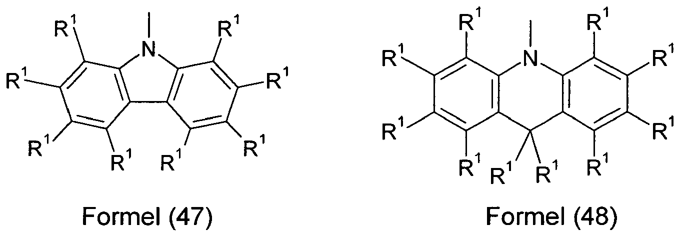 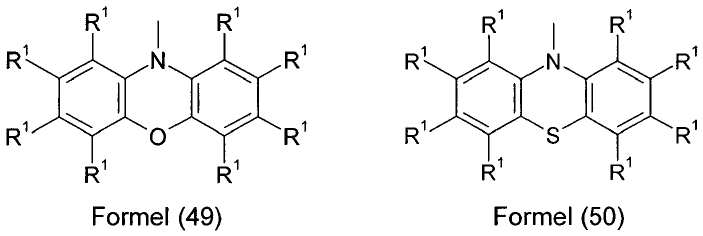 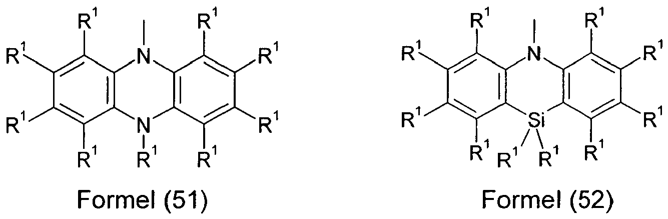 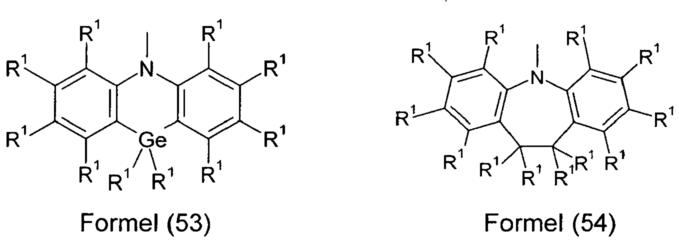 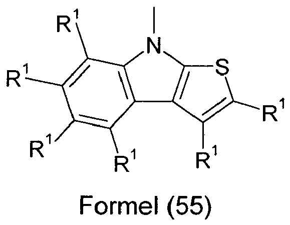 wobei die Linie die Position angibt an der die Bindung des Restes W zu dem Liganden erfolgt und wobei die verwendeten Symbole den in Anspruch 1 definierten Symbolen entsprechen.

3. Verbindung nach Anspruch 1 oder 2, **dadurch gekennzeichnet, dass** Struktur M(L)ₙ ausgewählt wird aus den Formeln (71) bis (85)

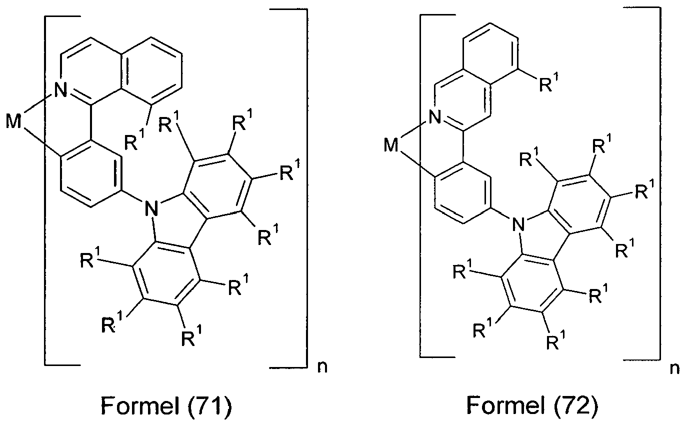

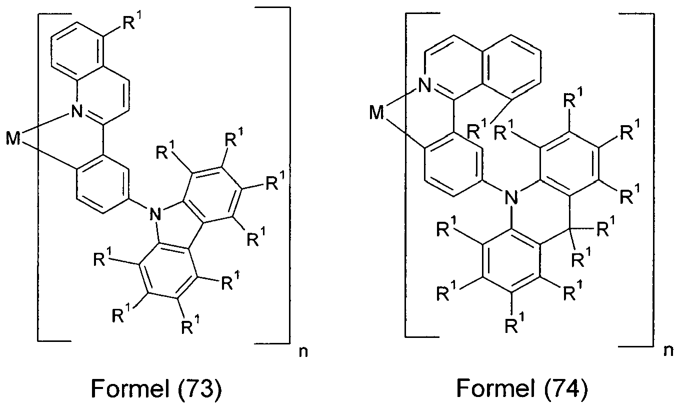

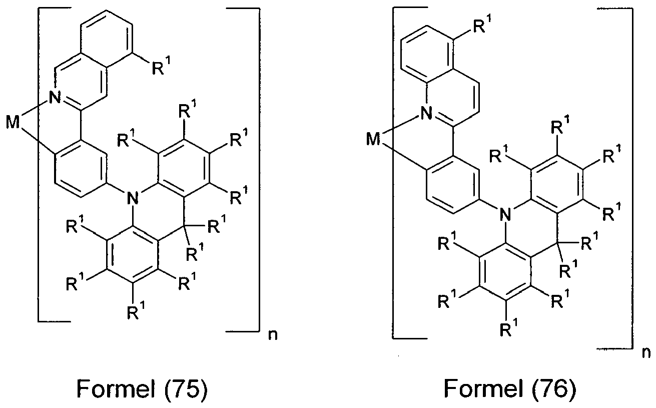

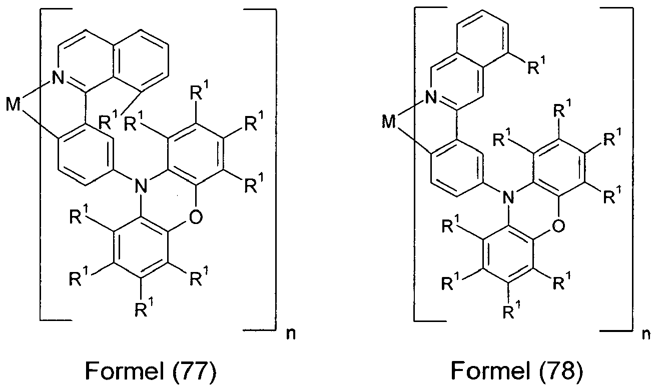

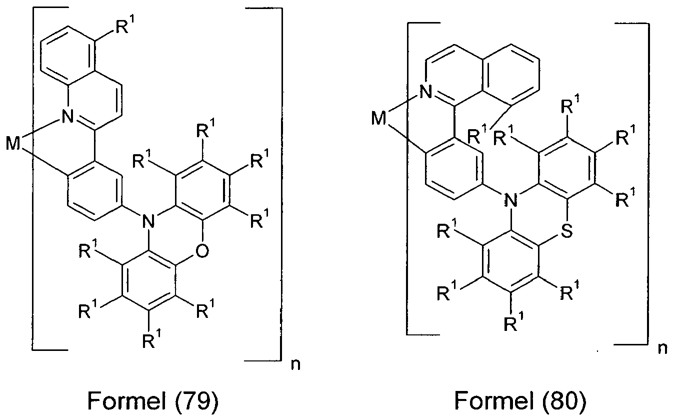

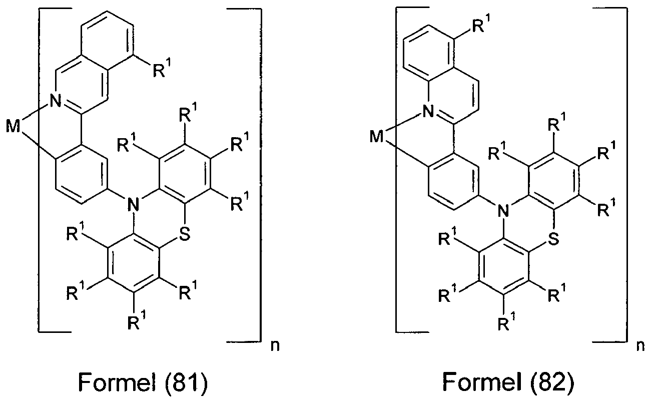

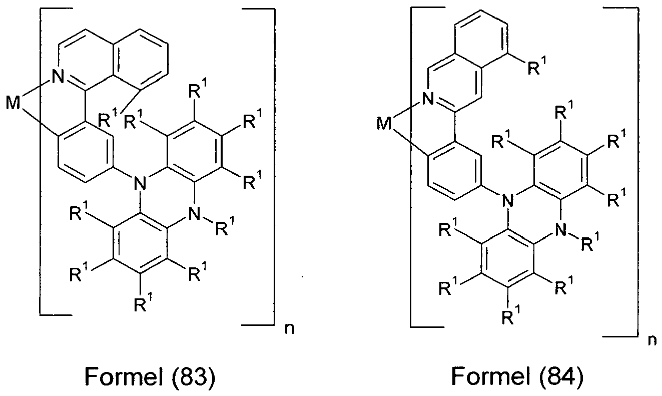

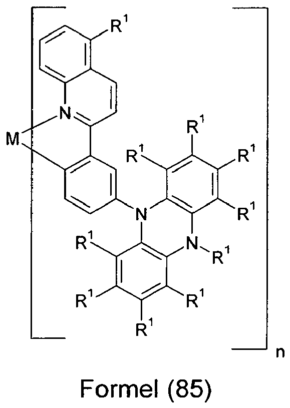

4. Verbindung nach einem oder mehreren der Ansprüche 1 bis 3, **dadurch gekennzeichnet, dass** R¹ gleich oder verschieden bei jedem Auftreten -H, -NH₂ oder ein Rest der Formeln (86) bis (224) wobei die Reste auch einfach oder mehrfach mit einem Rest R³ substituiert sein können, die bei mehrfachem Auftreten gleich oder unabhängig voneinander sein können

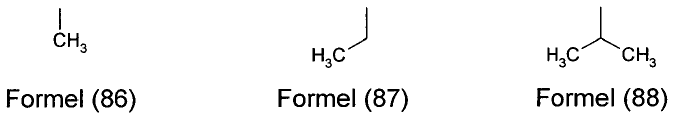

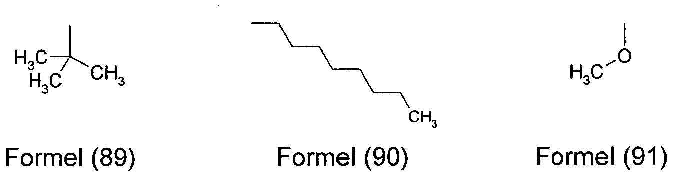

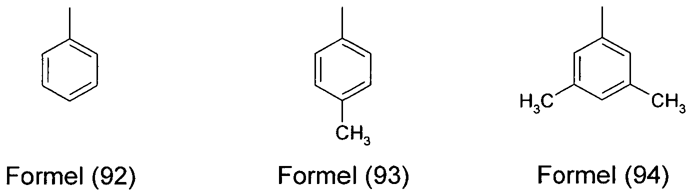

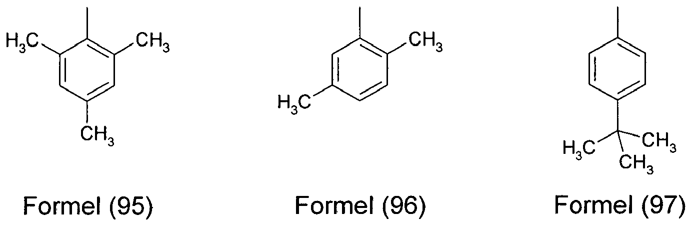

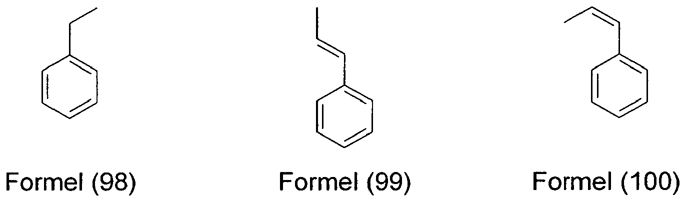

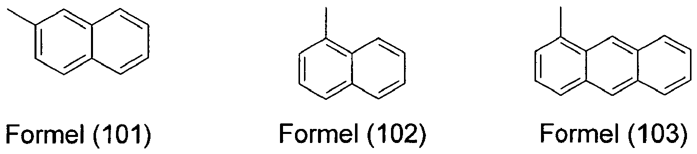

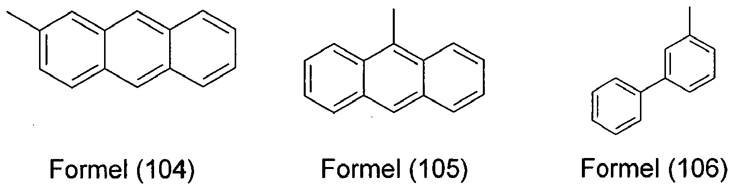

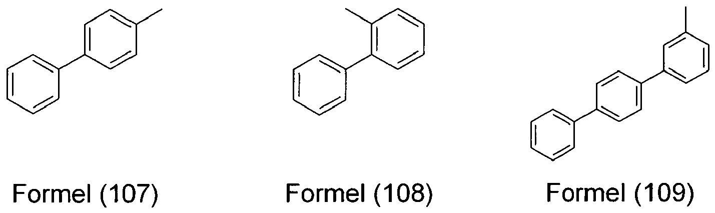

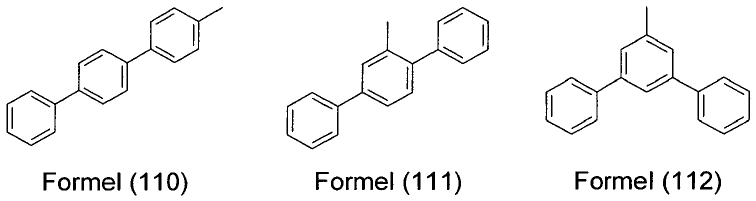

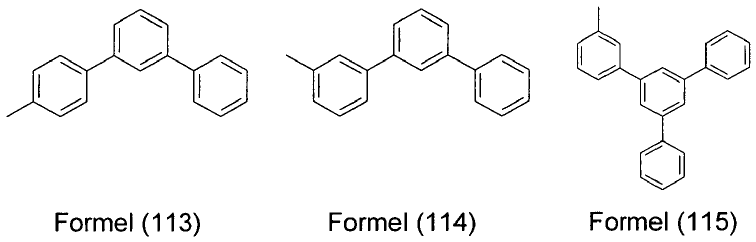

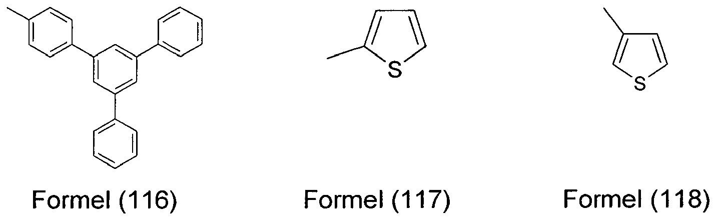

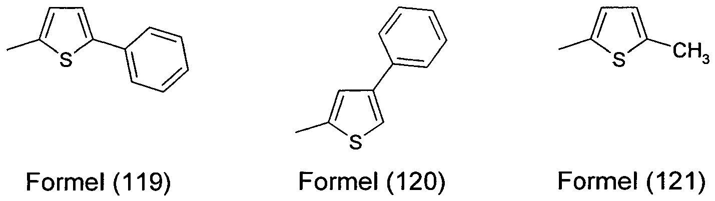

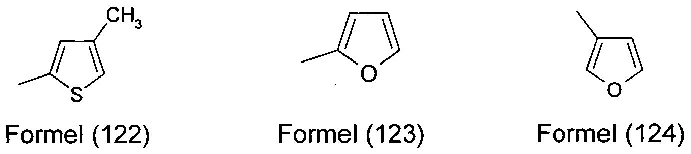

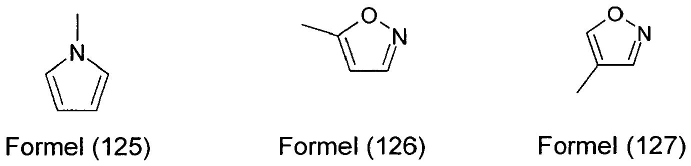

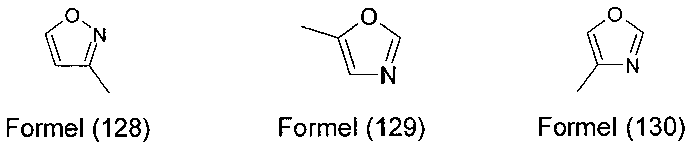

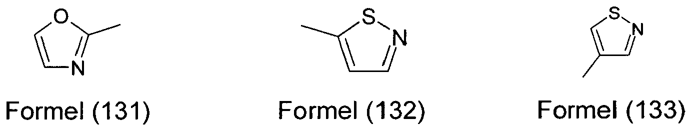

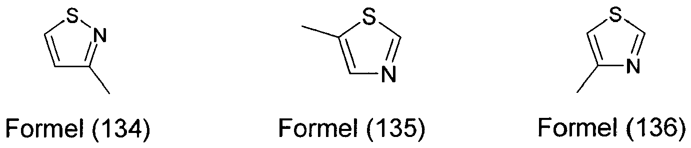

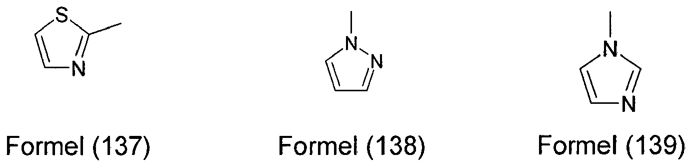

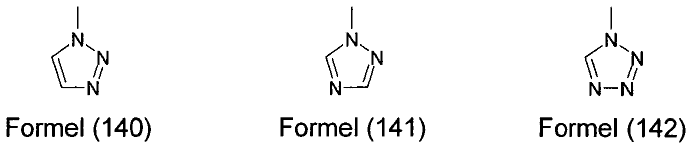

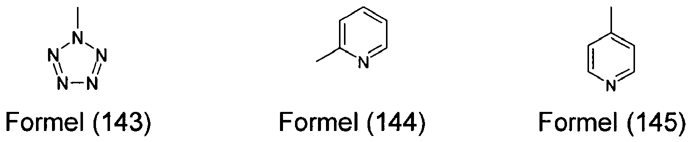

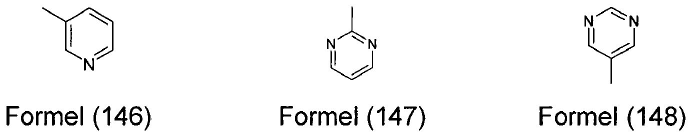

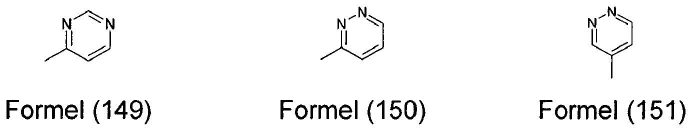

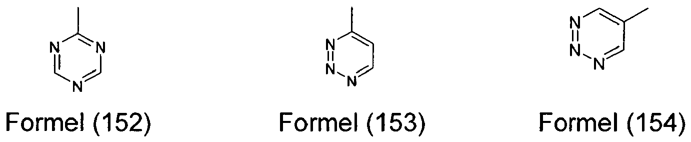

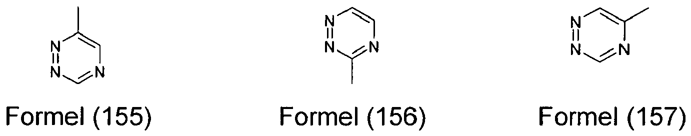

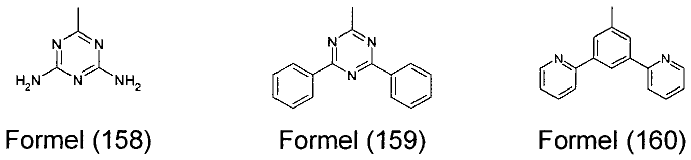

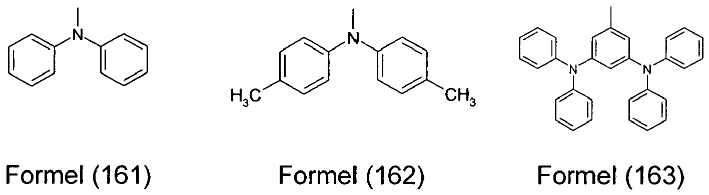

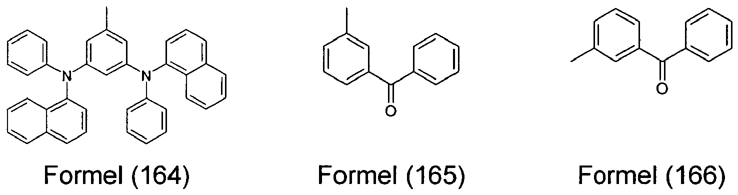

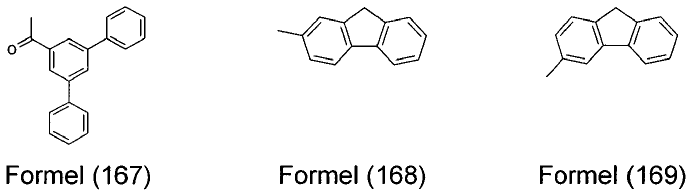

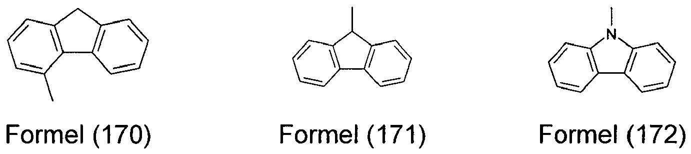

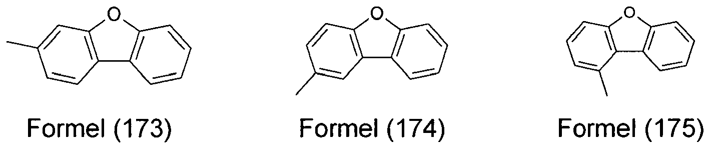

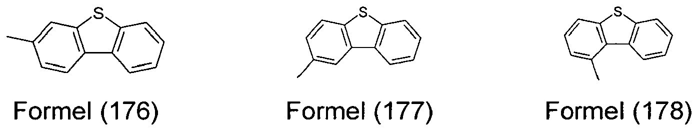

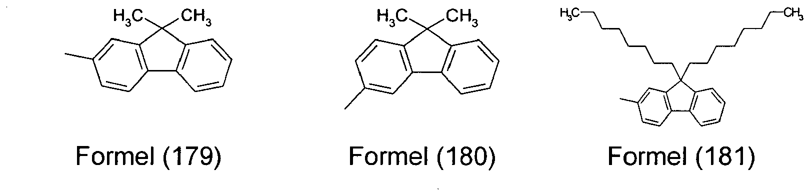

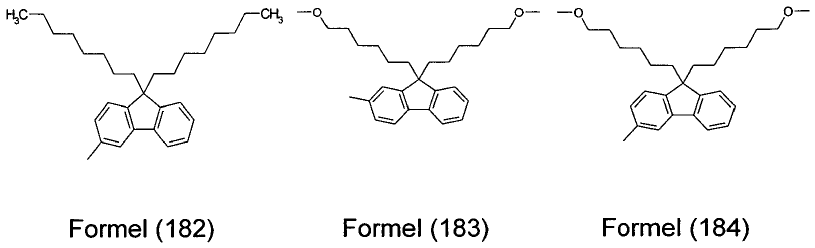

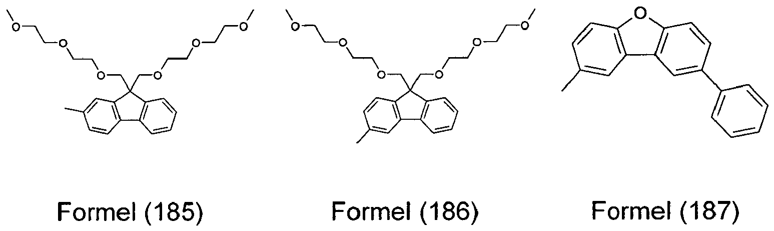

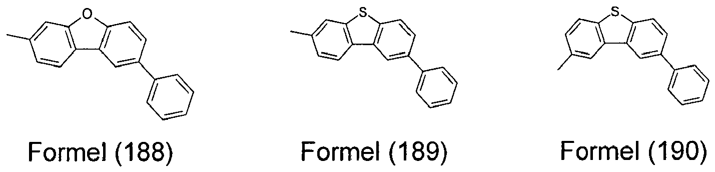

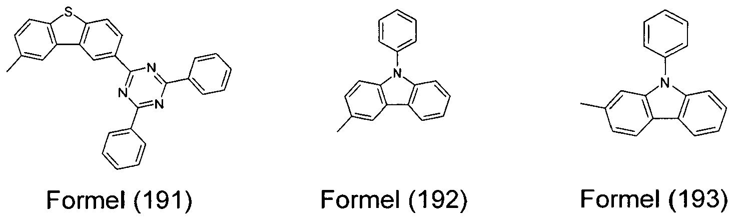

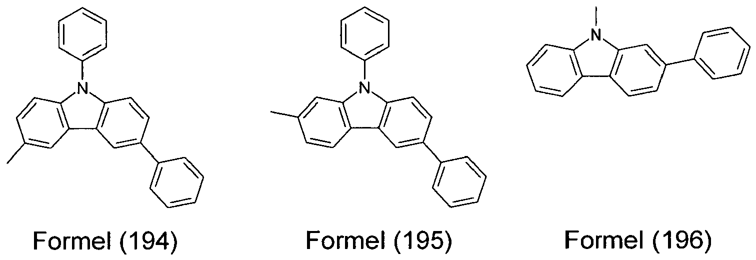

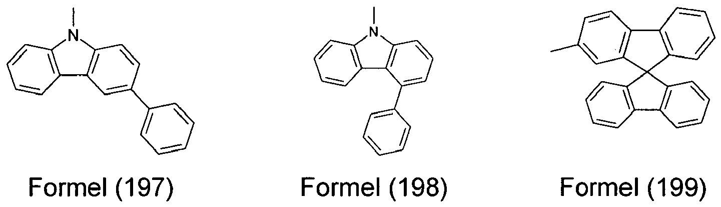

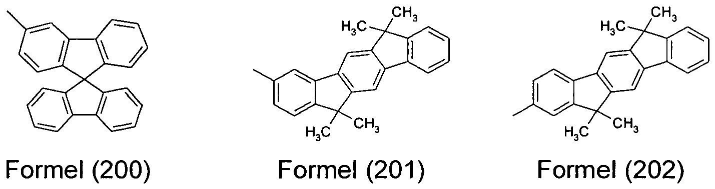

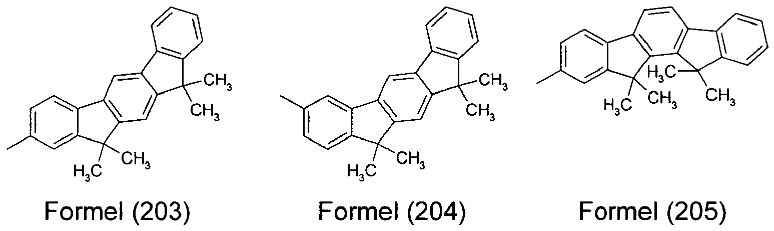

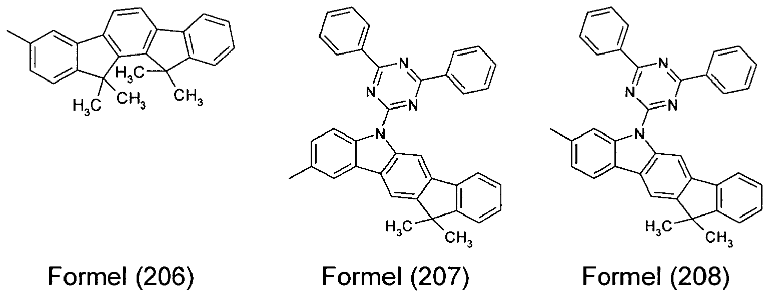

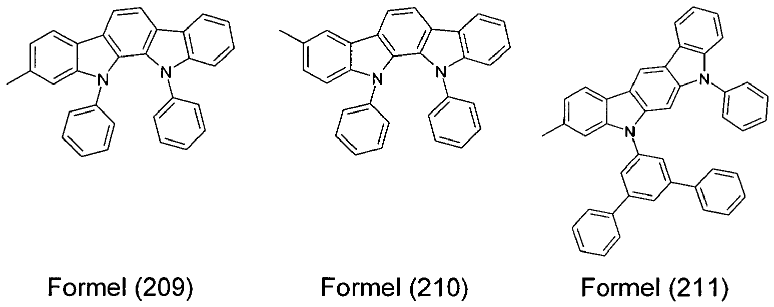

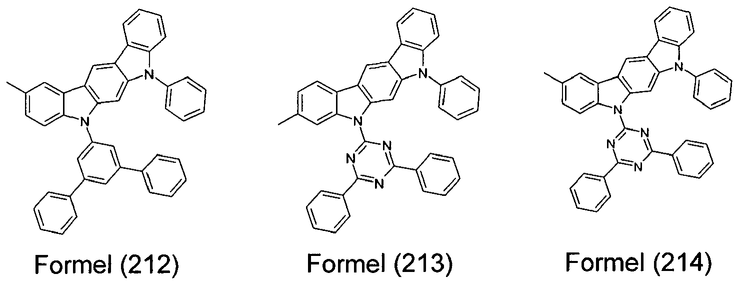

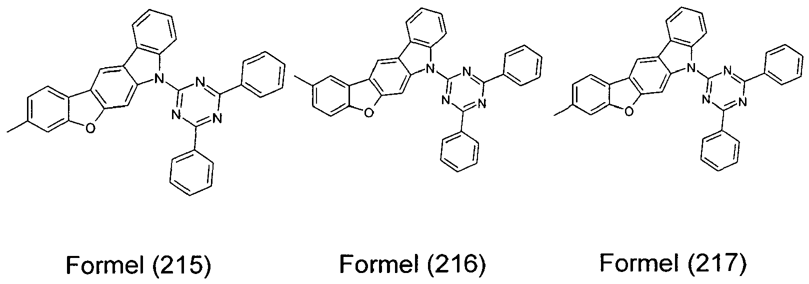

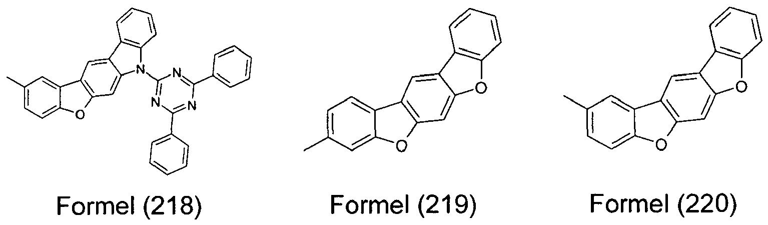

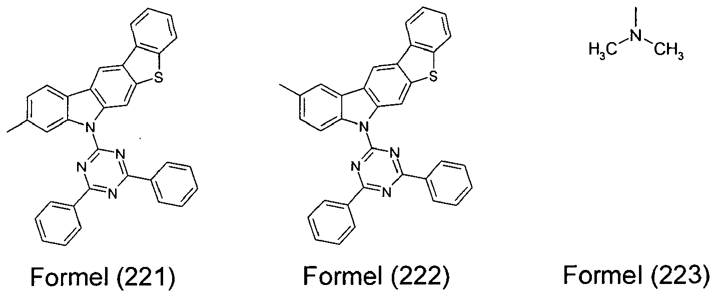

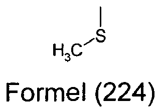

5. Verbindung nach einem oder mehreren der Ansprüche 1 bis 4, **dadurch gekennzeichnet, dass** M gleich Iridium ist.

6. Verfahren zur Herstellung einer Verbindung nach einem oder mehreren der Ansprüche 1 bis 5 durch Umsetzung der entsprechenden freien Liganden mit Metallalkoholaten der Formel (282), mit Metallketoketonaten der Formel (283) oder mit Metallhalogeniden der Formel (284)
M(OR¹)ₙ Formel (282) 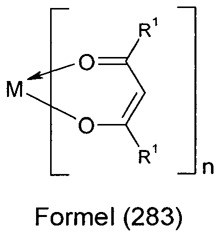
MHalₙ Formel (284)
wobei die Symbole M, n und R¹ die oben angegebenen Bedeutungen haben und Hal = F, Cl, Br oder I ist.

7. Formulierung enthaltend wenigstens eine Verbindung nach einem oder mehreren der Ansprüche 1 bis 5 und wenigstens ein Lösungsmittel.

8. Verwendung einer Verbindung nach einem oder mehreren der Ansprüche 1 bis 5 in einer elektronischen Vorrichtung, bevorzugt ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs) oder organischen Laserdioden (O-Laser).

9. Elektronische Vorrichtung, enthaltend mindestens eine Verbindung nach einem oder mehreren der Ansprüche 1 bis 5 bevorzugt ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), organischen optischen Detektoren, organischen Photorezeptoren, organischen Feld-Quench-Devices (O-FQDs), lichtemittierenden elektrochemischen Zellen (LECs) oder organischen Laserdioden (O-Laser).

10. Organische Elektrolumineszenzvorrichtung nach Anspruch 9, **dadurch gekennzeichnet, dass** die Verbindung nach einem oder mehreren der Ansprüche 1 bis 5 als emittierende Verbindung in einer oder mehreren emittierenden Schichten eingesetzt wird, bevorzugt in Kombination mit einem Matrixmaterial, wobei das Matrixmaterial bevorzugt ausgewählt ist aus der Gruppe bestehend aus Ketonen, Phosphinoxiden, Sulfoxiden, Sulfonen, Triarylaminen, Carbazolderivaten, Indolocarbazolderivaten, Indenocarbazolderivaten, Azacarbazolderivaten, bipolaren Matrixmaterialien, Silanen, Azaborolen, Boronestern, Triazinderivaten, Zinkkomplexen, Diaza- oder Tetraazasilolderivaten oder Diazaphospholderivaten oder Mischungen dieser Matrixmaterialien.

11. Vorrichtung nach Anspruch 9 oder 10 zur Verwendung in der Medizin zur Phototherapie.

12. Verwendung der Vorrichtung nach Anspruch 9 oder 10 in der Kosmetik zur Phototherapie.

## Claims

1. Compound of the formula M(L)ₙ selected from the following formulae: 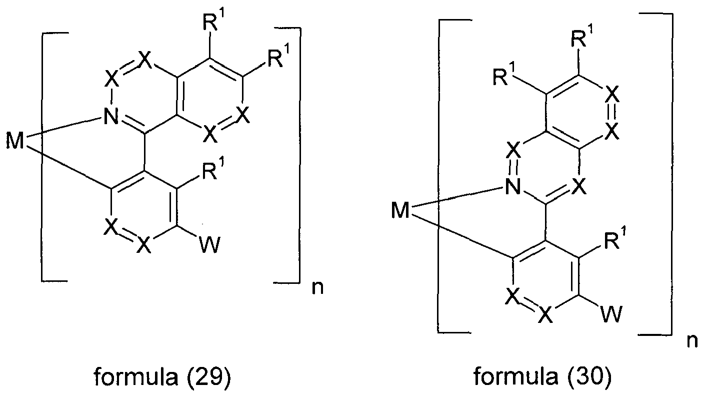 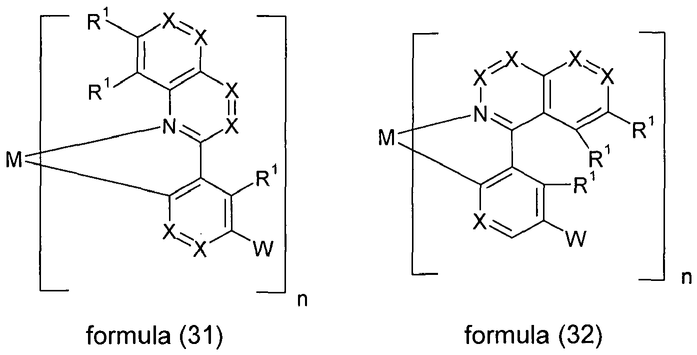 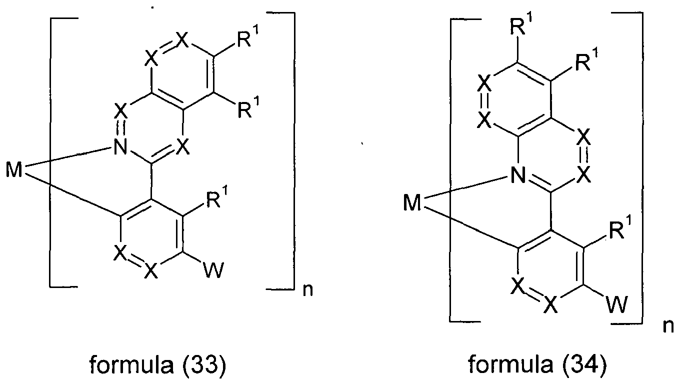 where the following applies to the symbols and indices used:
M is a metal selected from the group consisting of iridium, rhodium, platinum and palladium;
W is equal to the formula (3) 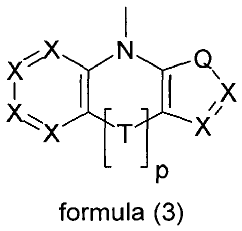 where T is selected, identically or differently on each occurrence, from the group consisting of -C(R¹)₂, -Si(R¹)₂, N, -NR¹, -O, -S, -C(=O), -S(=O), -SO₂, -CF₂, -SF₄, -P, -P(=O)R¹, -PF₂, -P(=S)R¹, -As, -As(=O), -As(=S), -Sb, -Ge, -Si, -Sb(=O), -C(R¹)₂-C(R¹)₂- and -Sb(=S);
Q is, identically or differently on each occurrence, R¹C=CR¹, R¹C=N, O, S, Se or NR¹;
X is, identically or differently on each occurrence, CR¹ or N;
p is 0 or 1, where, for p = 0, the central ring in formula (3) forms a five-membered ring;
n is 3 for M equal to iridium or rhodium and is 2 for M equal to platinum or palladium;
R¹ is, identically or differently on each occurrence, H, D, F, Cl, Br, I, N(R²)₂, CN, NO₂, Si(R²)₃, B(OR²)₂, C(=O)R², P(=O)(R²)₂, S(=O)R², S(=O)₂R², OSO₂R², a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy or thioalkoxy group having 3 to 40 C atoms, each of which may be substituted by one or more radicals R², where one or more non-adjacent CH₂ groups may be replaced by R²C=CR², C=C, Si(R²)₂, Ge(R²)₂, Sn(R²)₂, C=O, C=S, C=Se, C=NR², P(=O)(R²), SO, SO₂, NR²,O, S or CONR² and where one or more H atoms may be replaced by D, F, Cl, Br, I, CN or NO₂, or an aromatic or heteroaromatic ring system having 5 to 60 aromatic ring atoms, which may in each case be substituted by one or more radicals R², or an aryloxy or heteroaryloxy group having 5 to 60 aromatic ring atoms, which may be substituted by one or more radicals R², or a diarylamino group, diheteroarylamino group or arylheteroarylamino group having 10 to 40 aromatic ring atoms, which may be substituted by one or more radicals R², or a combination of two or more of these groups; two or more radicals R¹ here may also form a mono- or polycyclic, aliphatic, aromatic and/or benzo-fused ring system with one another;
R² is, identically or differently on each occurrence, H, D, F, Cl, Br, I, N(R³)₂, CN, NO₂, Si(R³)₃, B(OR³)₂, C(=O)R³, P(=O)(R³)₂, S(=O)R³, S(=O)₂R³, OSO₂R³, a straight-chain alkyl, alkoxy or thioalkoxy group having 1 to 40 C atoms or a straight-chain alkenyl or alkynyl group having 2 to 40 C atoms or a branched or cyclic alkyl, alkenyl, alkynyl, alkoxy or thioalkoxy group having 3 to 40 C atoms, each of which may be substituted by one or more radicals R³, where one or more non-adjacent CH₂ groups may be replaced by R³C=CR³, C=C, Si(R³)₂, Ge(R³)₂, Sn(R³)₂, C=O, C=S, C=Se, C=NR³, P(=O)(R³), SO, SO₂, NR³,O, S or CONR³ and where one or more H atoms may be replaced by D, F, Cl, Br, I, CN or NO₂, or an aromatic or heteroaromatic ring system having 5 to 60 aromatic ring atoms, which may in each case be substituted by one or more radicals R³, or an aryloxy or heteroaryloxy group having 5 to 60 aromatic ring atoms, which may be substituted by one or more radicals R³, or a diarylamino group, diheteroarylamino group or arylheteroarylamino group having 10 to 40 aromatic ring atoms, which may be substituted by one or more radicals R³, or a combination of two or more of these groups; two or more adjacent radicals R² here may form a mono- or polycyclic, aliphatic or aromatic ring system with one another;
R³ is, identically or differently on each occurrence, H, D, F or an aliphatic, aromatic and/or heteroaromatic hydrocarbon radical having 1 to 20 C atoms, in which, in addition, one or more H atoms may be replaced by F; two or more substituents R³ here may also form a mono- or polycyclic, aliphatic or aromatic ring system with one another;
the indices n and m here are selected so that the coordination number on the metal corresponds to 6 for M equal to iridium or rhodium and corresponds to 4 for M equal to platinum or palladium;
a plurality of ligands L here may also be linked to one another via any desired bridge Z and thus form a tridentate, tetradentate, penta-dentate or hexadentate ligand system.

2. Compound according to Claim 1, **characterised in that** the radical W is given by the formulae (47) to (55) 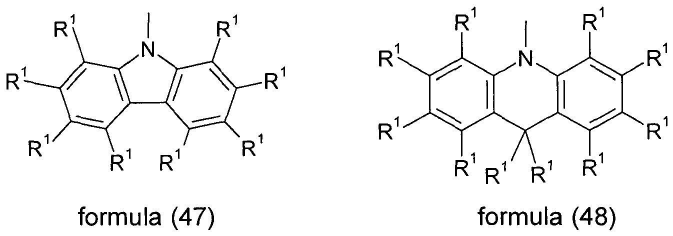 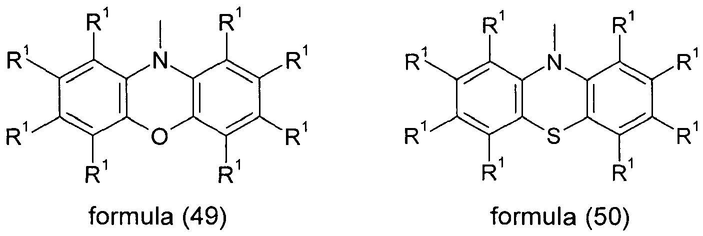 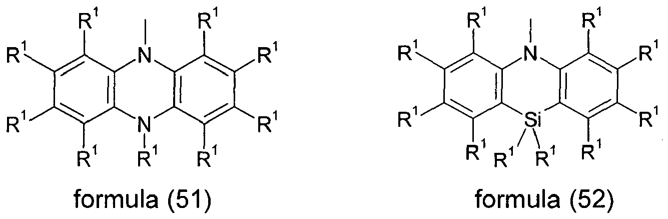 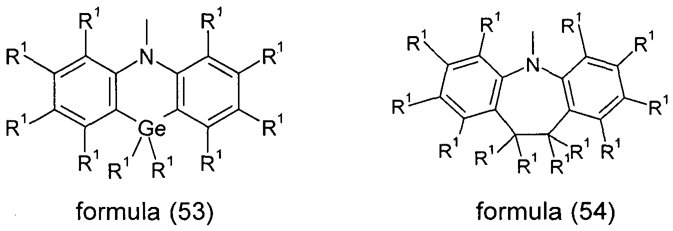 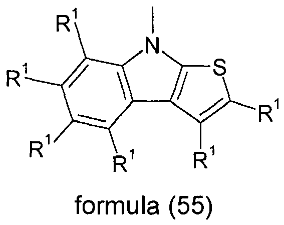 where the line indicates the position at which the bonding of the radical W to the ligand takes place and where the symbols used correspond to the symbols defined in Claim 1.

3. Compound according to Claim 1 or 2, **characterised in that** structure M(L)ₙ is selected from the formulae (71) to (85)

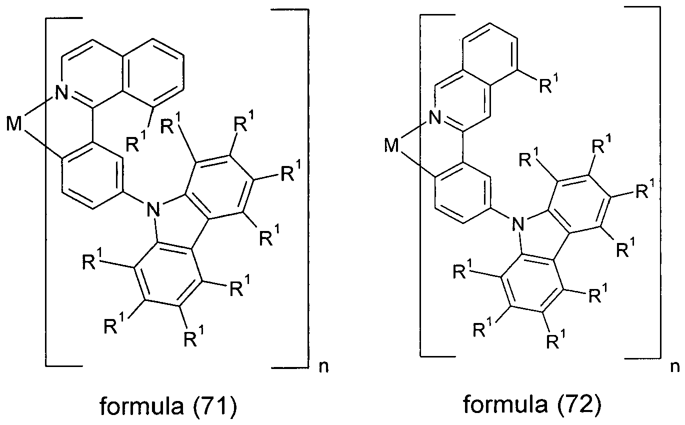

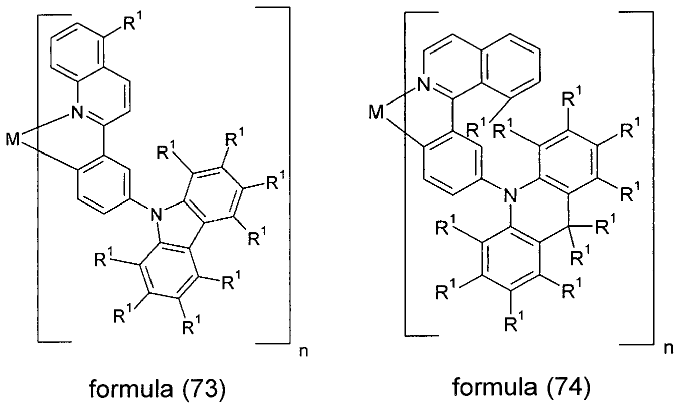

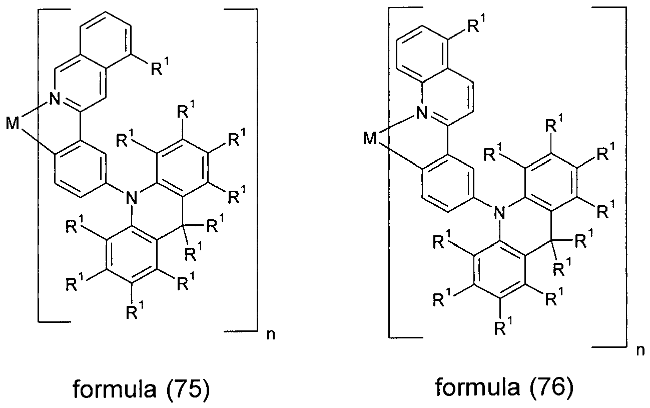

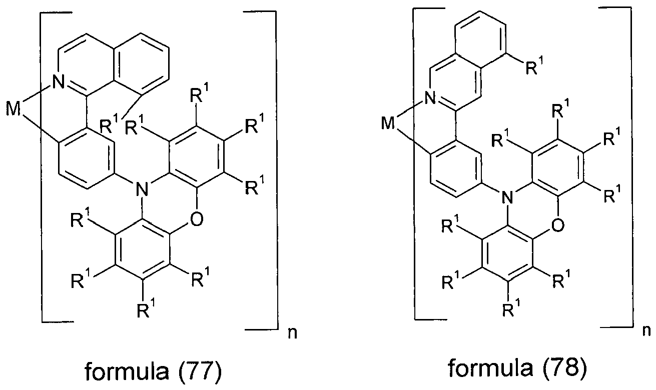

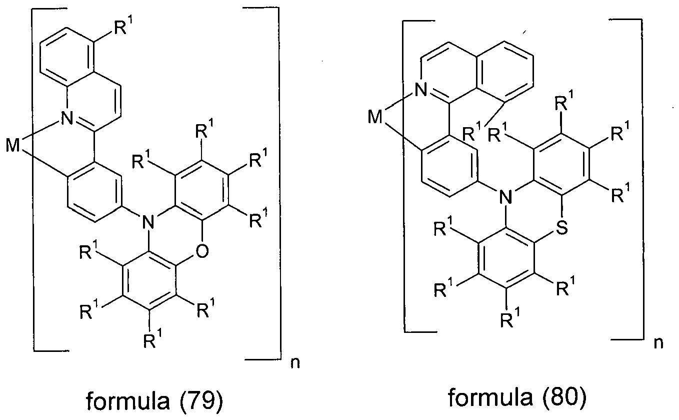

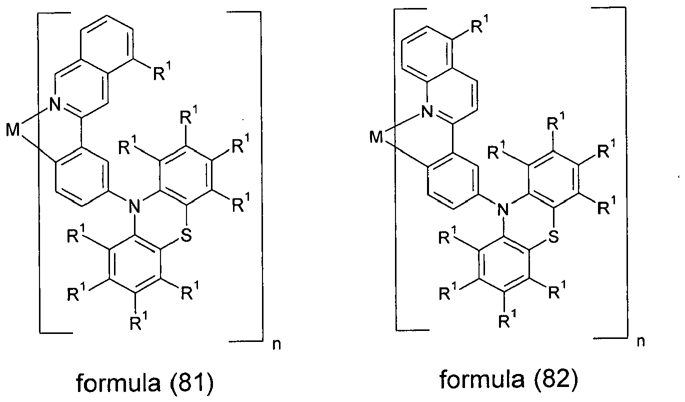

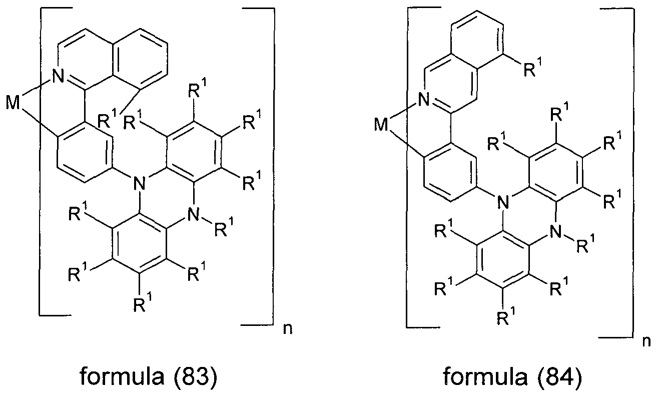

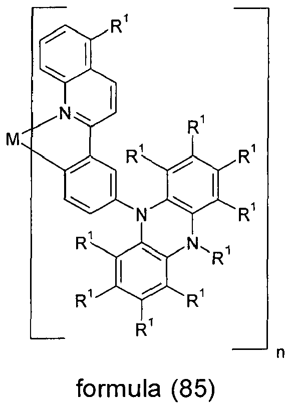

4. Compound according to one or more of Claims 1 to 3, **characterised in that** R¹ is, identically or differently on each occurrence, -H, -NH₂ or a radical of the formulae (86) to (224) where the radicals may also be mono- or polysubstituted by a radical R³, which, if they occur more than once, may be identical or independent of one another,

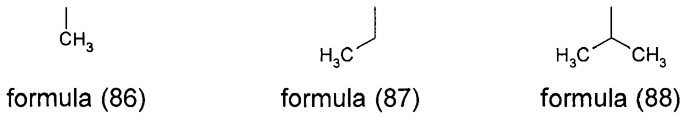

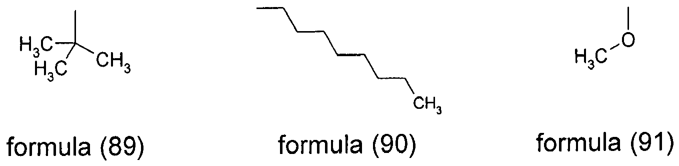

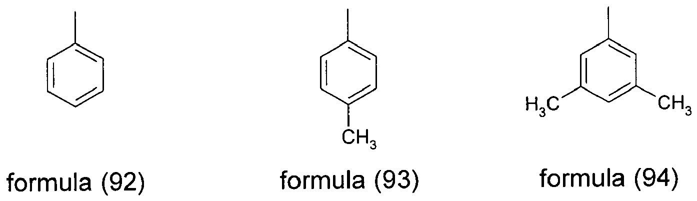

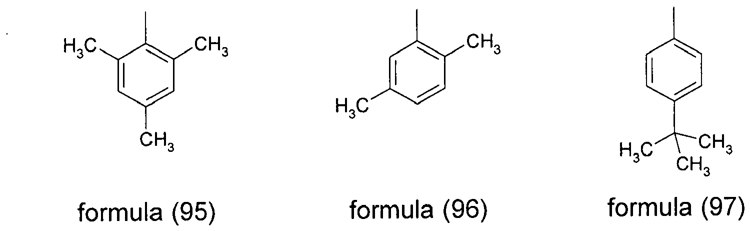

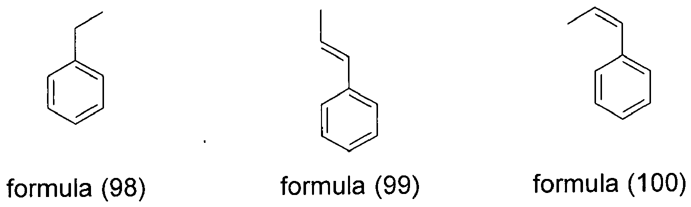

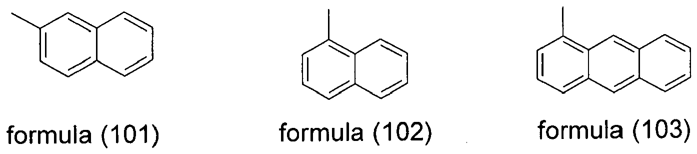

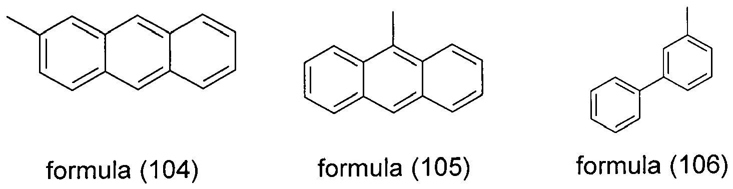

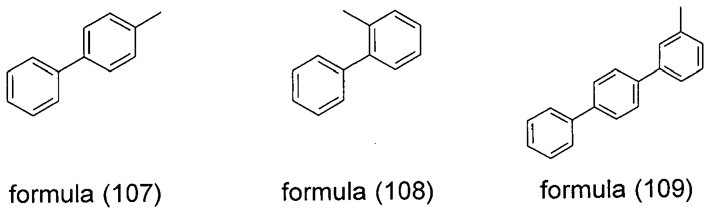

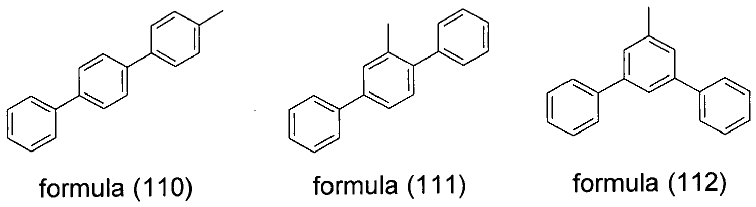

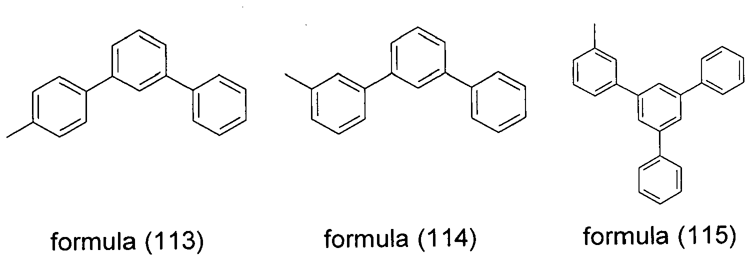

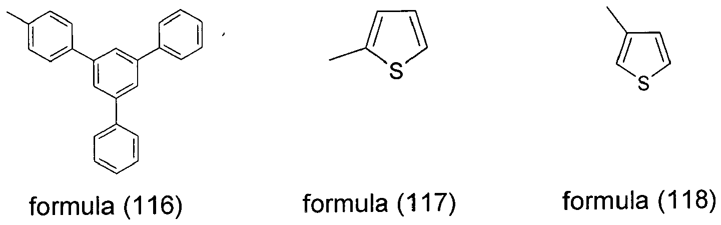

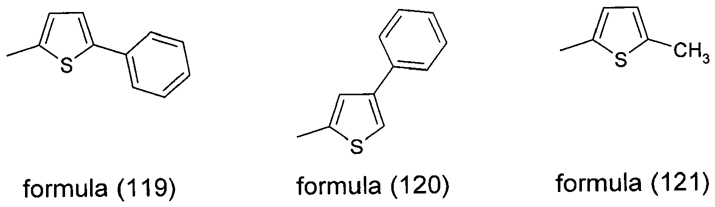

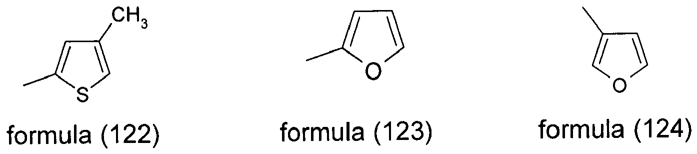

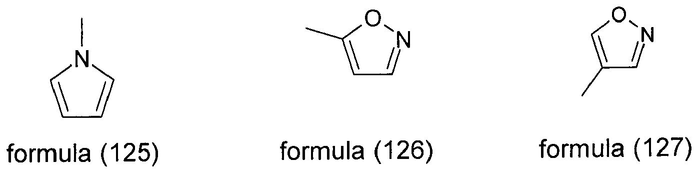

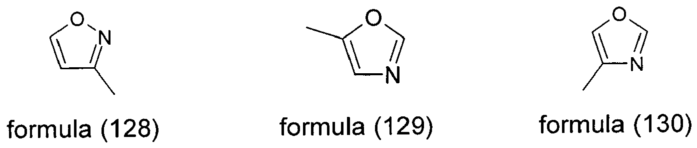

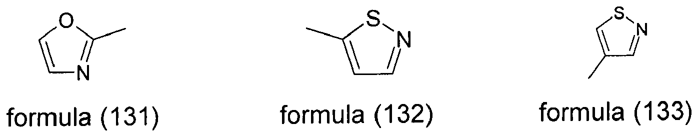

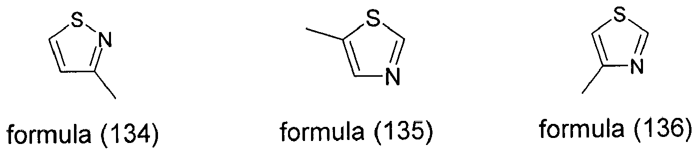

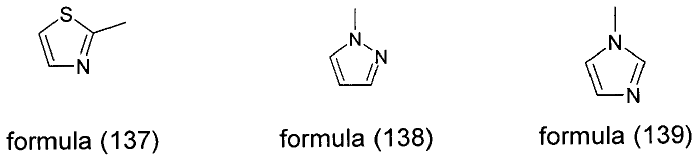

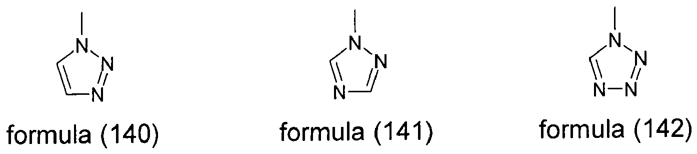

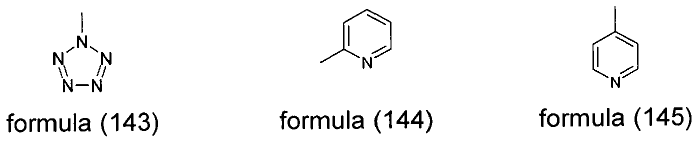

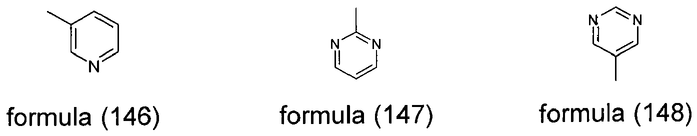

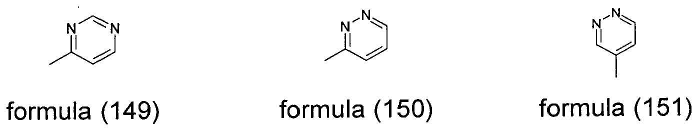

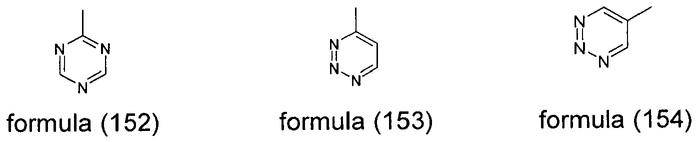

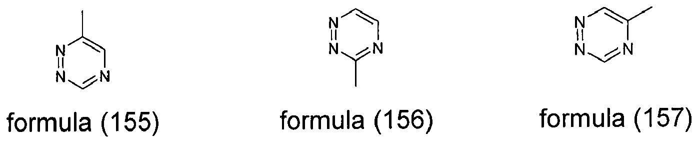

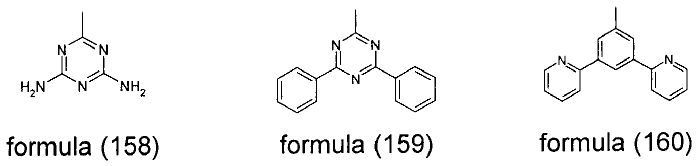

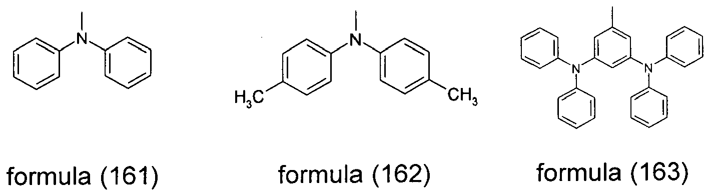

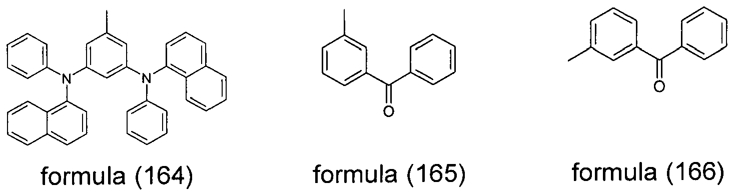

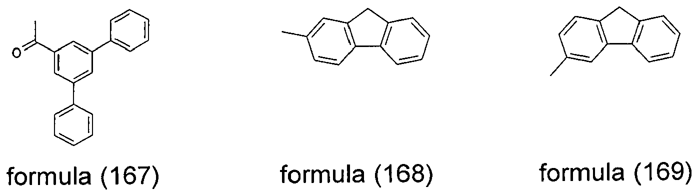

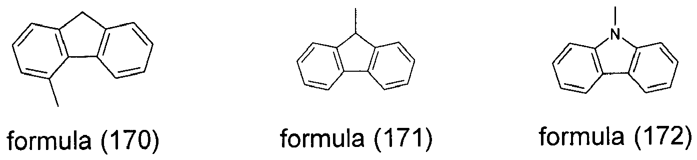

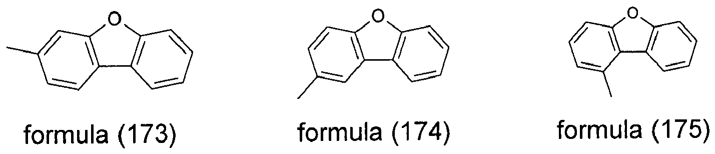

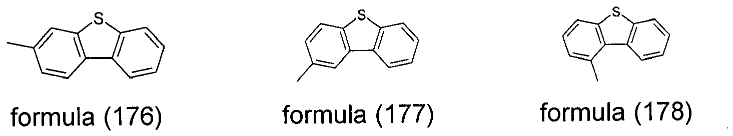

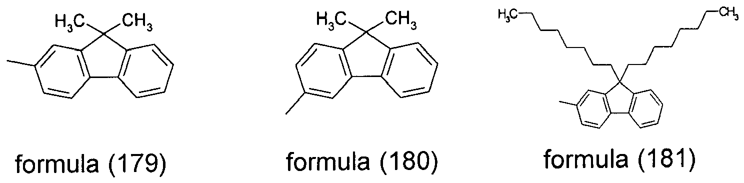

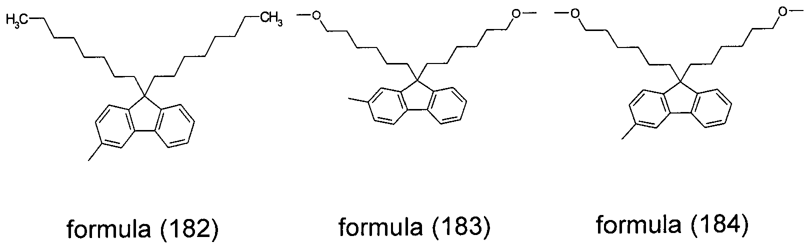

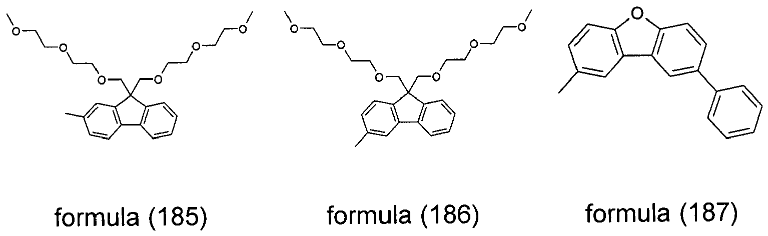

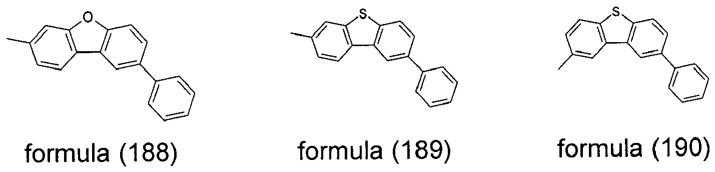

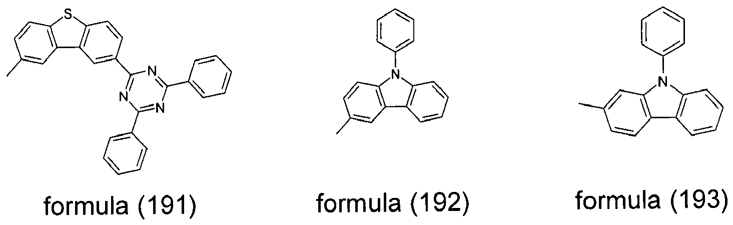

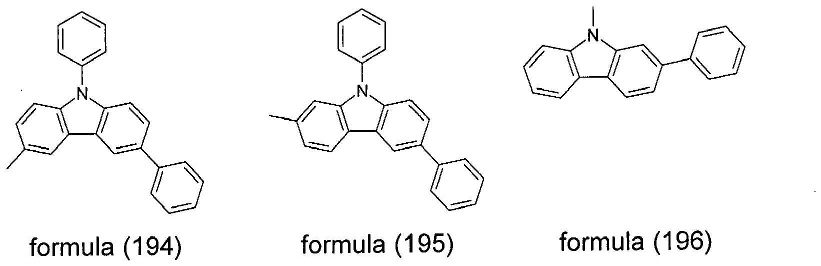

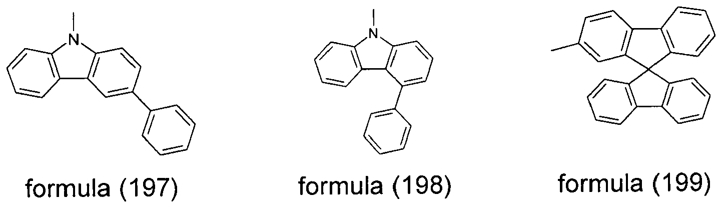

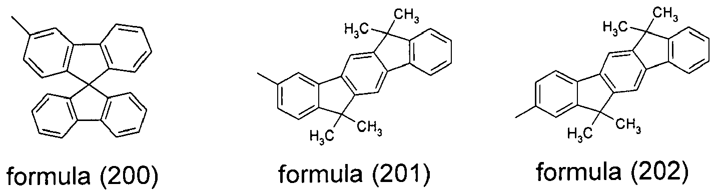

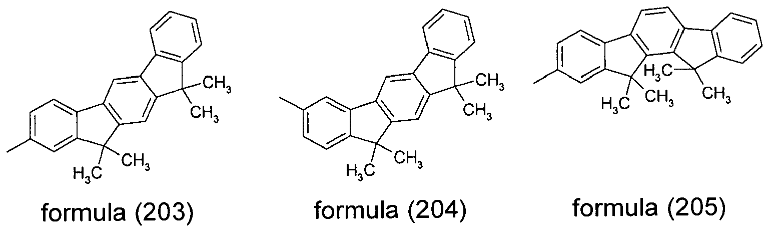

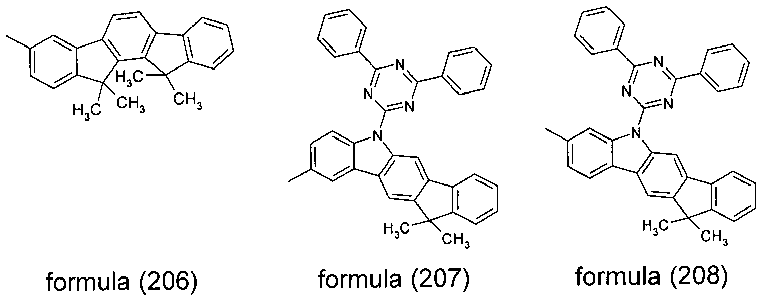

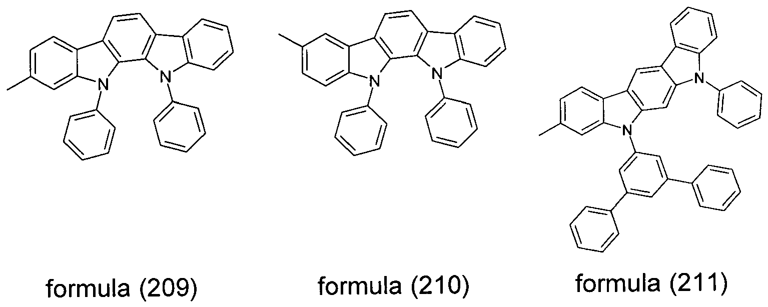

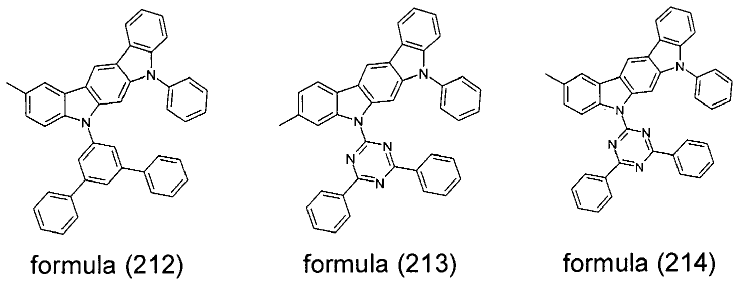

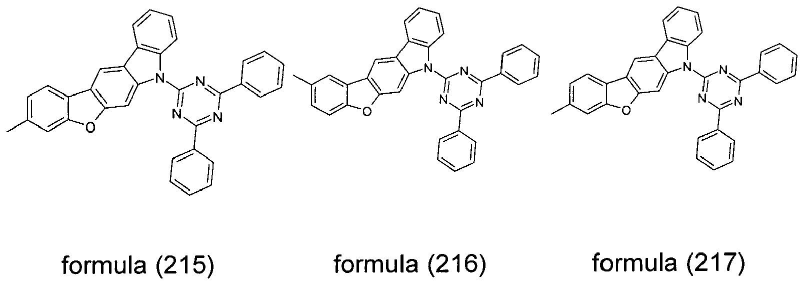

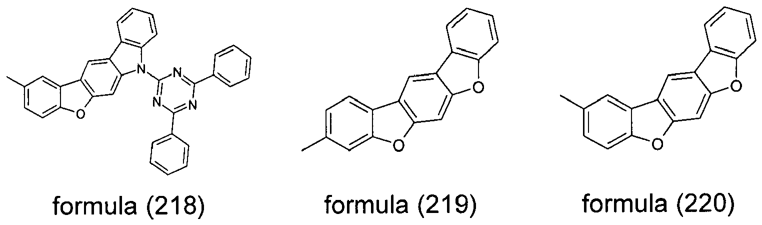

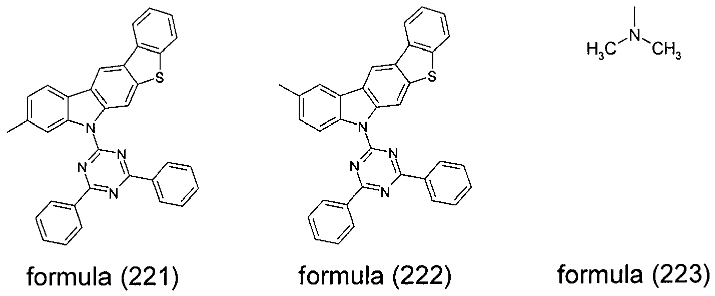

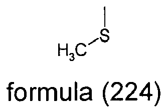

5. Compound according to one or more of Claims 1 to 4, **characterised in that** M is equal to iridium.

6. Process for the preparation of a compound according to one or more of Claims 1 to 5 by reaction of the corresponding free ligands with metal alcoholates of the formula (282), with metal ketoketonates of the formula (283) or with metal halides of the formula (284)
M(OR¹)ₙ formula (282) 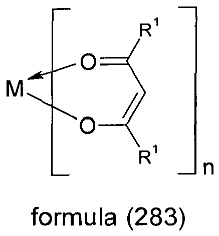
MHalₙ formula (284)
where the symbols M, n and R¹ have the meanings indicated above and Hal = F, Cl, Br or I.

7. Formulation comprising at least one compound according to one or more of Claims 1 to 5 and at least one solvent.

8. Use of a compound according to one or more of Claims 1 to 5 in an electronic device, preferably selected from the group consisting of organic electroluminescent devices (OLEDs, PLEDs), organic integrated circuits (O-ICs), organic field-effect transistors (O-FETs), organic thin-film transistors (O-TFTs), organic light-emitting transistors (O-LETs), organic solar cells (O-SCs), organic optical detectors, organic photoreceptors, organic field-quench devices (O-FQDs), light-emitting electrochemical cells (LECs) or organic laser diodes (O-lasers).

9. Electronic device comprising at least one compound according to one or more of Claims 1 to 5, preferably selected from the group consisting of organic electroluminescent devices (OLEDs, PLEDs), organic integrated circuits (O-ICs), organic field-effect transistors (O-FETs), organic thin-film transistors (O-TFTs), organic light-emitting transistors (O-LETs), organic solar cells (O-SCs), organic optical detectors, organic photoreceptors, organic field-quench devices (O-FQDs), light-emitting electrochemical cells (LECs) or organic laser diodes (O-lasers).

10. Organic electroluminescent device according to Claim 9, **characterised in that** the compound according to one or more of Claims 1 to 5 is employed as emitting compound in one or more emitting layers, preferably in combination with a matrix material, where the matrix material is preferably selected from the group consisting of ketones, phosphine oxides, sulfoxides, sulfones, triarylamines, carbazole derivatives, indolocarbazole derivatives, indenocarbazole derivatives, azacarbazole derivatives, bipolar matrix materials, silanes, aza-boroles, boronic esters, triazine derivatives, zinc complexes, diaza- or tetraazasilole derivatives or diazaphosphole derivatives or mixtures of these matrix materials.

11. Device according to Claim 9 or 10 for use in medicine for phototherapy.

12. Use of the device according to Claim 9 or 10 in the cosmetics field for phototherapy.

## Revendications

1. Composé de la formule M(L)ₙ choisie parmi les formules qui suivent : 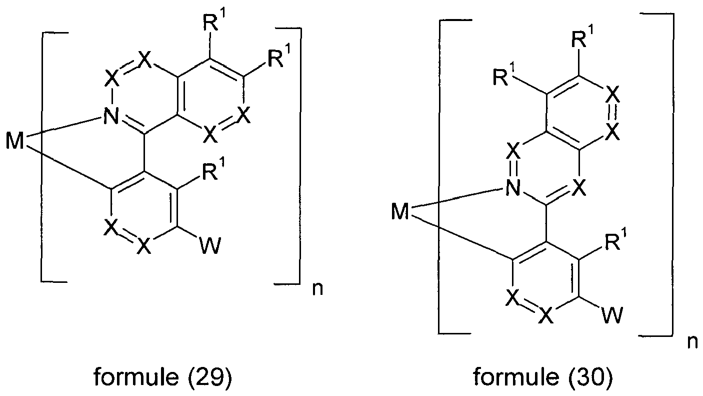 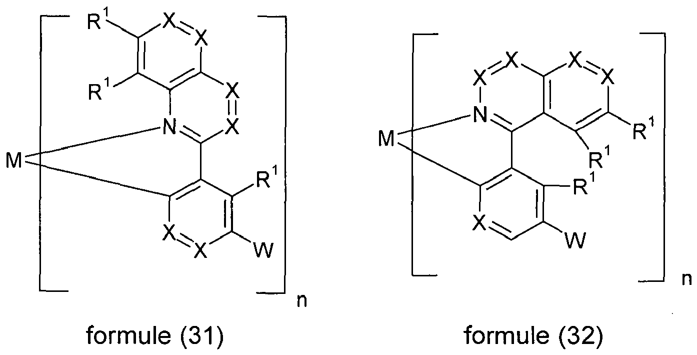 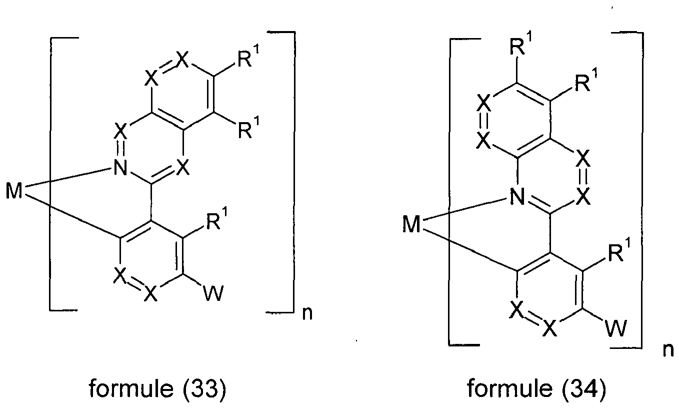 où ce qui suit s'applique aux symboles et indices utilisés :
M est un métal choisi parmi le groupe constitué par iridium, rhodium, platine et palladium ;
W est égal à la formule (3) 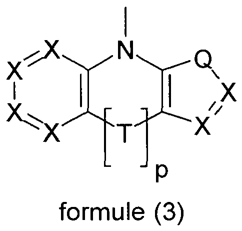 dans laquelle T est choisi, de manière identique ou différente pour chaque occurrence, parmi le groupe constitué par -C(R¹)₂, -Si(R¹)₂, N, -NR¹, -O, -S, -C(=O), -S(=O), -SO₂, -CF₂, -SF₄, -P, -P(=O)R¹, -PF₂, -P(=S)R¹, -As, -As(=O), -As(=S), -Sb, -Ge, -Si, -Sb(=O), -C(R')₂- C(R¹)₂- et -Sb(=S) ;
Q est, de manière identique ou différente pour chaque occurrence, R¹C=CR¹, R¹C=N, O, S, Se ou NR¹;
X est, de manière identique ou différente pour chaque occurrence, CR¹ ou N ;
p est 0 ou 1, où, pour p = 0, le cycle central dans la formule (3) forme un cycle à cinq éléments ;
n est 3 pour M égal à iridium ou rhodium et est 2 pour M égal à platine ou palladium ;
R¹ est, de manière identique ou différente pour chaque occurrence, H, D, F, Cl, Br, I, N(R²)₂, CN, NO₂, Si(R²)₃, B(OR²)₂, C(=O)R², P(=O)(R²)₂, S(=O)R², S(=O)₂R², OSO₂R², un groupe alkyle, alcoxy ou thioalcoxy en chaîne droite comportant de 1 à 40 atomes de C ou un groupe alkényle ou alkynyle en chaîne droite comportant de 2 à 40 atomes de C ou un groupe alkyle, alkényle, alkynyle, alcoxy ou thioalcoxy ramifié ou cyclique comportant de 3 à 40 atomes de C, dont chacun peut être substitué par un radical ou plusieurs radicaux R², où un ou plusieurs groupes CH₂ non adjacents peuvent être remplacé(s) par R²C=CR², C=C, Si(R²)₂, Ge(R²)₂, Sn(R²)₂, C=O, C=S, C=Se, C=NR², P(=O)(R²), SO, SO₂, NR², O, S ou CONR² et où un ou plusieurs atome(s) de H peut/ peuvent être remplacé(s) par D, F, Cl, Br, I, CN ou NO₂, ou un système de cycle aromatique ou hétéroaromatique comportant de 5 à 60 atomes de cycle aromatique, lesquels peuvent dans chaque cas être substitués par un radical ou plusieurs radicaux R², ou un groupe aryloxy ou hétéroaryloxy comportant de 5 à 60 atomes de cycle aromatique, lesquels peuvent être substitués par un radical ou plusieurs radicaux R², ou un groupe diarylamino, un groupe dihétéroarylamino ou un groupe arylhétéroarylamino comportant de 10 à 40 atomes de cycle aromatique, lesquels peuvent être substitués par un radical ou plusieurs radicaux R², ou une combinaison de deux de ces groupes ou plus ; deux radicaux R¹ ou plus peuvent ici former également un système de cycle aliphatique, aromatique et/ou benzo-fusionné l'un avec l'autre ou les uns avec les autres monocyclique ou polycyclique ;
R² est, de manière identique ou différente pour chaque occurrence, H, D, F, Cl, Br, I, N(R³)₂, CN, NO₂, Si(R³)₃, B(OR³)₂, C(=O)R³, P(=O)(R³)₂, S(=O)R³, S(=O)₂R³, OSO₂R³, un groupe alkyle, alcoxy ou thioalcoxy en chaîne droite comportant de 1 à 40 atome(s) de C ou un groupe alkényle ou alkynyle en chaîne droite comportant de 2 à 40 atomes de C ou un groupe alkyle, alkényle, alkynyle, alcoxy ou thioalcoxy ramifié ou cyclique comportant de 3 à 40 atomes de C, dont chacun peut être substitué par un radical ou plusieurs radicaux R³, où un ou plusieurs groupes CH₂ non adjacents peuvent être remplacés par R³C=CR³, C=C, Si(R³)₂, Ge(R³)₂, Sn11(R³)₂, C=O, C=S, C=Se, C=NR³, P(=O)(R³), SO, SO₂, NR³, O, S ou CONR³ et où un ou plusieurs atome(s) de H peut/peuvent être remplacé(s) par D, F, Cl, Br, I, CN ou NO₂, ou un système de cycle aromatique ou hétéroaromatique comportant de 5 à 60 atomes de cycle aromatique, lesquels peuvent dans chaque cas être substitués par un radical ou plusieurs radicaux R³, ou un groupe aryloxy ou hétéroaryloxy comportant de 5 à 60 atomes de cycle aromatique, lesquels peuvent être substitués par un radical ou plusieurs radicaux R³, ou un groupe diarylamino, un groupe dihétéroarylamino ou un groupe arylhétéroarylamino comportant de 10 à 40 atomes de cycle aromatique, lesquels peuvent être substitués par un radical ou plusieurs radicaux R³, ou une combinaison de deux de ces groupes ou plus ; deux radicaux R² adjacents ou plus peuvent ici former un système de cycle aliphatique ou aromatique l'un avec l'autre ou les uns avec les autres monocyclique ou polycyclique ;
R³ est, de manière identique ou différente pour chaque occurrence, H, D, F ou un radical hydrocarbone aliphatique, aromatique et/ou hétéroaromatique comportant de 1 à 20 atomes de C, où, en outre, un ou plusieurs atome(s) de H peut/peuvent être remplacé(s) par F ; deux substituants R³ ou plus peuvent ici également former un système de cycle aliphatique ou aromatique l'un avec l'autre ou les uns avec les autres monocyclique ou polycyclique ;
les indices n et m sont ici choisis de telle sorte que le nombre de coordinations sur le métal corresponde à 6 pour M égal à iridium ou rhodium et corresponde à 4 pour M égal à platine ou palladium ;
les ligands d'une pluralité de ligands L peuvent ici également être liés les uns aux autres via n'importe quel pont souhaité Z et par conséquent former un système de ligands tridentate, tétradentate, pentadentate ou hexadentate.

2. Composé selon la revendication 1, **caractérisé en ce que** le radical W est donné par les formules (47) à (55) : 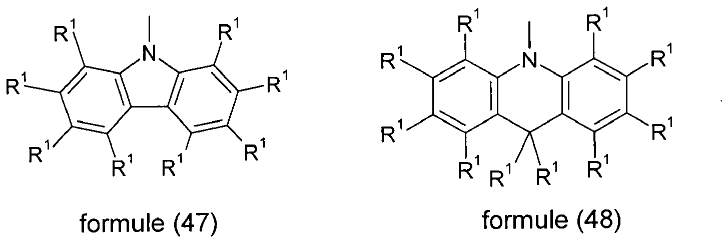 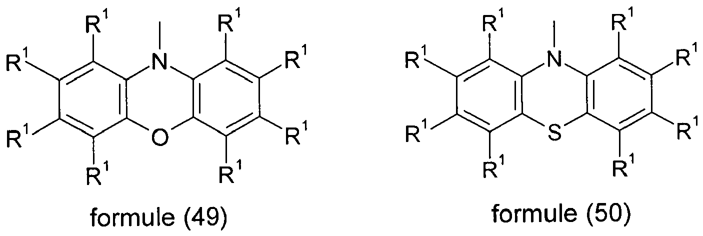 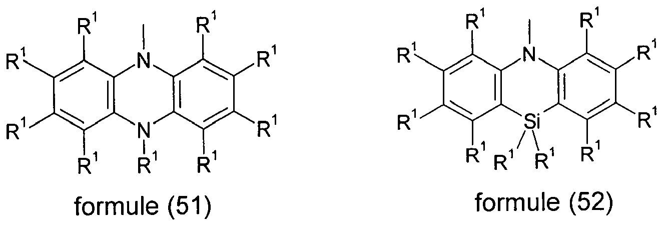 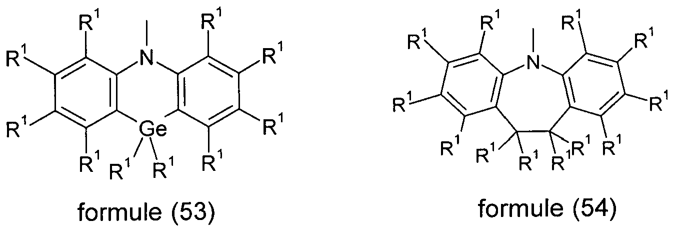 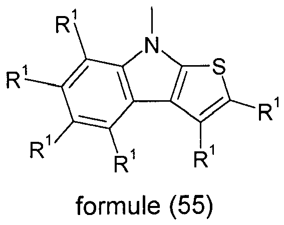 dans lesquelles la ligne indique la position au niveau de laquelle la liaison du radical W sur le ligand est réalisée et dans lesquelles les symboles utilisés correspondent aux symboles définis selon la revendication 1.

3. Composé selon la revendication 1 ou 2, **caractérisé en ce que** la structure M(L)ₙ est choisie parmi les formules (71) à (85) :

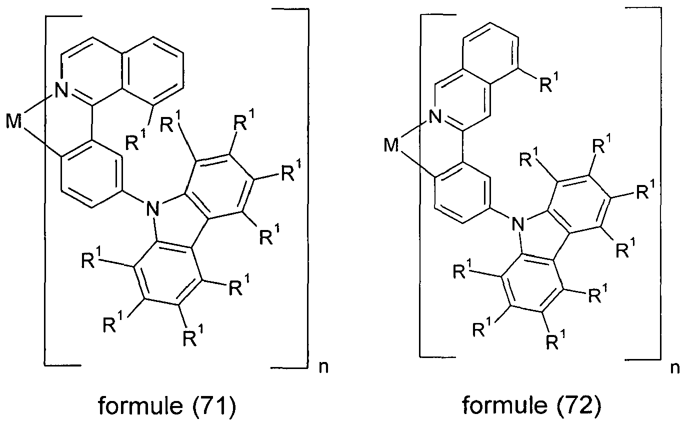

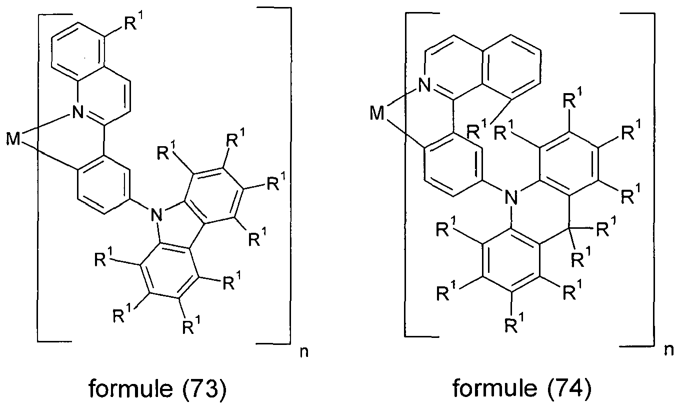

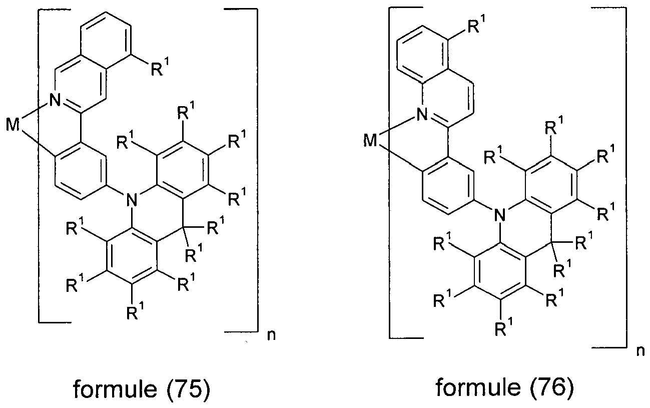

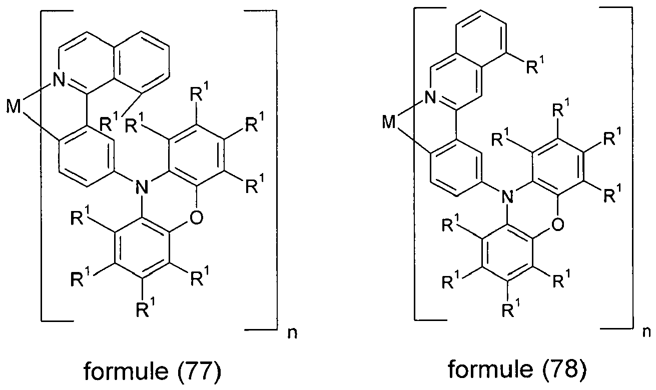

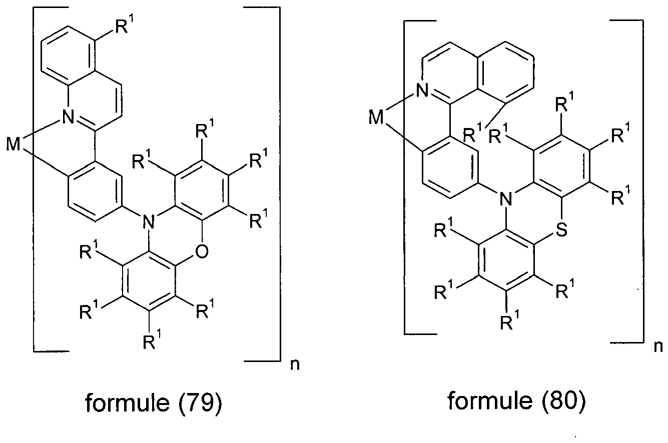

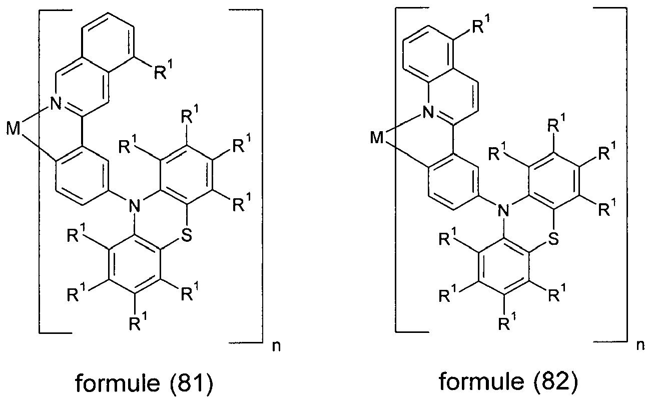

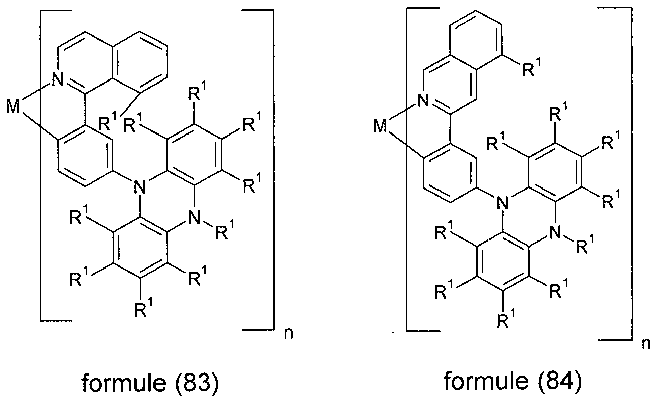

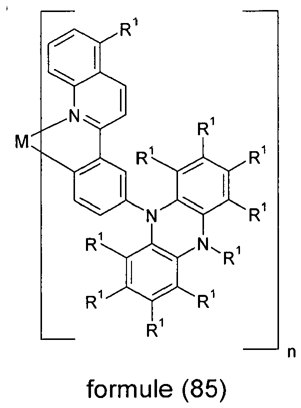

4. Composé selon une ou plusieurs des revendications 1 à 3, **caractérisé en ce que** R¹ est, de manière identique ou différente pour chaque occurrence, -H, -NH₂ ou un radical des formules (86) à (224) où les radicaux peuvent également être monosubstitués ou poly-substitués par un radical R³, lesquels, s'ils sont présents plus d'une fois, peuvent être identiques ou indépendants les uns des autres,

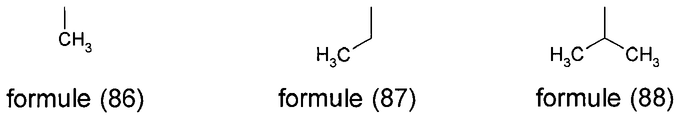

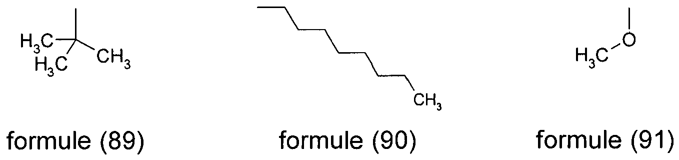

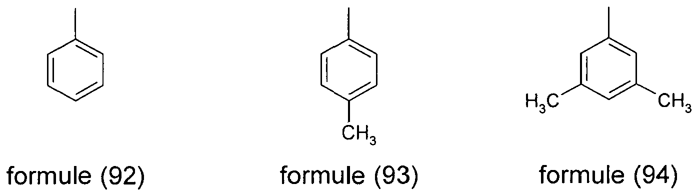

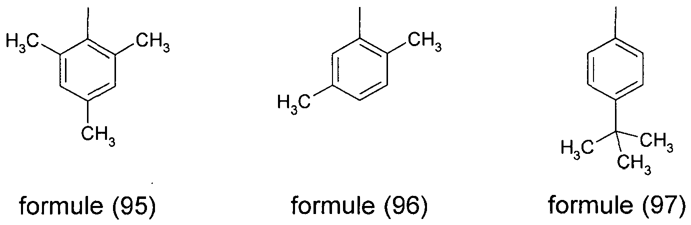

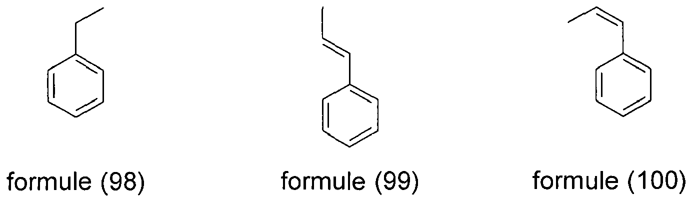

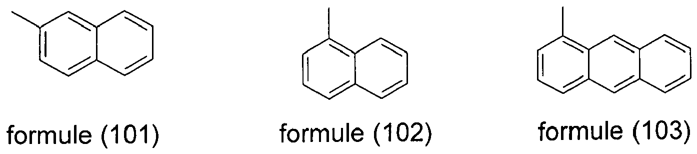

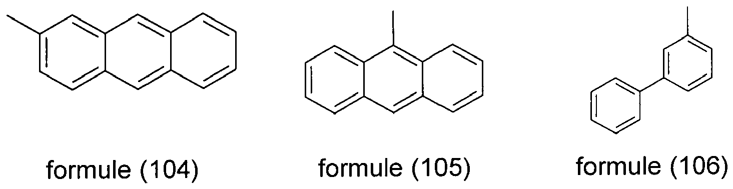

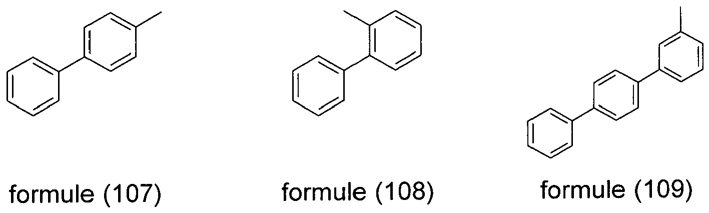

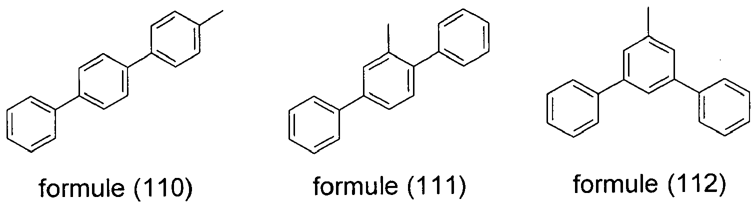

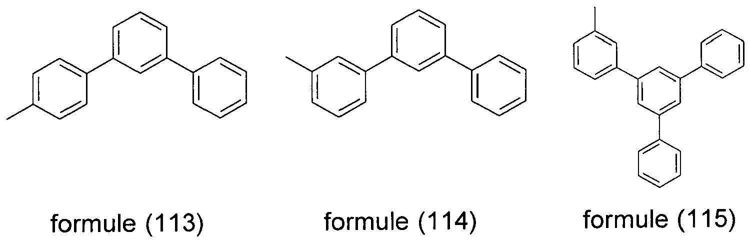

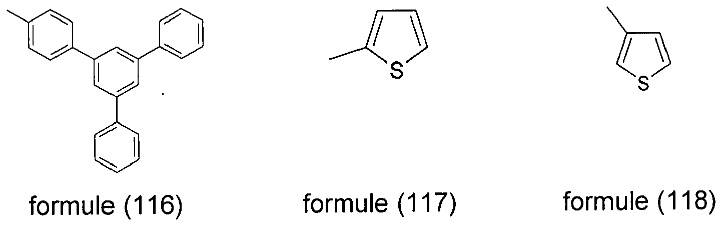

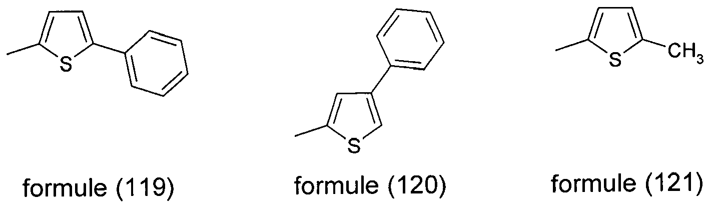

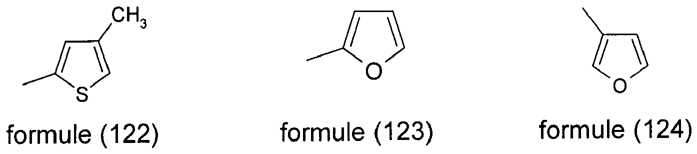

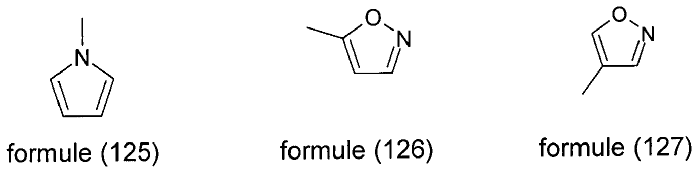

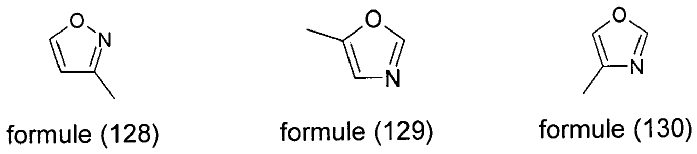

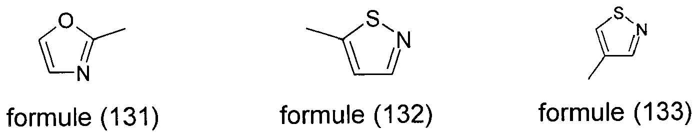

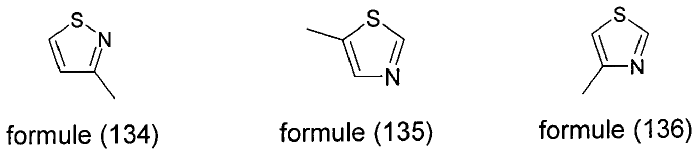

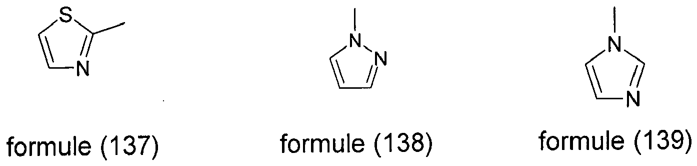

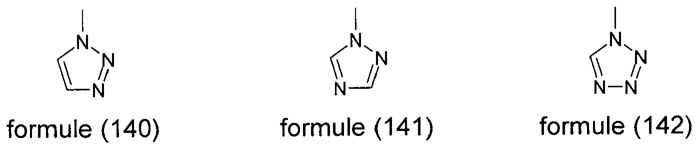

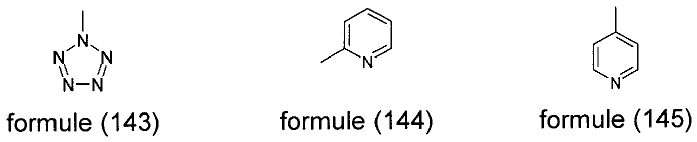

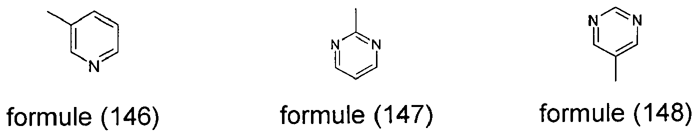

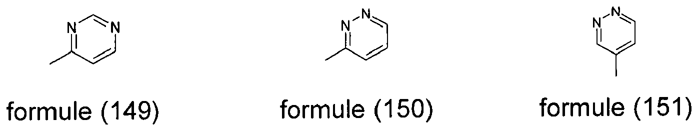

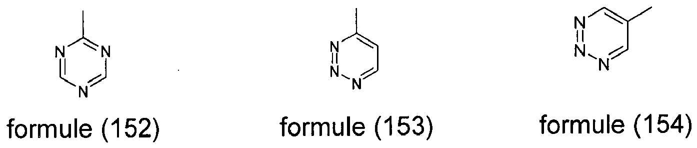

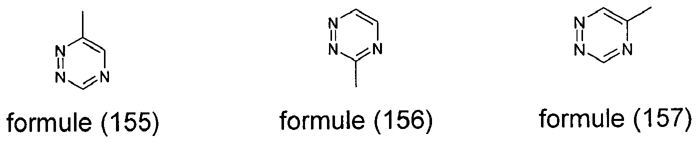

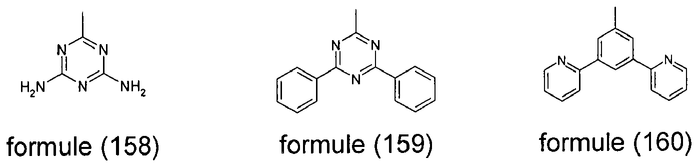

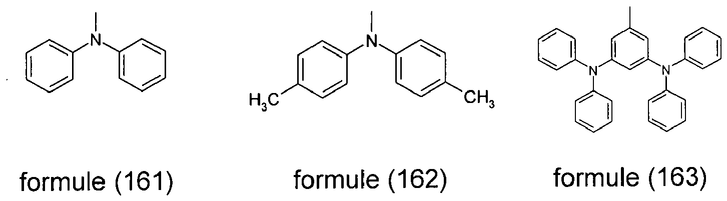

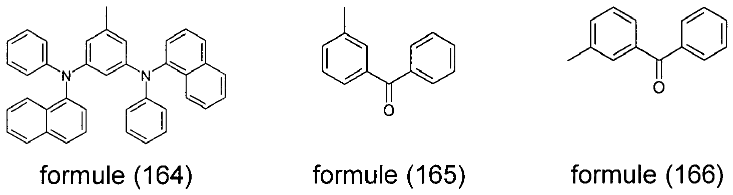

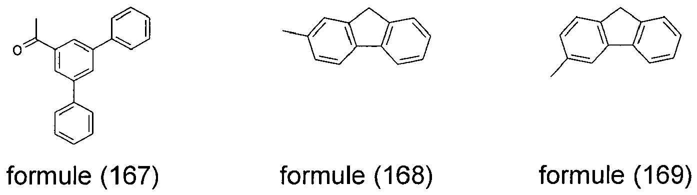

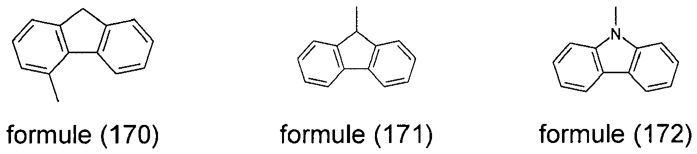

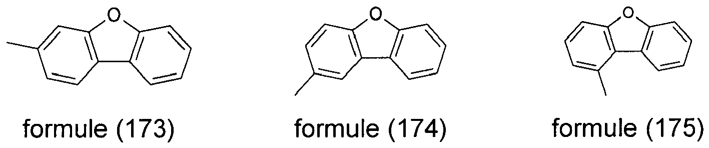

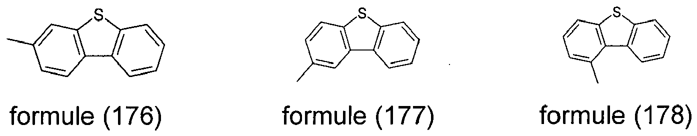

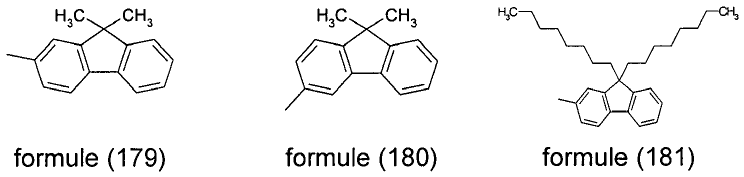

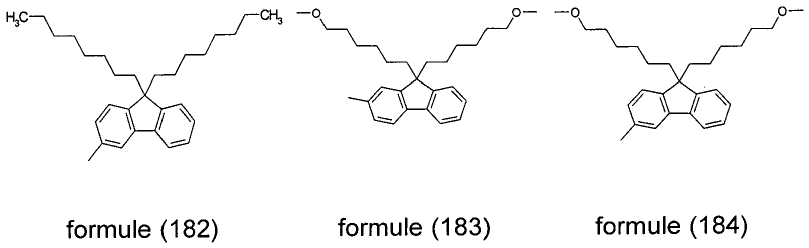

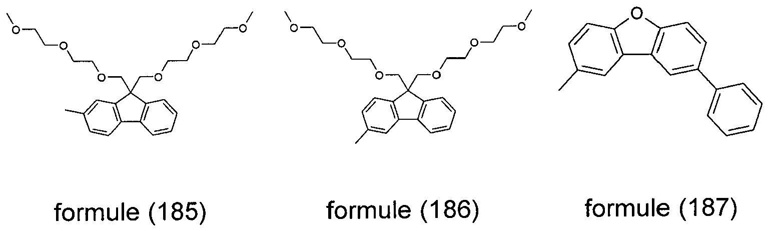

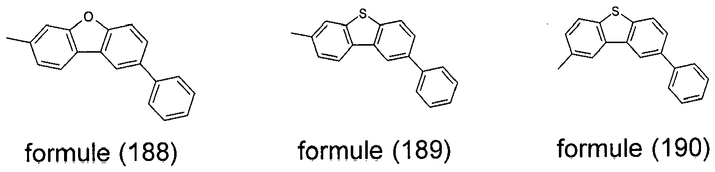

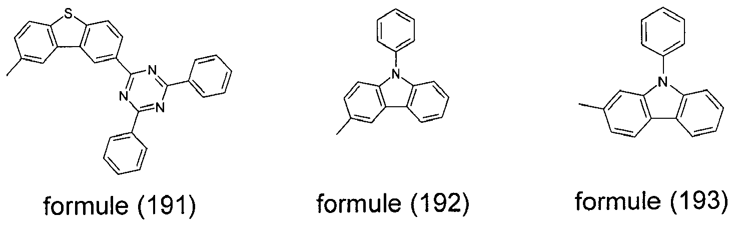

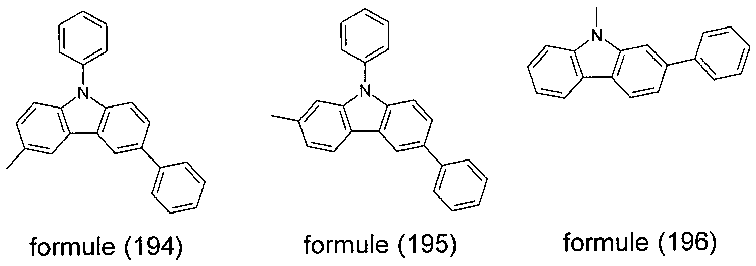

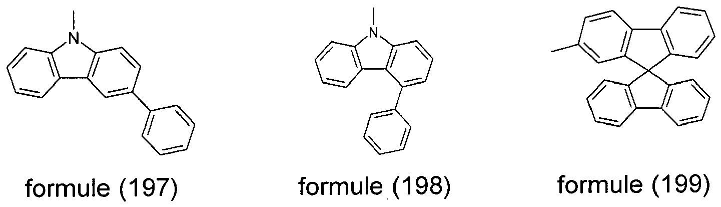

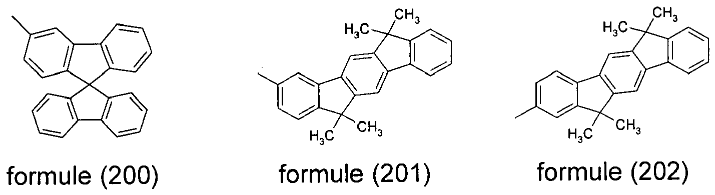

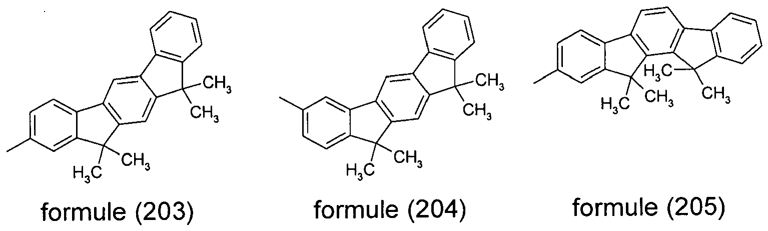

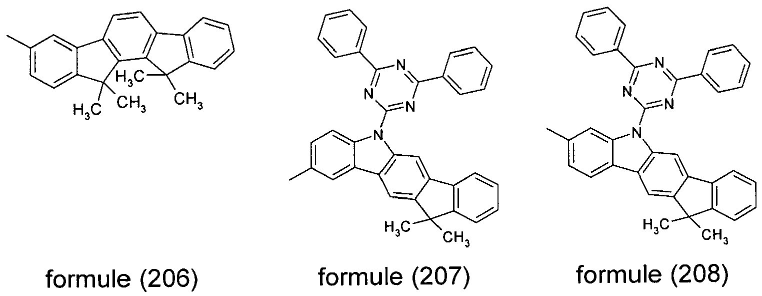

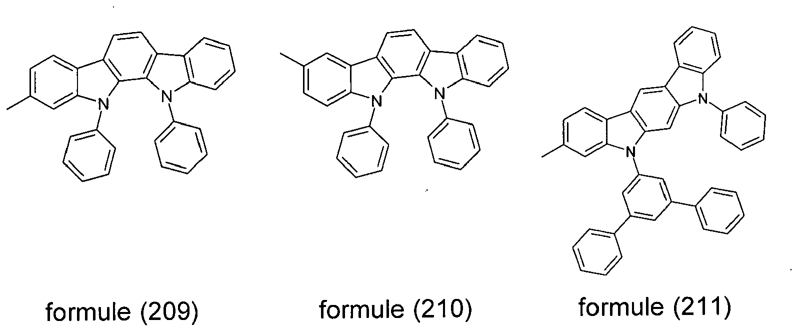

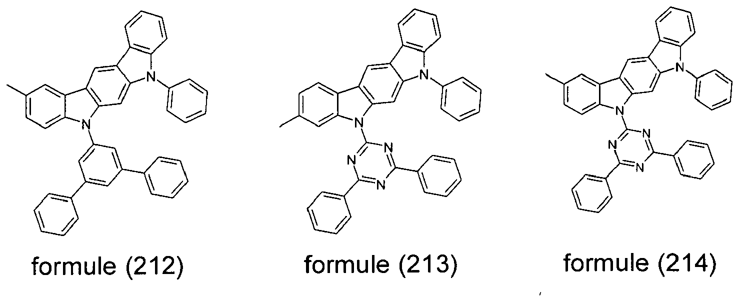

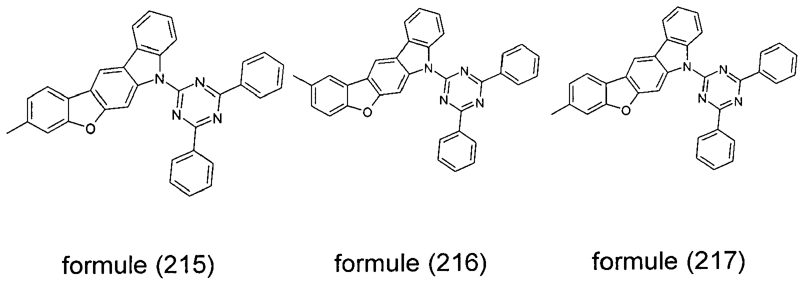

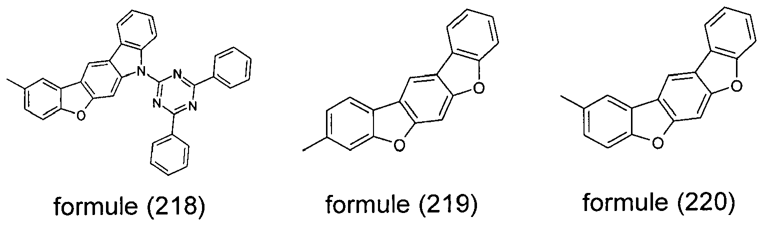

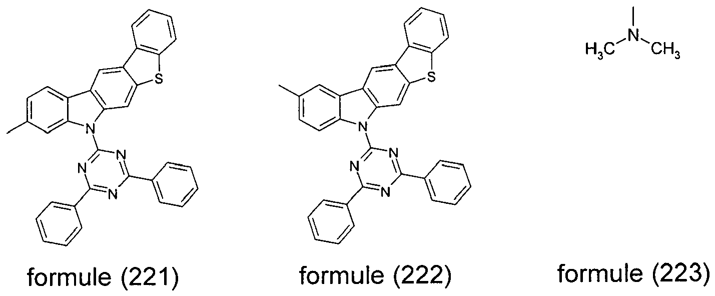

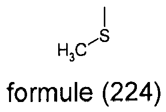

5. Composé selon une ou plusieurs des revendications 1 à 4, **caractérisé en ce que** M est égal à iridium.

6. Procédé pour la préparation d'un composé selon une ou plusieurs des revendications 1 à 5 par réaction des ligands libres correspondants avec des alcoolates métalliques de la formule (282) avec des cétocétonates métalliques de la formule (283) ou avec des halogénures métalliques de la formule (284)
M(OR¹)ₙ formule (282) 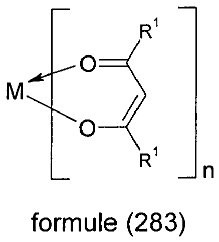
MHalₙ formule (284)
dans lesquelles les symboles M, n et R¹ présentent les significations indiquées ci avant et Hal = F, Cl, Br ou I.

7. Formulation comprenant au moins un composé selon une ou plusieurs des revendications 1 à 5 et au moins un solvant.

8. Utilisation d'un composé selon une ou plusieurs des revendications 1 à 5 dans un dispositif électronique, de façon préférable choisi parmi le groupe constitué par des dispositifs électroluminescents organiques (OLED, PLED), des circuits intégrés organiques (O-IC), des transistors à effet de champ organiques (O-FET), des transistors à film mince organiques (O-TFT), des transistors à émission de lumière organiques (O-LET), des cellules solaires organiques (O-SC), des détecteurs optiques organiques, des photorécepteurs organiques, des dispositifs à extinction de champ organiques (O-FQD), des cellules électrochimiques à émission de lumière (LEC) ou des diodes laser organiques (O-laser).

9. Dispositif électronique comprenant au moins un composé selon une ou plusieurs des revendications 1 à 5, de façon préférable choisi parmi le groupe constitué par des dispositifs électroluminescents organiques (OLED, PLED), des circuits intégrés organiques (O-IC), des transistors à effet de champ organiques (O-FET), des transistors à film mince organiques (O-TFT), des transistors à émission de lumière organiques (O-LET), des cellules solaires organiques (O-SC), des détecteurs optiques organiques, des photorécepteurs organiques, des dispositifs à extinction de champ organiques (O-FQD), des cellules électrochimiques à émission de lumière (LEC) ou des diodes laser organiques (O-laser).

10. Dispositif électroluminescent organique selon la revendication 9, **caractérisé en ce que** le composé selon une ou plusieurs des revendications 1 à 5 est utilisé en tant que composé d'émission dans une ou plusieurs couche(s) d'émission, de façon préférable en combinaison avec un matériau de matrice, dans lequel le matériau de matrice est de façon préférable choisi parmi le groupe constitué par des cétones, des oxydes de phosphine, des sulfoxydes, des sulfones, des triarylamines, des dérivés de carbazole, des dérivés d'indolocarbazole, des dérivés d'indénocarbazole, des dérivés d'azacarbazole, des matériaux de matrice bipolaires, des silanes, des aza-boroles, des esters boroniques, des dérivés de triazine, des complexes de zinc, des dérivés de diazasilole ou de tétraazasilole ou des dérivés de diazaphosphole ou des mélanges de ces matériaux de matrice.

11. Dispositif selon la revendication 9 ou 10 pour une utilisation en médecine pour la photothérapie.

12. Utilisation du dispositif selon la revendication 9 ou 10 dans le domaine des cosmétiques pour la photothérapie.
